# FASCICULE DE BREVET EUROPEEN

(11) **EP 0 679 161 B1**
(45) Date de publication et mention de la délivrance du brevet: **24.06.1998**
(21) Numéro de dépôt: 94904199.0
(22) Date de dépôt: 03.01.1994
(51) Int. Cl.: C07K 5/06, C07D 277/06, A61K 38/05, A61K 31/425

(54) **DERIVES DE THIAZOLIDINE, LEUR PREPARATION ET LES MEDICAMENTS LES CONTENANT**
THIAZOLIDINEDERIVATE IHRE HERSTELLUNG UND SIE ENTHALTENDE MEDIKAMENTE
THIAZOLIDINE DERIVATIVES, PREPARATION THEREOF AND DRUGS CONTAINING SAME

(30) Priorité: 07.01.1993 FR 9300076
(43) Date de publication de la demande: 02.11.1995
(73) Titulaire: RHONE-POULENC RORER S.A., 92160 Antony (FR)
(72) Inventeur: DUBROEUCQ, Marie-Christine, F-95880 Enghien-les-Bains (FR); MANFRE, Franco, F-94450 Limeil-Brévannes (FR)
(74) Mandataire: Savina, Jacques
(86) Numéro de dépôt international: FR9400007
(87) Numéro de publication internationale: WO9415955

(56) Documents cités:
- WO-A-91/13862
- WO-A-93/01167

## Description

La présente invention concerne des dérivés de formule : 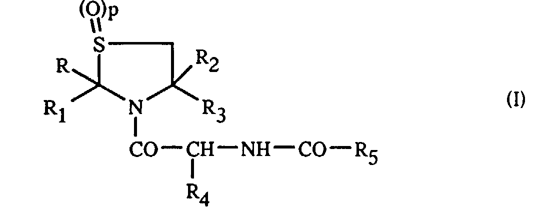 leurs sels, leur préparation et les médicaments les contenant.

Des dérivés de thiazolidine possédant une affinité pour les récepteurs de la cholécystokinine et de la gastrine sont décrits dans la demande de brevet WO93/01167.

Dans la formule (I),
R représente un radical alkyle contenant 1 à 12 atomes de carbone, en chaîne droite ou ramifiée et éventuellement mono ou polyinsaturé, cycloalkyle contenant 3 à 12 atomes de carbone et éventuellement mono ou polyinsaturé, polycycloalkyle contenant 6 à 12 atomes de carbone et éventuellement mono ou polyinsaturé, phénylalkyle dont le noyau phényle est éventuellement substitué (par un ou plusieurs substituants choisis parmi les radicaux alkyle, alcoxy ou les atomes d'halogène), diphénylalkyle, cinnamyle, pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle, oxo-2 pipéridyle, quinuclidinyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-NR₇R_{8,} -NH-CO-CH₃, trifluorométhyle, phényle ou trifluorométhoxy,
R₁ représente un atome d'hydrogène ou un radical alkyle, cycloalkyle, phénylalkyle ou phényle éventuellement substitué par un ou plusieurs substituants choisis parmi les atomes d'halogène et les radicaux alkyle et alcoxy,
R₂ représente une chaîne -(CH₂)ₙ-CO-R₆, -(CH₂)ₘ-O-CO-R"₆, -(CH₂)ₘ-NR₉R₁₀ ou un radical oxazolinyle éventuellement substitué par un ou plusieurs radicaux alkyle ou alkyl-3 oxadiazolyle,
R₃ représente un atome d'hydrogène ou un radical alkyle, cycloalkyle, phénylalkyle ou phényle éventuellement substitué par un ou plusieurs substituants choisis parmi les atomes d'halogène et les radicaux alkyle et alcoxy,
R₄ représente un atome d'hydrogène ou un radical alkyle,
R₅ représente un radical phényle (éventuellement substitué par un ou plusieurs substituants choisis parmi les atomes d'halogène et les radicaux alkyle, alcoxy et alkylthio), naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, --alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₁, -SO₂-NH-SO₂-R₁₁, -CO-NH-CO-R₁₁, -CO-NH-SO₂-R₁₁, -B(OH)₂, -C(NH₂)=NOH, -SO₂-NH-R₁₂, -CO-NH-R₁₂, 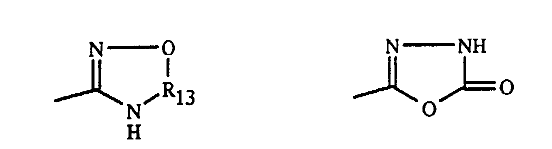 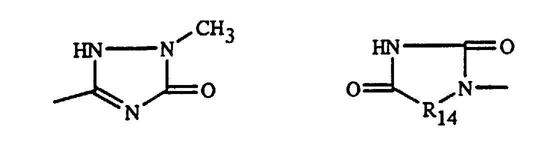 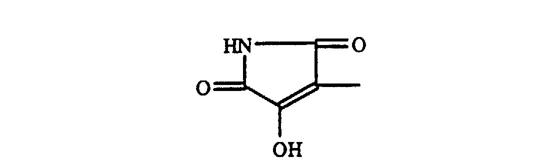 ou diméthyl-2,2 dioxo-4,6 dioxanne-1,3-yl-5,
R₆ représente un radical hydroxy, alcoxy, cycloalkyloxy, cycloalkylalkyloxy, phényle ou -NR₉R₁₀,
R"₆ représente un radical alcoxy, cycloalkyloxy, cycloalkylalkyloxy, phényle ou -NR₉R₁₀,
R₇ représente un atome d'hydrogène ou un radical alkyle, phénylalkyle ou phényle éventuellement substitué par un ou plusieurs substituants choisis parmi les atomes d'halogène et les radicaux alkyle, alcoxy et alkylthio,
R₈ représente un radical alkyle, phénylalkyle ou phényle éventuellement substitué par un ou plusieurs substituants choisis parmi les atomes d'halogène et les radicaux alkyle, alcoxy et alkylthio,
   ou bien R₇ et R₈ forment avec l'atome d'azote auquel ils sont rattachés un hétérocycle mono ou polycyclique saturé ou insaturé contenant 4 à 9 atomes de carbone et un ou plusieurs hétéroatomes (O, N) et éventuellement substitué par un ou plusieurs radicaux alkyle,
R₉ représente un atome d'hydrogène ou un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
R₁₀ représente un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
   ou bien R₉ et R₁₀ forment ensemble avec l'atome d'azote auquel ils sont rattachés un hétérocycle mono ou polycyclique saturé ou insaturé contenant 4 à 9 atomes de carbone et un ou plusieurs hétéroatomes (O, N, S) et éventuellement substitué par un ou plusieurs radicaux alkyle,
R₁₁ représente un radical alkyle, cycloalkyle, trifluorométhyle, phényle éventuellement substitué par un ou plusieurs substituants choisis parmi les radicaux cyano, alcoxy, nitro, amino et les atomes d'halogène,
R₁₂ représente un radical tétrazolyl-5,
R₁₃ représente C=O ou S=O,
R₁₄ représente O ou C=O,
p est égal à 0, 1 ou 2,
n est égal à 0, 1 ou 2,
m est égal à 1 ou 2,
X représente un atome d'hydrogène, un radical alkyle ou phénylalkyle,
alk représente un radical alkyle ou alkylène,
alk' représente un radical hydroxyalkyle, hydroxyalkylène, alkoxyalkyle ou alkoxyalkylène
étant entendu que n est différent de 0 lorsque R₁ et R₃ représentent chacun un atome d'hydrogène et R représente un radical pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-NR₇R_{8,} -NH-CO-CH₃, trifluorométhyle ou trifluorométhoxy.

Dans les définitions qui précédent et celles qui seront citées ci-après, sauf mention contraire, les radicaux alkyle, alkylène et alcoxy et les portions alkyle, alkylène et alcoxy contiennent 1 à 4 atomes de carbone en chaîne droite ou ramifiée, les radicaux ou portions acyle contiennent 2 à 4 atomes de carbone et les radicaux et portions cycloalkyle contiennent 3 à 6 atomes de carbone.

Lorsque R représente un radical alkyle insaturé, celui-ci est de préférence un radical isopropényle.

Lorsque R représente un radical cycloalkyle, celui-ci est de préférence un radical cyclohexyle.

Lorsque R représente un radical cycloalkyle insaturé, celui-ci est de préférence un radical tétrahydrophényle, cyclopentadiène ou dihydrophényle.

Lorsque R représente un radical polycycloalkyle, celui-ci est de préférence un radical norbornyle ou adamantyle.

Lorsque R représente un radical polycycloalkyle insaturé, celui-ci est de préférence un radical norbornényle.

Lorsque R₇ et R₈ forment avec l'atome d'azote auquel ils sont rattachés un hétérocycle, celui-ci est de préférence un cycle pipéridino éventuellement substitué par un ou plusieurs radicaux alkyle ou un cycle tétrahydro-1,2,3,4 quinoléine.

Lorsque R₉ et R₁₀ forment avec l'atome d'azote auquel ils sont rattachés un hétérocycle, celui-ci est de préférence un cycle pipéridino, perhydroazépinyl-1, tétrahydro-1,2,3,6 pyridyl-1, tétrahydro-1,2,3,4 quinolyl-1, pyrrolidinyl-1, tétrahydro-1,2,3,4 isoquinolyl-2, thiomorpholino ou indolinyl-1, ces cycles pouvant être éventuellement substitués par au moins un radical alkyle.

Les composés de formule (I) comportant un ou plusieurs centres asymétriques présentent des formes isomères. Ces isomères font également partie de l'invention.

Les composés de formule (I) pour lesquels p est égal à 0 et R₅ représente un radical phénylamino dont le noyau phényle est éventuellement substitué peuvent être préparés par action d'un dérivé réactif de l'acide carbamique, obtenu éventuellement in situ par action d'un dérivé réactif de l'acide carbonique choisi parmi le N,N'-diimidazole carbonyle, le phosgène, le diphosgène, le triphosgène et le chloroformiate de p-nitrophényle sur un dérivé de formule : 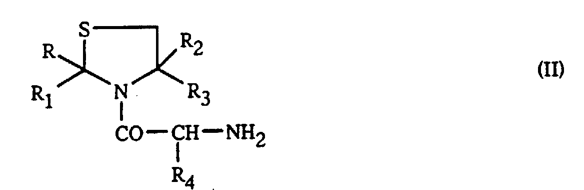 dans laquelle R, R₁, R_{2,} R₃ et R₄ ont les mêmes significations que dans la formule (I), sur une aniline dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H, -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, diméthyl-2,2 dioxo-4,6 dioxanne-1,3-yl-5, -alk-SO₂H, -SO₂-NH-CO-R₁₁, -SO₂-NH-SO₂-R₁₁, -CO-NH-CO-R₁₁, -CO-NH-SO₂-R₁₁, -B(OH)₂, -C(NH₂)=NOH, -SO₂-NH-R₁₂, -CO-NH-R₁₂, diméthyl-2,2 dioxo-4,6 dioxanne-1,3-yl-5 ou

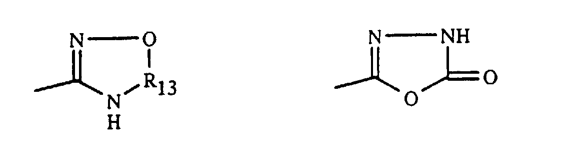

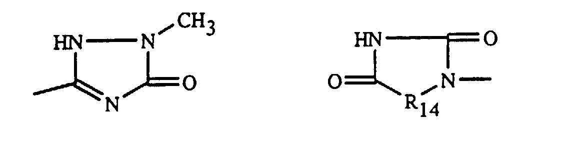

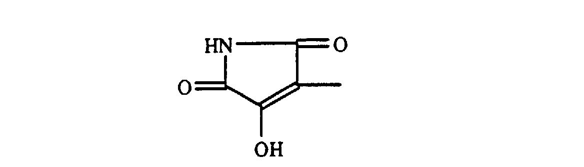

Cette réaction s'effectue généralement au sein d'un solvant inerte tel que le tétrahydrofuranne, le diméthylformamide, un solvant chloré (chloroforme, dichloro-1,2 éthane par exemple) ou un solvant aromatique (benzène, toluène par exemple) ou un mélange de ces solvants, à une température comprise entre 20°C et la température d'ébullition du solvant.

Le dérivé réactif de l'acide carbamique peut être obtenu dans les mêmes conditions de solvant et de température.

Les dérivés de formule (II) peuvent être obtenus par déprotection d'un dérivé de formule : 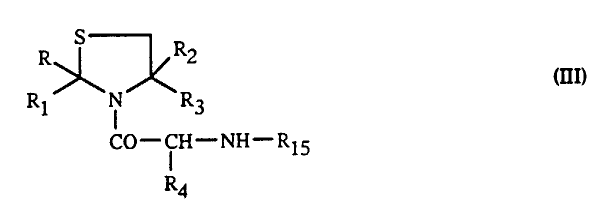 dans laquelle R, R₁, R_{2,} R₃ et R₄ ont les mêmes significations que dans la formule (I) et R₁₅ représente un groupe protecteur tel que tert-butoxycarbonyle ou benzyloxycarbonyle.

Lorsque R₁₅ représente un radical tert-butoxycarbonyle, cette déprotection s'effectue, de préférence, au moyen d'iodotriméthylsilane, au sein d'un solvant inerte tel qu'un solvant chloré (chloroforme, dichloro-1,2 éthane par exemple), à une température comprise entre 15 et 40°C. Lorsque R₁₅ représente un radical benzyloxycarbonyle cette déprotection s'effectue de préférence par hydrogénation au moyen d'hydrogène ou de formiate d'ammonium, en présence de palladium sur charbon, au sein d'un solvant inerte tel que l'acétate d'éthyle ou le méthanol, à une température comprise entre 20°C et la température d'ébullition du milieu réactionnel.

Les dérivés de formule (III) pour lesquels R₂ représente une chaîne -(CH₂)ₙ-CO-R₆, n est égal à 0 et R₆ représente un radical alcoxy, cycloalkyloxy ou cycloalkylalkyloxy peuvent être obtenus par action d'un dérivé de formule : 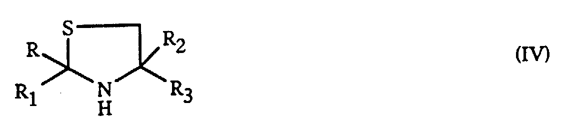 dans laquelle R, R₁ et R₃ ont les mêmes significations que dans la formule (I) et R₂ a les mêmes significations que précédemment, sur un acide de formule : 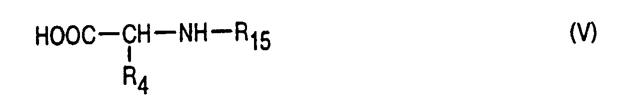 dans laquelle R₄ est défini comme dans la formule (I) et R₁₅ est défini comme dans la formule (III).

Cette réaction s'effectue au sein d'un solvant inerte tel que l'acétonitrile, le tétrahydrofuranne ou un solvant chloré, en présence d'un agent de condensation utilisé en chimie peptidique tel qu'un carbodiimide (N,N'-dicyclohexylcarbodiimide par exemple) ou un chloroformiate d'alkyle, à une température comprise entre 10 et 40°C.

Les dérivés de formule (V) peuvent être obtenus selon les méthodes habituelles de protection des acides aminés.

Les dérivés de formule (IV) pour lesquels R₂ représente une chaîne -(CH₂)ₙ-CO-R₆, n est égal à 0 et R₆ représente un radical hydroxy ou alcoxy peuvent être obtenus par action d'un dérivé de formule :

R-CO-R₁ (VI)

dans laquelle R et R₁ ont les mêmes significations que dans la formule (I), sur un dérivé de formule : 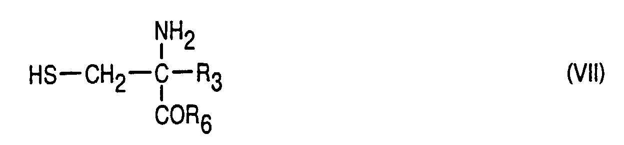 dans laquelle R₃ a les mêmes significations que dans la formule (I) et R₆ représente un radical hydroxy ou alcoxy ou un sel d'un tel composé.

Cette réaction s'effectue généralement au sein d'un solvant inerte tel qu'un alcool ou un solvant chloré, à la température d'ébullition du milieu réactionnel, éventuellement en présence d'une trialkylamine lorsque le composé (VI) est sous forme de sel ou par adaptation de la méthode décrite par J. C. SHEEMAN et coll., J. Am. Chem. Soc., 80, 1158 (1958).

Les dérivés de formule (VII) pour lesquels R₃ n'est pas un atome d'hydrogène peuvent être obtenus par application ou adaptation de la méthode décrite par O. W. GRIFFITH, J. Biol. Chem., 1591-1598 (1983).

Les dérivés de formule (IV) pour lesquels R n'est pas insaturé, R₂ représente une chaîne -(CH₂)ₙ-CO-R₆, n est égal à 0 et R₆ représente un radical alcoxy, cycloalkyloxy, cycloalkylalkyloxy peuvent être obtenus par estérification des dérivés correspondants pour lesquels R₆ représente un radical hydroxy.

Cette estérification s'effectue au moyen d'un alcool R₁₆-OH dans lequel R₁₆ représente un radical alkyle, cycloalkyle ou cycloalkylalkyle, en milieu acide, à la température d'ébullition du mélange réactionnel.

Les dérivés de formule (IV) pour lesquels R n'est pas insaturé et est différent de benzyle ou benzhydryle, R₂ représente une chaîne -(CH₂)ₙ-CO-R₆, n est égal à 0 et R₆ représente un radical tert-butoxy, peuvent être obtenus par action d'isobutène sur un dérivé de formule (IV) correspondant pour lequel R₆ représente un radical hydroxy.

Cette réaction s'effectue au sein d'un solvant inerte tel qu'un solvant chloré, en présence d'un acide tel que l'acide sulfurique, à une température voisine de 20°C.

Les dérivés de formule (IV) pour lesquels R₂ représente une chaîne -(CH₂)ₙ-CO-R₆, n est égal à 0 et R₆ représente un radical alcoxy (excepté méthoxy ou éthoxy), cycloalkyloxy, cycloalkylalkyloxy peuvent également être obtenus par déprotection d'un dérivé de formule : 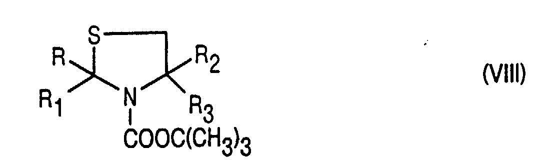 dans laquelle R, R₁ et R₃ ont les mêmes significations que dans la formule (IV) et R₂ représente une chaîne -(CH₂)ₙ-CO-R₆, n est égal à 0 et R₆ représente un radical alcoxy (excepté méthoxy ou éthoxy), cycloalkyloxy ou cycloalkylalkyoxy.

Cette réaction s'effectue au sein d'un solvant inerte tel qu'un solvant chloré, au moyen d'iodotriméthylsilane, à une température comprise entre 15°C et la température d'ébullition du milieu réactionnel.

Les dérivés de formule (VIII) peuvent être obtenus par estérification d'un acide de formule : 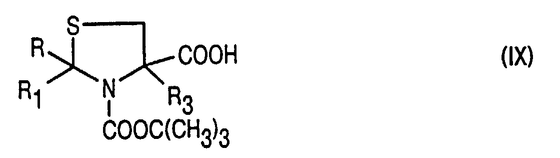 dans laquelle R, R₁ et R₃ ont les mêmes significations que dans la formule (IV).

Cette estérification s'effectue au moyen d'un alcool, en présence de chlorure de tosyle, au sein de la pyridine, à une température comprise entre 0 et 25°C.

Les acides de formule (IX) peuvent être obtenus par action de dicarbonate de ditert-butyle sur un dérivé de formule (IV) dans laquelle R, R₁ et R₃ ont les mêmes significations que dans la formule (IV), R₂ représente une chaîne -(CH₂)ₙ-CO-R₆, n est égal à 0 et R₆ représente un radical hydroxy.

Cette réaction s'effectue au sein d'un solvant inerte tel que l'eau, le dioxanne ou un mélange de ces solvants, en présence d'un carbonate de métal alcalin, à une température voisine de 20°C.

Les dérivés de formule (III) pour lesquels R₂ représente un radical -(CH₂)ₘ-O-CO-R"₆ peuvent être obtenus par action d'un dérivé de formule : 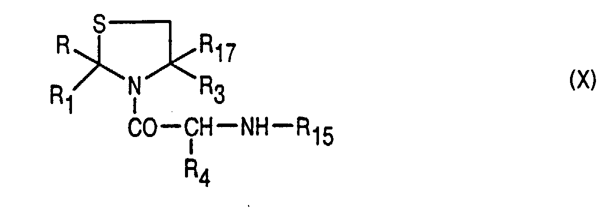 dans laquelle R, R₁ et R₃ ont les mêmes significations que dans la formule (III) et R₁₇ représente un radical -(CH₂)ₘ-OH, avec un halogénure de formule Hal-CO-R"₆ dans laquelle Hal représente un atome d'halogène, R"₆ a les mêmes significations que dans la formule (I) et R₁₅ représente un groupe protecteur tel que tert-butoxycarbonyle ou benzyloxycarbonyle.

Cette réaction s'effectue de préférence au sein d'un solvant inerte tel qu'un solvant chloré, en présence d'une trialkylamine, à une température voisine de 25°C ou éventuellement en présence d'hydrure de sodium dans le diméthylformamide à une température comprise entre 0 et 25°C

Les dérivés de formule (X) pour lesquels R₁₇ représente un radical -(CH₂)ₘ-OH peuvent être obtenus à partir des dérivés de formule (III) correspondants pour lesquels R₂ représente une chaîne -(CH₂)ₙ-CO-R₆, n est égal à 0 ou 1 et R₆ représente un radical alcoxy selon la technique décrite par .K. SOAI et coll., Synth. Comm., 12, 463 (1982) qui consiste à faire réagir le borohydrure de sodium sur un dérivé de formule (III) correspondant.

Cette réaction s'effectue au sein d'un solvant inerte et de préférence au sein d'un mélange de tert-butanol et de méthanol, à la température d'ébullition du mélange réactionnel.

Les composés de formule (III) pour lesquels R₂ représente un radical -(CH₂)ₘ-O-CO-R"₆, R"₆ représente un radical -NR₉R₁₀ et R₉ représente un atome d'hydrogène peuvent être également obtenus par réaction d'un composé de formule (X) dans laquelle R, R₁, et R₃ ont les mêmes significations que dans la formule (III) et R₁₇ représente un radical -(CH₂)ₘ-OH avec un isocyanate de formule R₁₀NCO dans laquelle R₁₀ a les mêmes significations que dans la formule (I).

Cette réaction s'effectue au sein d'un solvant inerte tel que le tétrahydrofuranne, à une température voisine de 20°C et éventuellement en présence d'un méthylate alcalin (sodium ou lithium par exemple).

Les isocyanates sont commercialisés ou peuvent être obtenus par application ou adaptation des méthodes décrites par R. RICHTER et coll., The Chemistry of Cyanate and their thio derivatives, S. PATAI, part 2, Wiley New York (1977) et dans les exemples.

Les dérivés de formule (III) pour lesquels R₂ représente un radical -(CH₂)ₘ-NR₉R₁₀ peuvent être obtenus par action d'une amine HNR₉R₁₀ dans laquelle R₉ et R₁₀ ont les mêmes significations que dans la formule (I) sur un dérivé de formule (X) dans laquelle R₁₇ représente un radical -(CH₂)ₘ-O-SO₂-CH₃.

Cette réaction s'effectue généralement soit en présence d'un large excès d'amine, à une température comprise entre 0 et 10°C soit lorsque l'on utilise le chlorhydrate de l'amine, au sein d'un solvant chloré, en présence d'une trialkylamine, à une température comprise entre 20°C et la température d'ébullition du milieu réactionnel.

Les dérivés de formule (X) dans laquelle R₁₇ représente un radical -(CH₂)ₘ-O-SO₂-CH₃ peuvent être obtenus par réaction d'un dérivé de formule (X) correspondant pour lequel R₁₇ représente un radical -(CH₂)ₘ-OH avec le chlorure de méthanesulfonyle.

Cette réaction s'effectue généralement au sein d'un solvant inerte tel que l'acétonitrile ou le chlorure de méthylène, en présence de triéthylamine, à une température comprise entre 0°C et la température d'ébullition du milieu réactionnel.

Les dérivés de formule (III) pour lesquels R₂ représente un radical -(CH₂)ₙ-CO-R₆ et R₆ représente un radical alcoxy, cycloalcoxy ou cycloalkylalkyloxy peuvent être obtenus par estérification des dérivés de formule (III) correspondants pour lesquels R₂ représente un radical -(CH₂)ₙ-CO-R₆ et R₆ représente un radical hydroxy.

Cette réaction s'effectue de préférence au moyen d'un alcool R₁₆-OH dans lequel R₁₆ représente un radical alkyle, cycloalkyle, cycloalkylalkyle, en présence de chlorure de tosyle, au sein de la pyridine, à une température comprise entre 0 et 25°C, soit lorsque R₁₅ représente un radical benzyloxycarbonyle en milieu acide, à la température d'ébullition du mélange réactionnel.

Les dérivés de formule (III) pour lesquels R₂ représente un radical -(CH₂)ₙ-CO-R₆, R₆ représente un radical hydroxy, R₁₅ représente un radical benzyloxycarbonyle et n est égal à 1 ou 2 peuvent être obtenus par hydrolyse d'un dérivé de formule (X) dans laquelle R₁₇ représente un radical -(CH₂)ₙ-CN.

Cette réaction se fait au moyen d'un acide tel que l'acide sulfurique ou l'acide chlorhydrique, à une température comprise entre 20 et 100°C.

Les dérivés de formule (X) dans laquelle R₁₇ représente un radical -(CH₂)ₙ-CN et n est égal à 1 ou 2 peuvent être obtenus par action de cyanure de potassium sur un dérivé de formule (X) dans laquelle R₁₇ représente un radical -(CH₂)ₘ-O-SO₂-CH₃.

Cette réaction s'effectue de préférence au sein d'un solvant inerte tel qu'un alcool, le diméthylformamide, le diméthylsulfoxyde, à une température comprise entre 25 et 100°C.

Les dérivés de formule (III) pour lesquels R₂ représente un radical -(CH₂)ₙ-CO-R₆ et R₆ représente un radical phényle peuvent être obtenus par action d'un dérivé de formule (III) correspondant pour lequel R₂ représente un radical -(CH₂)ₙ-CO-R₆ et R₆ représente un radical alcoxy avec le bromure de phénylmagnésium.

Cette réaction s'effectue de préférence au sein d'un solvant inerte tel que le tétrahydrofuranne ou l'éther éthylique, à une température comprise entre -70°C et la température d'ébullition du mélange réactionnel.

Les dérivés de formule (III) pour lesquels R n'est pas insaturé, R₂ représente un radical -(CH₂)ₙ-CO-R₆, R₆ représente un radical phényle et n est égal à 1 ou 2 peuvent également être obtenus par hydrolyse d'un dérivé correspondant de formule (X) dans laquelle R₁₇ représente un radical -(CH₂)ₙ-C(=NH)-C₆H₅ dans lequel n est égal à 1 ou 2.

Cette réaction s'effectue généralement en milieu acide (acide chlorhydrique 3N par exemple) à une température comprise entre 25°C et la température d'ébullition du milieu réactionnel.

Les dérivés de formule (X) dans laquelle R₁₇ représente un radical -(CH₂)ₙ-C(=NH)-C₆H₅ peuvent être obtenus par action de phényllithium sur un dérivé correspondant de formule (X) dans laquelle R₁₇ représente un radical -(CH₂)ₙ-CN.

Cette réaction s'effectue au sein d'un solvant inerte tel que le tétrahydrofuranne ou l'éther éthylique, à une température comprise entre 0 et 25°C.

Les composés de formule (III) pour lesquels R₂ représente un radical oxazolinyle éventuellement substitué peuvent être obtenus par action d'un dérivé de formule (III) correspondant pour lequel R₂ représente un radical -(CH₂)ₙ-CO-R₆, n est égal à 0 et R₆ représente un radical hydroxy, sur l'amino-2 éthanol éventuellement substitué par un ou plusieurs radicaux alkyle.

Cette réaction s'effectue au sein d'un solvant inerte tel que le toluène en éliminant l'eau formée, à la température d'ébullition du milieu réactionnel.

Les composés de formule (III) pour lesquels R₂ représente un radical -(CH₂)ₙ-CO-R₆, R₆ représente un radical hydroxy et n est égal à 0 peuvent être obtenus par saponification d'un dérivé de formule (III) correspondant pour lequel R₆ représente un radical alcoxy.

Cette réaction s'effectue au sein de solvants inertes tels que le tétrahydrofuranne, le méthanol ou le dioxane et de l'eau, en présence d'une base telle que la soude, la potasse ou l'hydroxyde de lithium, à une température comprise entre 0 et 25°C.

Les composés de formule (III) pour lesquels R₂ représente un radical alkyl-3 oxadiazolyle peuvent être obtenus par action d'un dérivé de formule (III) correspondant pour lequel R₂ représente un radical -(CH₂)ₙ-CO-R₆, n est égal à 0 et R₆ représente un radical alcoxy sur une alkylamidoxime.

Cette réaction s'effectue au sein d'un solvant inerte tel que le tétrahydrofurane, en présence d'hydrure de sodium, à une température comprise entre 25°C et la température d'ébullition du milieu réactionnel.

Les dérivés de formule (III) pour lesquels R₂ représente une chaîne -(CH₂)ₙ-CO-R₆ et R₆ représente un radical -NR₉R₁₀ peuvent être préparés par réaction d'un dérivé correspondant de formule (III) dans laquelle R₂ représente une chaîne -(CH₂)ₙ-CO-R₆ et R₆ représente un radical hydroxy ou un dérivé réactif de cet acide avec une amine HN-R₉R₁₀.

Lorsque l'on met en oeuvre l'acide, on opère en présence d'un agent de condensation utilisé en chimie peptidique tel qu'un carbodiimide (par exemple le N,N'-dicyclohexylcarbodiimide) ou le N,N'-diimidazole carbonyle, dans un solvant inerte tel qu'un éther (tétrahydrofuranne, dioxanne par exemple), un amide (diméthylformamide) ou un solvant chloré (chlorure de méthylène, dichloro-1,2 éthane, chloroforme par exemple) à une température comprise entre 0°C et la température de reflux du mélange réactionnel.

Lorsque l'on met en oeuvre un dérivé réactif de l'acide, il est possible de faire réagir l'anhydride, un anhydride mixte ou un ester (qui peut être choisi parmi les esters activés ou non de l'acide).

On opère alors soit en milieu organique, éventuellement en présence d'un accepteur d'acide tel qu'une base organique azotée (trialkylamine, pyridine, diaza-1,8 bicyclo [5.4.0] undécène-7 ou diaza-1,5 bicyclo [4.3.0] nonène-5 par exemple), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0 et 40°C.

Les anilines éventuellement substituées sont commercialisées ou peuvent être obtenues par application ou adaptation des méthodes décrites par R. SCHRÖTER, Methoden der organischen Chemie, Houben Weil, Band XI/1, p 360; G.J. ESSELEN et coll., J. Am. Chem. Soc., 36, 322 (1914); G. ADRIANT et coll., Bull. Soc. Chim. FR, 1511 (1970); W.A. JACOBS et coll., J. Am. Chem. Soc., 39, 2418 (1917) et J. Am. Chem. Soc., 39, 1435 (1917) et dans les exemples.

Les composés de formule (I) pour lesquels p est égal à 0 et R₅ représente un radical phénylamino dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est différent d'un atome d'hydrogène peuvent également être préparés par action d'un dérivé de formule (II), sur un phénylisocyanate dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est différent d'un atome d'hydrogène .

Cette réaction s'effectue généralement au sein d'un solvant inerte tel que le tétrahydrofuranne, le diméthylformamide, un solvant chloré (chloroforme, dichloro-1,2 éthane par exemple), un solvant aromatique (benzène, toluène par exemple), à une température comprise entre 10°C et la température d'ébullition du solvant.

Les phénylisocyanates sont commercialisés ou peuvent être obtenus par application ou adaptation des méthodes décrites par R. RICHTER et coll., The Chemistry of Cyanate and their thio derivatives, S. PATAI, part 2, Wiley New York (1977) et dans les exemples.

Les composés de formule (I) pour lesquels p est égal à 0 et R₅ représente un radical phényle éventuellement substitué, naphtyle, indolyle, quinolyle peuvent être préparés par action d'un dérivé de formule (II) sur un acide de formule HOOC-R₅ dans laquelle R₅ a les mêmes significations que précédemment ou un dérivé réactif de cet acide.

Lorsque l'on met en oeuvre l'acide, on opère en présence d'un agent de condensation utilisé en chimie peptidique tel qu'un carbodiimide (par exemple le N,N'-dicyclohexylcarbodiimide) ou le N,N'-diimidazole carbonyle, dans un solvant inerte tel qu'un éther (tétrahydrofuranne, dioxanne par exemple), un amide (diméthylformamide) ou un solvant chloré (chlorure de méthylène, dichloro-1,2 éthane, chloroforme par exemple) à une température comprise entre 0°C et la température de reflux du mélange réactionnel.

Lorsque l'on met en oeuvre un dérivé réactif de l'acide, il est possible de faire réagir l'anhydride, un anhydride mixte ou un ester (qui peut être choisi parmi les esters activés ou non de l'acide).

On opère alors soit en milieu organique, éventuellement en présence d'un accepteur d'acide tel qu'une base organique azotée (trialkylamine, pyridine, diaza-1,8 bicyclo [5.4.0] undécène-7 ou diaza-1,5 bicyclo [4.3.0] nonène-5 par exemple), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0 et 40°C.

Les composés de formule (I) pour lesquels p est égal à 0 et R₅ représente un radical phénylamino dont le noyau phényle est substitué par un radical carboxy, -alk-COOH, -O-alk-COOH, -alk'-COOH, -CH=CH-COOH, -CO-COOH, -S-alk-COOH, -SO-alk-COOH, -SO₂-alk-COOH, -C(=NOH)-COOH, -O-CH₂-alk'-COOH ou -CX=N-O-alk-COOH et/ou R₂ représente une chaîne -(CH₂)ₙ-COOH peuvent également être préparés par hydrolyse ou, selon le cas, hydrogénolyse des esters de formule (I) correspondants.

Lorsque l'on utilise les esters d'alkyle ou de phénylalkyle, il est avantageux d'effectuer l'hydrolyse au moyen d'une base telle que la soude, la potasse ou l'hydroxyde de lithium, au sein d'un solvant inerte tel que le tétrahydrofuranne, le méthanol, le dioxanne, l'eau ou un mélange de ces solvants, à une température comprise entre 20°C et 40°C. Lorsque l'on utilise un ester de triméthylsilyléthyle, il est avantageux d'opérer au sein d'un solvant inerte tel que le tétrahydrofuranne, au moyen d'un fluorure tel que le fluorure de tétrabutylammonium, à une température comprise entre 10 et 40°C. Lorsque l'on utilise des esters de phénylalkyle, il est peut être aussi avantageux d'effectuer une hydrogénolyse au moyen d'hydrogène ou de formiate d'ammonium en présence d'un catalyseur tel que le palladium sur charbon dans un solvant tel que le méthanol ou l'acétate d'éthyle. Lorsqu'on utilise des esters de tert-butyle, il est avantageux d'effectuer l'hydrolyse au moyen d'un acide tel que l'acide trifluoroacétique.

Les esters de triméthylsilyléthyle peuvent être obtenus par application ou adaptation de la méthode décrite par H. GERLACH, Helv. Chim. Acta, 60, 3039 (1977).

Les composés de formule (I) pour lesquels p est égal à 0 et R₅ représente un radical phénylamino dont le noyau phényle est substitué par un radical hydroxyiminoalkyle ou alcoxyiminoalkyle peuvent également être préparés par action du dérivé acylé correspondant de formule (I) sur un dérivé de formule :

H₂N - OR₁₈ (XI)

dans laquelle R₁₈ représente un atome d'hydrogène ou un radical alkyle.

Cette réaction s'effectue généralement au sein d'un solvant inerte tel qu'un alcool (méthanol, éthanol par exemple), l'eau ou un mélange de ces solvants, à la température d'ébullition du solvant et éventuellement en présence d'une base telle que la pyridine.

Les composés de formule (I) pour lesquels p est égal à 0, R₂ représente une chaîne -(CH₂)ₙ-CO-R₆, n est égal à 0, R₆ représente un radical alcoxy, cycloalkyloxy ou cycloalkylalkyloxy et R₅ représente un radical phényle éventuellement substitué, naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamdo, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est différent d'un atome d'hydrogène peuvent également être préparés par action d'un dérivé de formule (IV) sur un acide de formule : 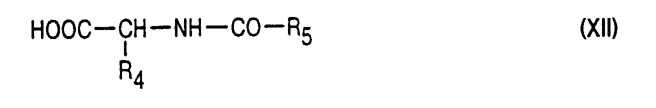 dans laquelle R₅ a les mêmes significations que ci-dessus ou un dérivé réactif de cet acide et R₄ a les mêmes significations que dans la formule (I).

Cette réaction s'effectue de préférence en présence d'un agent de condensation utilisé en chimie peptidique tel qu'un carbodiimide au sein d'un solvant tel que l'acétonitrile, le tétrahydrofuranne ou un solvant chloré ou au moyen de chlorure de thionyle dans le dichlorométhane à une température comprise entre 10° C et la température d'ébullition du solvant.

Les acides de formule (XII) peuvent être obtenus par application ou adaptation de la méthode décrite par J.R. JOHNSON et coll., J. Am. Chem. Soc., 69, 2370 (1947) ou pour les composés pour lesquels R₅ représente un radical phénylamino dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, trifluorométhylsulfonamido, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est différent d'un atome d'hydrogène, par action d'un phénylisocyanate dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, trifluorométhylsulfonamido, carbamoyle, alcoxyiminoalkyle, alcoxyaminocarbonyle, -alk-O-CO-alk, -CH=CH-alk', -alk-O-alk, trifluornméthyîsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est différent d'un atome d'hydrogène sur un dérivé de formule : 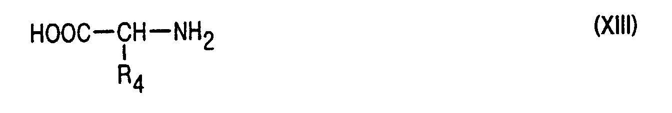 dans laquelle R₄ a les mêmes significations que dans la formule (I).

Cette réaction s'effectue généralement en solution aqueuse en présence d'une base telle qu'un bicarbonate de métal alcalin ou dans le dioxanne aqueux, à une température voisine de 20°C.

Les composés de formule (I) pour lesquels p est égal à 0 et R représente un radical cycloalkyle saturé ou un radical polycycloalkyle saturé peuvent être préparés par hydrogénation d'un dérivé insaturé correspondant de formule (I).

Cette réaction s'effectue au moyen d'hydrogène ou de formiate d'ammonium, en présence d'un catalyseur tel que le palladium sur charbon, au sein d'un solvant inerte tel que le méthanol ou l'acétate d'éthyle, à une température comprise entre 25°C et la température d'ébullition du milieu réactionnel.

Les composés de formule (I) pour lesquels p est égal à 1 ou 2 peuvent être préparés par oxydation des composés de formule (I) correspondants pour lesquels p est égal à 0, étant entendu que les autres radicaux et les autres substituants sont choisis de telle manière qu'ils soient insensibles aux conditions de la réaction.

Cette oxydation s'effectue généralement au moyen d'oxone^{R} (peroxymonosulfate de potassium) commercialisé par Aldrich, au sein d'un alcool tel que le méthanol ou un mélange méthanol-eau, à une température voisine de 25°C.

Il est entendu pour l'homme de métier que, la mise en oeuvre des procédés selon l'invention décrits précédemment, il peut être nécessaire afin d'éviter des réactions secondaires d'introduire des groupes protecteurs des fonctions amine, alcool, acide, cétone tels que ceux décrits par T. W. GREENE, protective groups in organic synthesis, John Wiley and Sons, New York. Par exemple les fonctions amine peuvent être bloquées sous forme de carbamates de tert-butyle ou de méthyle puis régénérées au moyen d'iodotriméthylsilane ou de carbamates de benzyle puis régénérées par hydrogénation après avoir mis en oeuvre le procédé selon l'invention. Les fonctions alcool peuvent par exemple être bloquées sous forme de benzoate puis régénérées par hydrolyse en milieu alcalin après avoir mis en oeuvre le procédé selon l'invention. Les fonctions cétone peuvent être bloquées sous forme de dioxolanne-1,3 puis régénérées au moyen de d'un mélange acide chlorhydrique-acide acétique.

Les énantiomères des composés de formule (I) contenant au moins un site asymétrique peuvent être obtenus par dédoublement des racémiques par exemple par chromatographie sur colonne chirale ou par synthèse à partir des précurseurs chiraux.

Les composés de formule (I) peuvent être purifiés par les méthodes connues habituelles, par exemple par cristallisation, chromatographie ou extractions.

Les composés de formule (I) comportant un reste basique peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) comportant un reste acide peuvent éventuellement être transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac, d'une amine ou d'un sel d'une amine sur un composé de formule (I), dans un solvant. Le sel formé est séparé par les méthodes habituelles.

Ces sels font également partie de l'invention.

Comme exemples de sels pharmaceutiquement acceptables, peuvent être cités les sels d'addition avec les acides minéraux ou organiques (tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalino-terreux (calcium, magnésium), le sel d'ammonium, les sels de bases azotées (éthanolamine, triméthylamine, méthylamine, benzylamine, N-benzyl-β-phénéthylamine, choline, arginine, leucine, lysine, N-méthyl glucamine).

Les composés de formule (I) présentent des propriétés pharmacologiques intéressantes. Ces composés possèdent une forte affinité pour les récepteurs de la cholécystokinine (CCK) et de la gastrine et sont donc utiles dans le traitement et la prévention des désordres liés à la CCK et à la gastrine au niveau du système nerveux et de l'appareil gastrointestinal.

C'est ainsi que ces composés peuvent être utilisés pour le traitement ou la prévention des psychoses, des troubles anxieux, de la dépression, de la neurodégénération, des attaques de panique, de la maladie de Parkinson, de la diskynésie tardive, du syndrôme du colon irritable, de la pancréatite aigué, des ulcères, des désordres de la motilité intestinale, de certaines tumeurs sensibles à la CCK, comme régulateur de l'appétit, dans le sevrage aux traitements chroniques et abus d'alcool ou de médicaments et comme constricteur de la pupille de l'oeil.

Ces composés ont également un effet de potentialisation sur l'activité analgésique des médicaments narcotiques et non narcotiques. En outre, ils peuvent avoir un effet analgésique propre.

Par ailleurs, les composés ayant une forte affinité pour les récepteurs CCK modifient les capacités de mémorisation. En conséquence, ces composés peuvent être efficaces dans les troubles de la mémoire.

L'affinité des composés de formule (I) pour les récepteurs CCK a été déterminée selon une technique inspirée de celle de A. SAITO et coll . (J. Neuro. Chem., 37, 483-490 (1981)) au niveau du cortex cérébral et au niveau du pancréas.

Dans ces tests, la CI₅₀ des composés de formule (I) est généralement inférieure ou égale à 1000 nM.

Par ailleurs, il est connu que les produits qui reconnaissent les récepteurs centraux de la CCK ont une spécificité similaire pour les récepteurs de la gastrine dans le tractus gastrointestinal (BOCK et coll., J. Med. Chem., 32, 16-23 (1989); REYFELD et coll., Am. J. Physiol., 240, G255-266 (1981); BEINFELD et coll., Neuropetides, 3, 411-427 (1983).

Les composés de formule (I) présentent une toxicité faible. Leur DL₅₀ est généralement supérieure à 40 mg/kg par voie sous cutanée chez la souris.

Les composés de formule (I) préférés sont ceux pour lesquels R représente un radical alkyle contenant 1 à 12 atomes de carbone en chaîne droite ou ramifiée, cycloalkyle contenant 3 à 12 atomes de carbone et éventuellement monoinsaturé, polycycloalkyle contenant 6 à 12 atomes de carbone et éventuellement mono ou polyinsaturé, phénylalkyle, phényle éventuellement substitué par un ou plusieurs substituants choisis parmi les atomes d'halogène et les radicaux alkyle, -alk-COOX et carboxy, R₁ représente un atome d'hydrogène, R₂ représente une chaîne -(CH₂)ₙ-CO-R₆, R₃.représente un atome d'hydrogène, R₄.représente un atome d'hydrogène et R₅ représente un radical phénylamino dont le noyau phényle est substitué par un radical alkyle, -alk-COOX ou carboxy.

D'un intéret particulier sont les composés suivants :
- acide {[(tert-butoxycarbonyl-4 cyclohexyl-2 thiazolidinyl-3)-2 oxo-2 éthyl]-3 uréido}-3 phénylacétique-(4R),
- {[(méthyl-3 phényl)-3 uréido]-2 acétyl}-3 benzyl-2 thiazolidinecarboxylate-4 de tert-butyle-(4R),
- acide {[(tert-butoxycarbonyl-4 benzyl-2 thiazolidinyl-3)-2 oxo-2 éthyl]-3 uréido}-3 phénylacétique-(4R),
- acide {{{tert-butoxycarbonyl-4 (tétrahydro-1,2,3,6 phényl-1-(RS))-2 thiazolidinyl-3]-2 oxo-2 éthyl}-3 uréido}-3 phénylacétique-(2R,4R),
- acide {{[tert-butoxycarbonyl-4 [(norbornéne-5 yl)-2-(2RS)]-2 thiazolidinyl-3]-2 oxo-2 éthyl}-3 uréido}-3 phénylacétique-(4R),
- {{[tert-butoxycarbonyl-4 [(norbornène-5 yl)-2-(2RS)]-2 thiazolidinyl-3]-2 oxo-2 éthyl}-3 uréido}-3 phénylacétate de méthyle-(4R),
- acide {{[tert-butoxycarbonyl-4 [(norbornène-5 yl)-2-(2RS)]-2 thiazolidinyl-3]-2 oxo-2 éthyl}-3 uréido}-3 phénylacétique-(4R),
- acide {{[tert-butoxycarbonyl-4 (norbomyl-2-(2RS))-2 thiazolidinyl-3]-2 oxo-2 éthyl}-3 uréido}-3 phénylacétique-(4R),
- {[(méthyl-3 phényl)-3 uréido]-2 acétyl}-3 [(norbornène-5 yl-2-(RS)]-2 thiazolidinecarboxylate de tert-butyle-(4R),
- acide {(tert-butoxycarbonyl-4 tert-butyl-2 thiazolidinyl-3)-2 oxo-2 éthyl]-3 uréido}-3 phénylacétique-(4R),
- acide {[(tert-butoxycarbonyl-4 butyl-2 thiazolidinyl-3-(4R))-2 oxo-2 éthyl]-3 uréido}-3 phényl-2 propionique-(S),
- {[(méthyl-3 phényl)-3 uréido]-2 acétyl}-3 butyl-2 thiazolidinecarboxylate-4 de tert-butyle-(4R),
- acide {[(tert-butoxycarbonyl-4 butyl-2 thiazolidinyl-3)-2 oxo-2 éthyl]-3 uréido}-3 benzoïque-(4R),
- {[(méthyl-3 phényl)-3 uréido]-2 acétyl}-3 (tétrahydro-1,2,3,6 phényl-1-(RS))-2 thiazolidinecarboxylate-4 de tert-butyle-(2R, 4R),
- acide {{[tert-butoxycarbonyl-4 (phényl-2 phényl)-2 thiazolidinyl-3]-2 oxo-2 éthyl}-3 uréido}-3 phénylacétique-(2R, 4R),
- {[(méthyl-3 phényl)-3 uréido]-2 acétyl}-3 (phényl-2 phényl)-2 thiazolidinecarboxylate-4 de tert-butyle-(2R,4R),
- acide {{{[carboxy-4 (fluoro-2 phényl)l-2 thiazolidinyl-3-(2R,4RS)]-2 oxo-2 éthyl}-3 uréido}-3 phényl-2 propionique-(S),
leurs isomères, les mélanges de leurs isomères et leurs sels.

Les exemples suivants illustrent l'invention sans la limiter.

### EXEMPLE 1

Dans un ballon contenant 2,0 g de {[(tert-butoxycarbonyl-4 cyclohexyl-2 thiazolidinyl-3)-2 oxo-2 éthyl]-3 uréido}-3 phénylacétate de benzyle-(4R) (isomère A), 1,7 g de formiate d'ammonium et 2,0 g de palladium sur charbon à 10%, on ajoute lentement sous atmosphère inerte 30 cm³ de méthanol. Le milieu réactionnel est chauffé à reflux pendant 2 heures, puis refroidi à une température voisine de 25°C. Le catalyseur est séparé par filtration et le filtrat est concentré à sec sous pression réduite à 40°C. Le résidu obtenu est dissous dans 25 cm³ d'une solution aqueuse d'hydroxyde de sodium 0,1N et lavé par 2 fois 10 cm³ d'oxyde de diéthyle. La phase aqueuse est amenée à pH 2 par addition d'une solution aqueuse d'acide sulfurique 1N. Le produit précipité est séparé par filtration, lavé par 2 fois 10 cm³ d'eau et séché à l'air. On obtient ainsi, 1,0 g d'acide {[(tert-butoxycarbonyl-4 cyclohexyl-2 thiazolidinyl-3)-2 oxo-2 éthyl]-3 uréido}-3 phénylacétique-(4R) (isomère A) fondant à 115°C; [α]_{D}²⁵ = -32,9° ± 1,2° (C% = 1,2; DMF).

RMN (200 MHz, δ en ppm, DMSO D₆ + 2 gouttes de CD₃COOD). On observe un mélange de deux rotamères dans les proportions 75/25. 0,70 à 2,00 (mt, 11H, -CH- et -CH₂- du cyclohexyl), 1,38 et 1,47 (2s, 9H en totalité, -C(CH₃)₃), 3,34 (d, J = 6,5 Hz, S-CH₂- du rotamère majoritaire), 3,07 et 3,49 (2 mt, -S-CH₂- du rotamère minoritaire), 3,47 (s, 2H, Ar-CH₂-COO-), 4,00 et 4,10 (2 AB, J = 17,0 Hz, 2H, N-CH₂-CO-), 4,69 (t, J = 8,5 Hz, N-CH-CO- du rotamère minoritaire), 5,03 (d, J = 9,0 Hz, -N-CH-S du rotamère minoritaire), 5,09 (t, J = 6,5 Hz, N-CH-CO- du rotamère majoritaire), 5,24 (d, J = 9,5 Hz, N-CH-S du rotamère majoritaire), 6,78 (d, J = 8,0 Hz, H en position 4 de Ar-NH-CO- du rotamère majoritaire), 6,86 (d, J = 8,0 Hz, H en position 4 de Ar-NH-CO- du rotamère minoritaire), 7,15 (t, J = 8,0Hz, 1H, H en position 5 de Ar-NH-CO-), 7,15 à 7,45 (mt, 2H de Ar-NH-CO-).
A Le {[(tert-butoxycarbonyl-4 cyclohexyl-2 thiazolidinyl-3)-2 oxo-2 éthyl]-3 uréido}-3 phénylacétate de benzyle-(4R) (isomère A) peut être préparé de la manière suivante : à une solution de 1,38 g d'(amino-2 acétyl)-3 cyclohexyl-2 thiazolidinecarboxylate-4 de tert-butyle-(4R) (isomère A) dans 25 cm³ de chloroforme, on ajoute à une température voisine de 25°C, 1,1 g d'isocyanato-3 phénylacétate de benzyle en solution dans 10 cm³ de chloroforme. Le mélange réactionnel est agité pendant 12 heures à une température voisine de 25 °C puis concentré à sec sous pression réduite à 35°C. Le produit brut obtenu est purifié par chromatographie sur silice [éluant : acétate d'éthyle-cyclohexane (40/60 en volumes)]. Les fractions contenant le produit attendu sont réunies et concentrées à sec sous pression réduite à 40°C. On obtient ainsi, 2,1 g de {[(tert-butoxycarbonyl cyclohexyl-2 thiazolidinyl-3)-2 oxo-2 éthyl]-3 uréido}-3 phénylacétate de benzyle-(4R) (isomère A) sous forme d'une huile jaune utilisée telle quelle dans les synthèses ultérieures.
B L'(amino-2 acétyl)-3 cyclohexyl-2 thiazolidinecarboxylate-4 de tert-butyle-(4R) (isomère A) peut être préparé de la manière suivante : à une solution de 2,14 g de (tert-butoxycarbonylamino-2 acétyl)-3 cyclohexyl-2 thiazolidinecarboxylate-4 de tert-butyle-(4R) (isomère A) dans 25 cm³ de chloroforme, on ajoute goutte à goutte 0,8 cm³ d'iodotriméthylsilane à une température voisine de 25°C. Le milieu réactionnel est agité pendant 3 heures à une température voisine de 25°C, puis on ajoute 50 cm³ d'eau. La phase aqueuse est séparée par décantation et extraite par 2 fois 10 cm³ de chloroforme. Les phases organiques réunies sont lavées successivement par 50 cm³ d'eau, 50 cm³ d'une solution aqueuse saturée d'hydrogénocarbonate de sodium et 50 cm³ d'une solution aqueuse saturée de chlorure de sodium, puis séchées sur sulfate de magnésium et concentrées à sec sous pression réduite à 40°C. On obtient ainsi 1,38 g d'(amino-2 acétyl)-3 cyclohexyl-2 thiazolidinecarboxylate-4 de tert-butyle-(4R) (isomère A) sous forme d'une huile jaune utilisée telle quelle dans les synthèses ultérieures.
C Le (tert-butoxycarbonylamino-2 acétyl)-3 cyclohexyl-2 thiazolidinecarboxylate-4 de tert-butyle-(4R) (isomère A) peut être préparé de la manière suivante : à une solution de 10,6 g de cyclohexyl-2 thiazolidinecarboxylate-4 de tert-butyle-(2RS, 4R) et de 6,8 g d'acide tert-butoxycarbonylamino-2 acétique dans 100 cm³ de tétrahydrofuranne maintenu à une température voisine de 0°C, on ajoute en 30 minutes une solution de 8,0 g de N,N'-dicyclohexylcarbodiimide dans 75 cm³ de tétrahydrofuranne. Le milieu réactionnel est amené à une température voisine de 25°C, puis est agité pendant 16 heures. Le produit insoluble est séparé par filtration et lavé par 3 fois 20 cm³ d'acétonitrile. Le filtrat est concentré à sec sous pression réduite à 40°C. Le produit brut obtenu est purifié par chromatographie sur silice [éluant : chlorure de méthylène-méthanol (98/2 en volumes)]. Les fractions contenant le produit attendu sont réunies et concentrées à sec sous pression réduite à 40°C. On obtient ainsi 9,7 g de (tert-butoxycarbonylamino-2 acétyl)-3 cyclohexyl-2 thiazolidinecarboxylate-4 de tert-butyle-(4R) (isomère A) sous forme d'une huile jaune utilisée telle quelle dans les synthèses ultérieures.
D Le cyclohexyl-2 thiazolidinecarboxylate-4 de tert-butyle-(2RS, 4R) peut être préparé de la manière suivante : à une suspension, refroidie à une température voisine de 5°C, de 12,4 g d'acide cyclohexyl-2 thiazolidinecarboxylique-4-(2RS,4R) dans 125 cm³ de chloroforme, on ajoute goutte à goutte 3,0 cm³ d'acide sulfurique concentré. Le milieu réactionnel est saturé par de l'isobutène pendant 2 heures en maintenant la température du milieu réactionnel au voisinage de 5°C. Après retour à une température voisine de 20°C, l'agitation est poursuivie pendant 12 heures. Le milieu réctionnel est traité par 200 cm³ d'une solution aqueuse saturée d'hydrogénocarbonate de sodium. La phase organique est décantée et la phase aqueuse est extraite par 2 fois 100 cm³ de chloroforme. Les phases organiques réunies sont lavées par 100 cm³ d'eau et par 100 cm³ d'une solution aqueuse saturée de chlorure de sodium, séchées sur sulfate de magnésium et concentrées à sec sous pression réduite à 40°C. On obtient ainsi 10,6 g de cyclohexyl-2 thiazolidinecarboxylate-4 de tert-butyle-(2RS, 4R) sous forme d'une huile jaune utilisée telle quelle dans les synthèses ultérieures.
E L'acide cyclohexyl-2 thiazolidinecarboxylique-4-(2RS,4R) peut être préparé de la manière suivante : à une suspension de 7,26 g de L-cystéine dans 100 cm³ d'éthanol, on ajoute à une température voisine de 50°C, 7,0 g de cyclohexylcarboxaldéhyde. Le milieu réactionnel est chauffé à reflux pendant 3 heures. Après refroidissement à une température voisine de 25°C, le produit insoluble est séparé par filtration et lavé successivement par 2 fois 50 cm³ d'éthanol et 2 fois 50 cm³ d'oxyde de diéthyle. On obtient ainsi 12,4g d'acide cyclohexyl-2 thiazolidinecarboxylique-4-(2RS,4R) fondant à 248°C.
F L'isocyanato-3 phénylacétate de benzyle peut être préparé de la manière suivante : à une suspension de 0,5 g de charbon dans un mélange de 2,6 cm³ de chloroformiate de trichlorométhyle et de 75 cm³ de toluène, on ajoute en 15 minutes à une température voisine de -30°C, une solution de 5,0 g d'amino-3 phénylacétate de benzyle dans 40 cm³ de toluène. Le mélange réactionnel est agité pendant 2 heures à une température voisine de 20°C, puis chauffé à reflux pendant 3 heures. Après refroidissement à une température voisine de 25°C, le milieu réactionnel est dégazé par barbottage d'azote; le catalyseur est séparé par filtration et le filtrat est concentré à sec sous pression réduite à 40°C. On obtient ainsi 6,0 g isocyanato-3 phénylacétate de benzyle sous forme d'une huile jaune utilisée telle quelle dans les synthèses ultérieures.
G L'amino-3 phénylacétate de benzyle peut être préparé de la manière suivante : à un mélange de 28 g de nitro-3 phénylacétate de benzyle dans 125cm³ de méthanol et 1300 cm³ d'eau, on ajoute 265 g de chlorure d'ammonium et 130 g de zinc en poudre. Le milieu réactionnel est chauffé à reflux pendant 1 heure puis refroidi à une température voisine de 10°C. Les sels insolubles sont séparés par filtration et le filtrat est extrait par 3 fois 500 cm³ d'oxyde de diéthyle. Les phases organiques collectées sont lavées successivement par 100 cm³ d'eau et 200 cm³ d'une solution aqueuse saturée de chlorure de sodium. On obtient ainsi, après séchage sur sulfate de magnésium et concentration à sec sous pression réduite, 20,5 g d'amino-3 phénylacétate de benzyle sous forme d'une huile jaune utilisée telle quelle dans les synthèses ultérieures.
H Le nitro-3 phénylacétate de benzyle peut être préparé de la manière suivante : à un mélange contenant 21,0 g d'acide nitro-3 phénylacétique et 0,5cm³ de diméthylformamide dans 200 cm³ de dichloro-1,2 éthane, on ajoute lentement 10,3 cm³ de dichlorure d'oxalyle. Le milieu réactionnel est agité 3 heures à une température voisine de 25°C, puis on ajoute 12,5 g d'alcool benzylique. L'agitation est poursuivie 12 heures à cette même température, puis le milieu réactionnel est lavé successivement par 2 fois 100 cm³ d'une solution aqueuse saturée d'hydrogénocarbonate de sodium, 100 cm³ d'eau et 100 cm³ d'une solution aqueuse saturée de chlorure de sodium. La phase organique collectée est séchée sur sulfate de magnésium et concentrée à sec sous pression réduite à 40°C. Le résidu obtenu est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi, 28,0 g de nitro-3 phénylacétate de benzyle sous forme d'une huile jaune utilisée telle quelle dans les synthèses ultérieures.

### EXEMPLE 2

On opère d'une manière analogue à celle décrite à l'exemple lA mais à partir de 3,4 g d' (amino-2 acétyl)-3 benzyl-2 thiazolidinecarboxylate-4 de tert-butyle-(4R) (isomère A) et de 1,3 g d'isocyanate de méthyl-3 phényle. Le produit brut obtenu est purifié par chromatographie sur silice [éluant: acétate d'éthyle-cyclohexane (30/70 en volumes)]. Les fractions contenant le produit attendu sont réunies et concentrées à sec sous pression réduite à 40°C. On obtient ainsi, après cristallisation dans l'oxyde de diéthyle, 0,34 g de {[(méthyl-3 phényl)-3 uréido]-2 acétyl}-3 benzyl-2 thiazolidinecarboxylate-4 de tert-butyle-(4R) (isomère A) fondant à 104°C; [α]_{D}²⁵ = -107,3° ± 1,5° (C% = 0,61; CH₃OH).

RMN (250 MHz, δ en ppm, DMSO D₆, 403 K). 1,55 (1s, 9H, -C(CH₃)₃), 2,29 (s, 3H, Ar-CH₃), 2,90 (dd, J = 14,0 et 8,5 Hz, 1H, 1H du Ph-CH₂), 3,38 (d, J = 7,0 Hz, 2H, S-CH₂-), 3,53 (dd, J = 14,0 et 4,0 Hz, 1H, 1H du Ph-CH₂), 3,95 et 4,05 (2dd, J = 17,5 Hz et 5,0 Hz, 2H, N-CH₂-CO-), 5,02 (t, J = 7,0 Hz, 1H, -N-CH-CO-), 5,52 (dd, J = 8,5 Hz et 4,0 Hz,1H, N-CH-S-), 6,24 (t, J = 5,0 Hz, 1H, -NH-CO-), 6,77 (d, J = 8,0 Hz, 1H, H en position 4 de Ar-NH-CO), 7,13 (t, J = 8,0 Hz, 1H, H en position 5 de Ar-NH-CO-), 7,15 à 7,40 (mt, 7H, Ph du benzyl et 2H de Ar-NH-CO-), 8,38 (s large, 1H, H de -CO-NH-Ar).
A L'(amino-2 acétyl)-3 benzyl-2 thiazolidinecarboxylate-4 de tert-butyle-(4R) (isomère A) peut être préparé d'une manière analogue à celle décrite à l'exemple 1B mais à partir de 8,50 g de (tert-butoxycarbonylamino-2 acétyl)-3 benzyl-2 thiazolidinecarboxylate-4 de tert-butyle-(4R) (isomère A) et de 2,84 cm³ d'iodotriméthylsilane. On obtient ainsi 6,3 g d'(amino-2 acétyl)-3 benzyl-2 thiazolidinecarboxylate-4 de tert-butyle-(4R) (isomère A) sous forme d'une huile jaune utilisée telle quelle dans les synthèses ultérieures.
B Le (tert-butoxycarbonylamino-2 acétyl)-3 benzyl-2 thiazolidinecarboxylate-4 de tert-butyle-(4R) (isomère A) peut être préparé d'une manière analogue à celle décrite à l'exemple 1C mais à partir de 25,7 g de benzyl -2 thiazolidine-carboxylate-4 de tert-butyle-(2RS,4R), de 13,9 g d'acide tert-butoxycarbonylamino-2 acétique et de 16,3 g de N,N'-dicyclohexylcarbodiimide. Le produit brut est purifié par chromatographie sur silice [éluant : chlorure de méthylène-méthanol (99/1 en volumes)]. Les fractions contenant le produit attendu sont réunies et concentrées à sec sous pression réduite à 40°C. On obtient ainsi 12,1 g de (tert-butoxycarbonylamino-2 acétyl)-3 benzyl-2 thiazolidinecarboxylate-4 de tert-butyle-(4R) (isomère A) sous forme d'une meringue crème utilisée telle quelle dans les synthèses ultérieures.
C Le benzyl-2 thiazolidinecarboxylate-4 de tert-butyle-(2RS,4R) peut être préparé de la manière suivante : à une solution de 30,5 g de tert-butoxycarbonyl-3 benzyl-2 thiazolidinecarboxylate-4 de tert-butyle-(4R) (isomère A) dans 250 cm³ de chloroforme, on introduit lentement 11,8 cm³ d'iodotriméthylsilane à une température voisine de 25°C. Le mélange réactionnel est agité pendant 1 heure, puis on ajoute 10 cm³ d'eau. La phase organique est séparée et la phase aqueuse est extraite par 2 fois 20 cm³ de chlorure de méthylène. Les phases organiques réunies sont lavées par 100 cm³ d'eau et par 100 cm³ d'une solution aqueuse saturée d'hydrogénocarbonate de sodium, séchées sur sulfate de magnésium et concentrées à sec sous pression réduite à 40°C. On obtient ainsi 25,0 g de benzyl-2 thiazolidinecarboxylate.4 de tert-butyle-(2RS, 4R) sous forme d'une huile jaune utilisée telle quelle dans les synthèses ultérieures.
D Le tert-butoxycarbonyl-3 benzyl-2 thiazolidinecarboxylate-4 de tert-butyle-(4R) (isomère A) peut être préparé de la manière suivante : à une solution d'acide tert-butoxycarbonyl-3 benzyl-2 thiazolidinecarboxylique-4-(4R) (isomère A) et de 29,5 g de chlorure de paratoluènesulfonyle dans 230 cm³ de pyridine refroidie à une température voisine de 5°C, on ajoute 11,6 g de tert-butanol. Après retour à une température voisine de 25°C, le milieu réactionnel est agité pendant 20 heures, puis concentré sous pression réduite à 50°C. Le résidu est repris dans 100 cm³ d'eau et extrait par 3 fois 150 cm³ d'acétate d'éthyle. Les phases organiques réunies sont lavées successivement par 100 cm³ d'eau, 100 cm³ d'une solution aqueuse saturée d'hydrogénocarbonate de sodium, 100 cm³ d'eau et 100 cm³ d'une solution aqueuse saturée de chlorure de sodium; puis séchées sur sulfate de magnésium et concentrées à sec sous pression réduite. Le produit brut obtenu est purifié par chromatographie sur silice [éluant : acétate d'éthyle-cyclohexane (50/50 en volumes)]. Les fractions contenant le produit attendu sont réunies et concentrées à sec sous pression réduite à 40°C. On obtient ainsi 30,0 g de tert-butoxycarbonyl-3 benzyl-2 thiazolidinecarboxylate-4 de tert-butyle-(4R) (isomère A) sous forme d'une huile orangée utilisée telle quelle dans les synthèses ultérieures.
E L'acide tert-butoxycarbonyl-3 benzyl-2 thiazolidinecarboxylique-4-(4R) (isomère A) peut être préparé de la manière suivante: à une solution de 40,0 g d'acide benzyl-2 thiazolidine carboxylique-4 (2RS,4R) dans 360 cm³ d'une solution aqueuse de soude 0,5N et 230 cm³ de dioxanne on ajoute,à une température voisine de 5°C, une solution de 39,4 g de dicarbonate de ditert-butyle dans 40 cm³ de dioxanne. Après retour à une température voisine de 25°C le mélange réactionnel est agité pendant 20 heures puis concentré à sec sous pression réduite à 40°C. Le résidu obtenu est repris dans 300 cm³ d'eau, acidifié à pH voisin de 2 par addition d'une solution aqueuse d'acide sulfurique 1N et extrait par 3 fois 100 cm³ d'acétate d'éthyle. Les phases organiques réunies sont lavés par 50 cm³ d'eau et par 50 cm³ d'une solution aqueuse saturée de chlorure de sodium, séchées sur sulfate de magnésium et concentrées à sec sous pression réduite à 40°C. On obtient ainsi 51,6 g d'acide tert-butoxycarbonyl-3 benzyl-2 thiazolidinecarboxylique-4-(4R) (isomère A) sous forme de cristaux jaune clair fondant à 156°C.
F L'acide benzyl-2 thiazolidine carboxylique-4-(2RS,4R) peut être préparé d'une manière analogue à celle décrite à l'exemple 1E mais à partir de 53,7 g de L-cystéine et de 56,5 g de phénylacétaldéhyde. On obtient ainsi 75,8 g d'acide benzyl-2 thiazolidinecarboxylique-4-(2RS,4R) fondant à 200 °C.

### EXEMPLE 3

On opère d'une manière analogue à celle décrite à l'exemple 1 mais à partir de 2,58 g de {[(tert-butoxycarbonyl-4 benzyl-2 thiazolidinyl-3)-2 oxo-2 éthyl]-3 uréido}-3 phénylacétate de benzyle-(4R) (isomère A), de 1,6 g de formiate d'ammonium et de 1,3 g de palladium sur charbon à 10%. On obtient ainsi 1,06 g d'acide {[(tert-butoxycarbonyl-4 benzyl-2 thiazolidinyl-3)-2 oxo-2 éthyl]-3 uréido}-3 phénylacétique-(4R) (isomère A) fondant à 110°C; [α]_{D}²⁵ = -89,0° ± 1,6° (C% = 0,51; méthanol).

RMN (200 MHz, δ en ppm, DMSO D₆ + 2 gouttes de CD₃COOD, 393 K). 1,55 (1s, 9H, -C(CH₃)₃), 2,92 (dd, J = 14,0 et 9,5 Hz, 1H, 1H du Ph-CH₂), 3,38 (d, J = 7,0 Hz, 2H, S-CH₂-), 3,50 (s, 2H, Ar-CH₂-COO-), 3,53 (dd, J = 14,0 et 4,5 Hz, 1H, 1H du Ph-CH₂), 3,95 et 4,05 (2d, J = 17,0 Hz, 2H, N-CH₂-CO-), 5,02 (t, J = 7,0 Hz, 1H, -N-CH-CO-), 5,50 (dd, J = 9,5 et 4,5 Hz, 1H, N-CH-S-), 6,84 (d, J = 8,0 Hz, 1H, H en position 4 de Ar-NH-CO), 7,17 (t, J = 8,0 Hz, 1H, H en position 5 de Ar-NH-CO-), 7,20 à 7,40 (mt, 7H, Ph du benzyl et 2H de Ar-NH-CO-).

Le {[(tert-butoxycarbonyl-4 benzyl-2 thiazolidinyl-3)-2 oxo-2 éthyl]-3 uréido}-3 phénylacétate de benzyle-(4R) (isomère A) peut être préparé d'une manière analogue à celle décrite à l'exemple lA mais à partir de 2,8 g d'(amino-2 acétyl)-3 benzyl-2 thiazolidinecarboxylate-4 de tert-butyle-(4R) (isomère A) et de 2,22 g d'isocyanato-3 phénylacétate de benzyle. Le produit brut obtenu est purifié par chromatographie sur silice [éluant : acétate d'éthyle-cyclohexane (40/60 en volumes)]. Les fractions contenant le produit attendu sont réunies et concentrées à sec sous pression réduite à 40°C. On obtient ainsi, après battage dans l'hexane, 2,82 g de ([(tert-butoxycarbonyl-4 benzyl-2 thiazolidinyl-3)-2 oxo-2 éthyl]-3 uréido}-3 phénylacétate de benzyle-(4R) (isomère A) sous forme d'un produit amorphe utilisé tel quel dans les synthèses ultérieures.

### EXEMPLE 4

A une solution de 0,82 g de {{[tert-butoxycarbonyl-4 (tétrahydro-1,2,3,6 phényl-1-(RS))-2 thiazolidinyl-3]-2 oxo-2 éthyl}-3 uréido}-3 phénylacétate de méthyle-(2R,4R) dans 8 cm³ d'un mélange eau-méthanol (30/70 en volumes) on ajoute, à une température voisine de 25°C, 0,10 g d'hydroxyde de potassium. Le mélange réactionnel est agité pendant 4 heures à une température voisine de 25°C, puis concentré de moitié environ sous pression réduite. La solution obtenue est diluée avec 40 cm³ d'eau, lavée par 2 fois 20cm³ d'oxyde de diéthyle, puis amenée à un pH voisin de 2 par addition d'une solution aqueuse d'acide sulfurique 1N et extraite par 2 fois 50 cm³ d'acétate d'éthyle. Les phases organiques réunies sont successivement lavées par 20cm³ d'eau, 20 cm³ d'une solution aqueuse saturée de chlorure de sodium, séchées sur sulfate de magnésium et concentrées à sec sous pression réduite à 40°C. Le produit brut obtenu est purifié par chromatographie sur silice [éluant : chlorure de méthylène-méthanol (90/10 en volumes)]. Les fractions contenant le produit attendu sont réunies et concentrées à sec sous pression réduite à 40°C. On obtient ainsi, 0,31 g d'acide {{[tert-butoxycarbonyl-4 (tétrahydro-1,2,3,6 phényl-1-(RS))-2 thiazolidinyl-3]-2 oxo-2 éthyl}-3 uréido}-3 phénylacétique-(2R,4R) sous forme d'un produit amorphe.

RMN (200 MHz, δ en ppm, DMSO D₆, 393 K).On observe le mélange des deux diastéréoisomères dans les proportions 50/50. 1,10 à 1,50 et de 1,80 à 2,30 (mt, 7H, -CH= et -CH₂- du tétrahydro-1,2,5,6 phényl), 1,47 et 1,49 (2s, 9H en totalité, -C(CH₃)₃), 3,25 à 3,45 (mt, 2H, S-CH₂-), 3,45 (s, 2H, Ar-CH₂-COO-), 4,08 (mt, 2H, N-CH₂-CO-), 5,02 (t, J = 7,5 Hz, 1H, -N-CH-CO-), 5,32 (mt, 1H, N-CH-S-), 5,62 (mt, 2H, -CH=CH- du tétrahydro-1,2,5,6 phényl), 6,22 (t, J = 5,5 Hz, 1H, NH-CO-), 6,84 (d, J = 8,0 Hz, 1H, H en position 4 de Ar-NH-CO), 7,13 (t, J = 8,0 Hz, 1H, H en position 5 de Ar-NH-CO-), 7,25 à 7,35 (mt, 2H de Ar-NH-CO-), 8,45 (s large, 1H, 1H de -CO-NH-Ar).
A Le {{[tert-butoxycarbonyl-4 (tétrahydro-1,2,3,6 phényl-1-(RS))-2 thiazolidinyl-3]-2 oxo-2 éthyl}-3 uréido}-3 phénylacétate de méthyle-(2R,4R) peut être préparé d'une manière analogue à celle décrite à l'exemple lA mais à partir de 2,18 g d' (amino-2 acétyl)-3 (tétrahydro-1,2,3,6 phényl-1-(RS))-2 thiazolidinecarboxylate-4 de tert-butyle-(2R,4R) et de 1,34 g d'isocyanato-3 phénylacétate de méthyle. Le produit brut est purifié par chromatographie sur silice [éluant : chlorure de méthylène-acétate d'éthyle (80/20 en volumes)]. Les fractions contenant le produit attendu sont réunies et concentrées à sec sous pression réduite à 40°C. On obtient ainsi 1,15 g de {{[tert-butoxycarbonyl-4 (tétrahydro-1,2,3,6 phényl-1-(RS))-2 thiazolidinyl-3]-2 oxo-2 éthyl}-3 phénylacétate de méthyle-(2R,4R) sous forme d'une huile épaisse utilisée telle quelle dans les synthèses ultérieures.
B L'(amino-2 acétyl)-3 (tétrahydro-1,2,3,6 phényl-1-(RS))-2 thiazolidinecarboxylate-4 de tert-butyle-(2R,4R) peut être préparé d'une manière analogue à celle décrite à l'exemple 1B mais à partir de 2,28 g de (tert-butoxycarbonylamino-2 acétyl)-3 (tétrahydro-1,2,3,6 phényl-1-(RS))-2 thiazolidinecarboxylate-4 de tert-butyle-(2R,4R) et de 0,78 cm³ d'iodotriméthylsilane. On obtient ainsi 2,18 g d'(amino-2 acétyl)-3 (tétrahydro-1,2,3,6 phényl-1-(RS))-2 thiazolidinecarboxylate-4 de tert-butyle-(2R,4R) sous forme d'une huile jaune utilisée telle quelle dans les synthèses ultérieures.
C Le (tert-butoxycarbonylamino-2 acétyl)-3 (tétrahydro-1,2,3,6 phényl-1-(RS))-2 thiazolidinecarboxylate-4 de tert-butyle-(2R,4R) peut être préparé d'une manière analogue à celle décrite à l'exemple 1C mais à partir de 4,4 g de (tétrahydro-1,2,3,6 phényl-1-(RS))-2 thiazolidinecarboxylate-4 de tert-butyle-(2RS,4R), de 2,28 g d'acide tert-butoxycarbonylamino-2 acétique et de 2,68 g de N,N'-dicyclohexylcarbodiimide. Le produit brut est purifié par chromatographie sur silice [éluant : acétate d'éthyle-cyclohexane (75/25 en volumes)]. Les fractions contenant le produit attendu sont réunies et concentrées à sec sous pression réduite à 40°C. On obtient ainsi 2,32 g de (tert-butoxycarbonylamino-2 acétyl)-3 (tétrahydro-1,2,3,6 phényl-1-(RS))-2 thiazolidinecarboxylate-4 de tert-butyle-(2R,4R) sous forme d'une huile utilisée telle quelle dans les synthèses ultérieures.
D Le (tétrahydro-1,2,3,6 phényl-1-(RS))-2 thiazolidinecarboxylate de tert-butyle-(2RS,4R) peut être préparé d'une manière analogue à celle décrite à l'exemple 2C mais à partir de 8,0 g de tert-butoxycarbonyl-3 (tétrahydro-1,2,3,6 phényl-1-(RS))-2 thiazolidinecarboxylate-4 de tert-butyle (2R,4R) et de 3,57 cm³ d'iodotriméthylsilane. On obtient ainsi 4,43 g de (tétrahydro-1,2,3,6 phényl-1-(RS))-2 thiazolidinecarboxylate-4 de tert-butyle-(2RS, 4R) sous forme d'une huile jaune utilisée telle quelle dans les synthèses ultérieures.
E Le tert-butoxycarbonyl-3 (tétrahydro-1,2,3,6 phényl-1-(RS))-2 thiazolidine-carboxylate-4 de tert-butyle (2R,4R) peut être préparé d'une manière analogue à celle décrite à l'exemple 2D mais à partir de : 25,5 g d'acide tert-butoxycarbonyl-3 (tétrahydro-1,2,3,6 phényl-1-(RS))-2 thiazolidinecarboxylique (2R, 4R), de 15,5 g de chlorure de paratoluènesulfonyle et de 6,1 g de tert-butanol. On obtient ainsi 15,2 g de tert-butoxycarbonyl-3 (tétrahydro-1,2,3,6 phényl-1-(RS))-2 thiazolidinecarboxylate-4 de tert-butyle (2R,4R) sous forme d'une huile orangée utilisée telle quelle dans les synthèses ultérieures.
F L'acide tert-butoxycarbonyl-3 (tétrahydro-1,2,3,6 phényl-1-(RS))-2 thiazolidinecarboxylique (2R, 4R) peut être préparé d'une manière analogue à celle décrite à l'exemple 2E mais à partir de 16,7 g d'acide (tétrahydro-1,2,3,6 phényl-1-(RS))-2 thiazolidinecarboxylique (2RS, 4R), de 160 cm³ d'une solution aqueuse de soude 0,5 N et de 17,3 g de dicarbonate de ditert-butyle. On obtient ainsi 24,6 g d'acide tert-butoxycarbonyl-3 (tétrahydro-1,2,3,6 phényl-1-(RS))-2 thiazolidinecarboxylique (2R, 4R) utilisé tel quel dans les synthèses ultérieures.
G L'acide tétrahydro-1,2,3,6 phényl-1-(RS))-2 thiazolidinecarboxylique-4 (2RS,4R) peut être préparé d'une manière analogue à celle décrite à l'exemple 1E mais à partir de 12,1g de L-cystéine et de 11,0 g de tétrahydro-1,2,3,6 benzaldéhyde. On obtient ainsi 18,7 g d'acide tétrahydro-1,2,3,6 phényl-1-(RS))-2 thiazolidinecarboxylique-4 (2RS,4R) fondant à 236°C.
H L'isocyanato-3 phénylacétate de méthyle peut être préparé d'une manière analogue à celle décrite à l'exemple 1F mais à partir de 1 g de charbon, de 6 cm³ de diphosgène et de 8,25 g d'amino-3 phénylacétate de méthyle. On obtient ainsi 9,3 g d'isocyanato-3 phénylacétate de méthyle sous forme d'un liquide jaune conservé sous argon et utilisé tel quel dans les synthèses ultérieures.
I L'amino-3 phénylacétate de méthyle peut être préparé de la manière suivante : dans un monocol purgé à l'azote on introduit successivement 3,1 g de palladium à 5% sur charbon et une solution de 60,0 g de nitro-3 phénylacétate de méthyle dans 870 cm³ de méthanol. La suspension est agitée pendant 4 heures sous atmosphère d'hydrogène (130 kPa) à une température voisine de 25°C. Le catalyseur est séparé par filtration et le filtrat est concentré à sec sous pression réduite à 40°C. On obtient ainsi 51,0 g d'amino-3 phénylacétate de méthyle sous forme d'une huile orangée utilisée telle quelle dans les synthèses ultérieures.

Le nitro-3 phénylacétate de méthyle peut être préparé selon la méthode décrite par M. Segers et A. Bruylants, Bull. Soc. Chim. Belg., 64, 87 (1955).

### EXEMPLE 5

On opère comme à l'exemple 4 mais à partir de 0,27 g de {{[tert-butoxycarbonyl-4 [(norbornène-5 yl)-2-(2RS)]-2 thiazolidinyl-3]-2 oxo-2 éthyl)-3 uréido}-3 phénylacétate de méthyle-(4R) (mélange d'isomères A et B) et de 0,033 g d'hydroxyde de potassium. On obtient ainsi 0,09 g d'acide {{[tert-butoxycarbonyl-4 [(norbornène-5 yl)-2-(2RS)]-2 thiazolidinyl-31-2 oxo-2 éthyl}-3 uréido}-3 phénylacétique-(4R) (mélange d'isomères A et B) sous forme d'un produit blanc amorphe.

RMN (200 MHz, δ en ppm, DMSO D₆ + 2 gouttes de CD₃COOD, 393 K). 0,60 à 2,90 (mt, 7H, -CH- et -CH₂- du norbornènyl), 1,50 (s, 9H, C(CH₃)₃), 3,25 à 3,55 (mt, 2H, S-CH₂-), 3,50 (s, 2H, Ar-CH₂-COO-), 3,95 et 4,13 (2d, J = 17,0 Hz, 2H, N-CH₂-CO-), 4,96 (d, J = 11,0 Hz, 1H, -N-CH-S-), 5,02 (dd, J = 8,0 et 6,0 Hz, 1H, -N-CH-CO-) 6,23 (mt, 2H, -CH=CH- du norboményl), 6,83 (d, J = 8,0 Hz, 1H, H en position 4 de Ar-NH-CO), 7,15 (t, J = 8,0 Hz, 1H, H en position 5 de Ar-NH-CO-), 7,20 à 7,40 (mt, 2H de Ar-NH-CO-).

### EXEMPLE 6

On opère comme à l'exemple 1A mais à partir de 0,9 g d'(amino-2 acétyl)-3 [(norbornène-5 yl)-2-(2RS)]-2 thiazolidinecarboxylate-4 de tert-butyle-(4R) (mélange d'isomères A et B) et de 0,57 g d'isocyanato-3 phénylacétate de méthyle. Le produit brut est purifié par chromatographie sur silice [éluant : acétate d'éthyle-cyclohexane (40/60 en volumes)]. Les fractions contenant le produit attendu sont réunies et concentrées à sec sous pression réduite à 40°C. On obtient ainsi 0,4 g de (([tert-butoxycarbonyl-4 [(norbornène-5 yl)-2-(2RS)]-2 thiazolidinyl-3]-2 oxo-2 éthyl}-3 uréido}-3 phénylacétate de méthyle-(4R) (mélange d'isomères A et B) sous forme d'un produit amorphe.

RMN (200 MHz, δ en ppm, DMSO D₆ , 393 K). 0,50 à 2,90 (mt, 7H, -CH- et -CH₂- du norbornènyl), 1,50 (s, 9H, C(CH₃)₃), 3,20 à 3,60 (mt, 2H, S-CH₂-), 3,60 (s, 5H, Ar-CH₂-COOCH₃), 3,90 et 4,10 (2dd, J = 17,0 et 5,5 Hz, 2H, N-CH₂-CO-), 4,92 (d, J = 11,5 Hz, 1H, -N-CH-S-), 5,12 (dd, J = 8,0 et 6,0 Hz, 1H, -N-CH-CO-) 6,20 (mt, 2H, -CH=CH- du norbornènyl), 6,42 (t, J = 5,5 Hz, 1H NH-CO-), 6,80 (d, J = 8,0 Hz, 1H, H en position 4 de Ar-NH-CO), 7,17 (t, J = 8,0 Hz, 1H, H en position 5 de Ar-NH-CO-), 7,20 à 7,40 (mt, 2H de Ar-NH-CO-), 8 86 (s large, 1H, -CO-NH-Ar).

L'amino-2 acétyl)-3 [(norbornène-5 yl)-2-(2RS)]-2 thiazolidinecarboxylate-4 de tert-butyle-(4R) (mélange d'isomères A et B) peut être préparé d'une manière analogue à celle décrite à l'exemple 1B mais à partir de 0,95 g de (tert-butoxycarbonylamino-2 acétyl)-3 [(norbornène-5 yl)-2-(2RS)]-2 thiazolidinecarboxylate-4 de tert-butyle-(4R) (mélange d'isomères A et B) et de 0,32 cm³ d'iodotriméthylsilane. On obtient ainsi 0,7 g d'(amino-2 acétyl)-3 [(norbomène-5 yl)-2-(2RS)]-2 thiazolidinecarboxylate-4 de tert-butyle-(4R) (mélange d'isomères A et B) sous forme d'une huile orangée utilisée telle quelle dans les synthèses ultérieures.

Le (tert-butoxycarbonylamino-2 acétyl)-3 [(norbornène-5 yl)-2-(2RS)]-2 thiazolidinecarboxylate.4 de tert-butyle-(4R) (mélange d'isomères A et B) peut être préparé d'une manière analogue à celle décrite à l'exemple 1C mais à partir de 2,3 g de [(norbornène-5 yl)-2-(2RS)]-2 thiazolidinecarboxylate-4 de tert-butyle-(4R) (mélange d'isomères A et B), de 1,4 g d'acide tert-butoxycarbonylamino-2 acétique et de 1,68 g de N,N'-dicyclohexylcarbodiimide. Le produit brut est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi 1,5 g de (tert-butoxycarbonylamino-2 acétyl)-3 [(norbornène-5 yl)-2-(2RS)]-2 thiazolidinecarboxylate-4 de tert-butyle-(4R) (mélange d'isomères A et B) sous forme d'une huile jaune utilisée telle quelle dans les synthèses ultérieures.

Le [(norbornène-5 yl)-2-(2RS)]-2 thiazolidinecarboxylate-4 de tert-butyle-(4R) (mélange d'isomères A et B) peut être préparé d'une manière analogue à celle décrite à l'exemple 2C mais à partir de 3,0 g de tert-butoxycarbonyl-3 [(norbornène-5 yl)-2-(2RS)]-2 thiazolidinecarboxylate-4 de tert-butyle (4R) (mélange d'isomères A et B) et de 1,24 cm³ d'iodotriméthylsilane. On obtient ainsi 2,3 g de [(norbornène-5 yl)-2-(2RS)]-2 thiazolidinecarboxylate-4 de tert-butyle-(4R) (mélange d'isomères A et B) sous forme d'une huile jaune utilisée telle quelle dans les synthèses ultérieures.

Le tert-butoxycarbonyl-3 [(norbornène-5 yl)-2-(2RS)]-2 thiazolidinecarboxylate-4 de tert-butyle (4R) (mélange d'isomères A et B) peut être préparé d'une manière analogue à celle décrite à l'exemple 2D mais à partir de 46,0 g d'acide tert-butoxycarbonyl-3 [(norbornène-5 yl)-2-(2RS)]-2 thiazolidinecarboxylique-4-(4R) (mélange d'isomères), de 26,6 g de chlorure de paratoluènesulfonyle et de 10,5 g de tert-butanol. Le produit brut est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi 40,0 g de tert-butoxycarbonyl-3 [(norbornène-5 yl)-2-(2RS)]-2 thiazolidinecarboxylate-4 de tert-butyle (4R) (mélange d'isomères A,B,C et D) sous forme d'une huile orangée. Une deuxième chromatographie sur silice [éluant : acétate d'éthyle-cyclohexane (5/95 en volumes)] réalisée sur 6,0 g de ce mélange permet d'isoler 0,3 g de tert-butoxycarbonyl-3 [(norbornène-5 yl)-2-(2RS)]-2 thiazolidinecarboxylate-4 de tert-butyle-(4R) (mélange d'isomères A et B) (premiers produits d'élution) et 3,5 g de tert-butoxycarbonyl-3 [(norbomène-5 yl)-2-(2RS)]-2 thiazolidinecarboxylate-4 de tert-butyle-(4R) (mélange d'isomères C et D) sous forme d'huiles jaunes utilisées telles quelles dans les synthèses ultérieures.

L'acide tert-butoxycarbonyl-3 [(norbornène-5 yl)-2-(2RS)]-2 thiazolidinecarboxylique-4-(4R) (mélange d'isomères) peut être préparé d'une manière analogue à celle décrite à l'exemple 2E mais à partir de 40,0 g d'acide [(norbornène-5 yl)-2-(2RS)]-2 thiazolidinecarboxylique-4-(4R) (mélange d'isomères), de 360 cm³ d'une solution aqueuse de soude 0,5 N et de 39,0 g de dicarbonate de ditert-butyle. On obtient ainsi 46,0 g d'acide tert-butoxycarbonyl-3 [(norbornène-5 yl)-2-(2RS)]-2 thiazolidinecarboxylique-4-(4R) (mélange d'isomères) utilisé tel quel dans les synthèses ultérieures.

L'acide [(norbornène-5 yl)-2-(2RS)]-2 thiazolidinecarboxylique-4-(4R) (mélange d'isomères) peut être préparé d'une manière analogue à celle décrite à l'exemple 1E mais à partir de 23,0 g de L-cystéine et de 25,0 g de (norbornène-5 yl)-2-carboxaldéhyde-(RS) (mélanges d'isomères endo et exo). On obtient ainsi 40,0 g d'acide [(norbornène-5 yl)-2-(2RS)]-2 thiazolidinecarboxylique-4-(4R) (mélange d'isomères) fondant à 220°C et utilisé tel quel dans les synthèses ultérieures.

### EXEMPLE 7

On opère comme à l'exemple 4 mais à partir de 0,6 g de {{[tert-butoxycarbonyl-4 [(norbornène-5 yl)-2-(2RS)]-2 thiazolidinyl-3]-2 oxo-2 éthyl}-3 uréido}-3 phénylacétate de méthyle-(4R) (mélange d'isomères C et D) et de 0,074 g d'hydroxyde de potassium. On obtient ainsi 0,03 g d'acide {{tert-butoxycarbonyl [(norbornène-5 yl)-2-(2RS)]-2 thiazolidinyl-3]-2 oxo-2 éthyl}-3 uréido}-3 phénylacétique-(4R) (mélange d'isomères C et D) sous forme d'un produit blanc amorphe.

RMN (200 MHz, δ en ppm, DMSO D₆ + 2 gouttes de 2 gouttes de CD₃COOD, 393 K). de 1,00 à 1,90 et de 2,20 à 2,9 (mt, 7H, -CH- et -CH₂-du norbornènyl), 1,50 (s, 9H, C(CH₃)₃), 3,42 (AB, 2H, S-CH₂-), 3,48 (s, 2H, Ar-CH₂-COO), 4,02 (mt, 2H, N-CH₂-CO-), 4,97 (t, J = 7,5 Hz, 1H, -N-CH-CO-), 5,30 (mt, 1H, -N-CH-S-), de 6,00 à 6,40 (mt, 2H, -CH=CH- du norbornènyl), 6,81 (d, J = 8,0 Hz, 1H, H en position 4 de Ar-NH-CO), 7,12 (t, J = 8,0 Hz, 1H, H en position 5 de Ar-NH-CO-), 7,20 à 7,40 (mt, 2H de Ar-NH-CO-).

Le {{[tert-butoxycarbonyl-4 [(norbornène-5 yl)-2-(2RS)]-2 thiazolidinyl-3]-2 oxo-2 éthyl}-3 uréido}-3 phénylacétate de méthyle-(4R) (mélange d'isomères C et D) peut être préparé d'une manière analogue à celle décrite à l'exemple lA mais à partir de 0,77 g d'(amino-2 acétyl)-3 [(norbornène-5 yl)-2-(2RS)]-2 thiazolidinecarboxylate-4 de tert-butyle-(4R) (mélange d'isomères C et D) et de 0,57 g d'isocyanato-3 phénylacétate de méthyle. Le produit brut est purifié par chromatographie sur silice [éluant : acétate d'éthyle-cyclohexane (40/60 en volumes)]. Les fractions contenant le produit attendu sont réunies et concentrées à sec sous pression réduite à 40°C. On obtient ainsi 0,6 g de {{[tert-butoxycarbonyl-4 [(norbornène-5 yl)-2-(2RS)]-2 thiazolidinyl-3]-2 oxo-2 éthyl}-3 uréido}-3 phénylacétate de méthyle-(4R) (mélange d'isomères C et D) sous forme d'une huile jaune utilisée telle quelle dans les synthèses ultérieures.

L'amino-2 acétyl)-3 [(norbornène-5 yl)-2-(2RS)]-2 thiazolidinecarboxylate-4 de tert-butyle-(4R) (mélange d'isomères C et D) peut être préparé d'une manière analogue à celle décrite à l'exemple 1B mais à partir de 1,0 g de (tert-butoxycarbonylamino-2 acétyl)-3 [(norbornène-5 yl)-2-(2RS)]-2 thiazolidinecarboxylate-4 de tert-butyle-(4R) (mélange d'isomères Cet D) et de 0,36cm³ d'iodotriméthylsilane. On obtient ainsi 0,77 g d'(amino-2 acétyl)-3 [(norbornène-5 yl)-2-(2RS)]-2 thiazolidinecarboxylate-4 de tert-butyle-(4R) (mélange d'isomères C et D) sous forme d'une huile jaune utilisée telle quelle dans les synthèses ultérieures.

Le (tert-butoxycarbonylamino-2 acétyl)-3 [(norbornène-5 yl)-2-(2RS)]-2 thiazolidinecarboxylate-4 de tert-butyle-(4R) (mélange d'isomères C et D) peut être préparé d'une manière analogue à celle décrite à l'exemple 1C mais à partir de 2,8 g de [(norbornène-5 yl)-2-(2RS)]-2 thiazolidinecarboxylate-4 de tert-butyle-(4R) (mélange d'isomères C et D), de 1,75 g d'acide tert-butoxycarbonylamino-2 acétique et de 2,0 g de N,N'-dicyclohexylcarbodiimide. Le produit brut est purifié par chromatographie sur silice [éluant : acétate d'éthyle-cyclohexane (35/65 en volumes)]. Les fractions contenant le produit attendu sont réunies et concentrées à sec sous pression réduite à 40°C. On obtient ainsi 2,0 g de (tert-butoxycarbonylamino-2 acétyl)-3 [(norbornène-5 yl)-2-(2RS)]-2 thiazolidinecarboxylate-4 de tert-butyle-(4R) (mélange d'isomères C et D) sous forme d'une huile jaune utilisée telle quelle dans les synthèses ultérieures.

Le [(norbornène-5 yl)-2-(2RS)]-2 thiazolidinecarboxylate-4 de tert-butyle-(4R) (mélange d'isomères C et D) peut être préparé d'une manière analogue à celle décrite à l'exemple 2C mais à partir de 4,3 g de tert-butoxycarbonyl-3 [(norbornène-5 yl)-2-(2RS)]-2 thiazolidinecarboxylate-4 de tert-butyle (4R) (mélange d'isomères C et D) et de 4,2 cm³ d'iodotriméthylsilane. On obtient ainsi 2,8 g de [(norbornène-5 yl)-2-(2RS)]-2 thiazolidinecarboxylate-4 de tert-butyle-(4R) (mélange d'isomères C et D) sous forme d'une huile incolore utilisée telle quelle dans les synthèses ultérieures.

### EXEMPLE 8

On opère comme à l'exemple 1 mais à partir de 0,2 g de {{[tert-butoxycarbonyl-4 [(norbornène-5 yl)-2-(2RS)]-2 thiazolidinyl-3]-2 oxo-2 éthyl}-3 uréido}-3 phénylacétate de benzyle-(4R) (mélange d'isomères C et D), de 0,13 g de formiate d'ammonium et de 0,2 g de palladium sur charbon à 10%. On obtient ainsi 0,07 g d'acide {{[tert-butoxycarbonyl-4 (norbomyl-2-(2RS))-2 thiazolidinyl-3]-2 oxo-2 éthyl}-3 uréido}-3 phénylacétique-(4R) (mélange d'isomères C et D) sous forme d'un produit blanc fondant à 115°C.

RMN (200 MHz, δ en ppm, DMSO D₆ + 2 gouttes de CD₃COOD). On observe un mélange de rotamères et d'isomères. de 1,00 à 2,50 (mt, 11H, -CH-et -CH₂- du norbomyl), 1,35 à 1,50 (mt, 9H, C(CH₃)₃), 3,10 à 3,50 (mt, 2H, S-CH₂-), 3,50 (s, 2H, Ar-CH₂-COO), 3,80 à 4.30 (mt, 2H, N-CH₂-CO-), de 4,70 à 5,20 (mt, 1H, -N-CH-CO-), 5,10 à 5,70 (mt, 1H, -N-CH-S-), de 6,80 à 6,95 (mt, 1H, H en position 4 de Ar-NH-CO), 7,18 (t, J = 8,0 Hz, 1H, H en position 5 de Ar-NH-CO-), 7,30 à 7,45 (mt, 2H de Ar-NH-CO-)

Le {{[tert-butoxycarbonyl-4 [(norbornène-5 yl)-2-(2RS)]-2 thiazolidinyl-3]-2 oxo-2 éthyl}-3 uréido}-3 phénylacétate de benzyle-(4R) (mélange d'isomères C et D) peut être préparé d'une manière analogue à celle décrite à l'exemple lA mais à partir de 1,6 g d'(amino-2 acétyl)-3 [(norbornène-5 yl)-2-(2RS)]-2 thiazolidinecarboxylate-4 de tert-butyle-(4R) (mélange d'isomères C et D) et de 1,26 g d'isocyanato-3 phénylacétate de benzyle. Le produit brut est purifié par chromatographie sur silice [éluant : acétate d'éthyle-oxyde de diisopropyle (10/90 en volumes)]. Les fractions contenant le produit attendu sont réunies et concentrées à sec sous pression réduite à 40°C. On obtient ainsi 0,2 g de {{[tert-butoxycarbonyl-4 [(norbornène-5 yl)-2-(2RS)]-2 thiazolidinyl-3]-2 oxo-2 éthyl}-3 uréido}-3 phénylacétate de benzyle-(4R) (mélange d'isomères C et D) sous forme d'un produit amorphe utilisé tel quel dans les synthèses ultérieures.

### EXEMPLE 9

On opère comme à l'exemple 1A mais à partir de 0,35 g d'(amino-2 acétyl)-3 [(norbornène-5 yl)-2-(2RS)]-2 thiazolidinecarboxylate-4 de tert-butyle-(4R) (mélange d'isomères) et de 0,14 g d'isocyanate de méthyl-3 phényle. Le produit brut est purifié par chromatographie sur silice [éluant : chlorure de méthylène-méthanol (99/1 en volumes)]. Les fractions contenant le produit attendu sont réunies et concentrées à sec sous pression réduite à 40°C. On obtient ainsi, après recristallisation dans l'oxyde de diisopropyle, 0,1 g de {[(méthyl-3 phényl)-3 uréido]-2 acétyl}-3 [(norbomène-5 yl-2-(RS)]-2 thiazolidinecarboxylate de tert-butyle-(4R) (mélange d'isomères) sous forme d'un solide blanc fondant à 114°C.

RMN (200 MHz, δ en ppm, DMSO D₆ + 2 gouttes de CD₃COOD). 0,40 à 1,90 et de 2,20 à 2,90 (mt, 7H, -CH- et -CH₂- du norbornènyl), 1,58 (s, 9H, C(CH₃)₃), 2,27 (s, 3H, Ar-CH₃), 3,44 (AB, 2H, S-CH₂-), 3,92 et 4,12 (2d, J = 17,0 Hz, 2H, N-CH₂-CO-), 4,93 (d, J = 11,5 Hz, 1H, -N-CH-S-), 5,12 (mt, 1H, -N-CH-CO-), 6,22 (mt, 2H, -CH=CH- du norbomènyl), 6,74 (d, J = 8,0 Hz, 1H, H en position 4 de Ar-NH-CO), 7,12 (t, J = 8,0 Hz, 1H, H en position 5 de Ar-NH-CO-), 7,15 à 7,35 (mt, 2H de Ar-NH-CO-).

L'(amino-2 acétyl)-3 [(norbornène-5 yl)-2-(2RS)]-2 thiazolidinecarboxylate-4 de tert-butyle-(4R) (mélange d'isomères) peut être préparé comme à l'exemple 1B mais à partir de 0,70 g de (tert-butoxycarbonylamino-2 acétyl)-3 [(norbomène-5 yl)-2-(2RS)]-2 thiazolidinecarboxylate-4 de tert-butyle-(4R) (mélange d'isomères) et de 0,23 cm³ d'iodotriméthylsilane. On obtient ainsi 0,35 g d'(amino-2 acétyl)-3 [(norbornène-5 yl)-2-(2RS)]-2 thiazolidinecarboxylate-4 de tert-butyle-(4R) (mélange d'isomères) sous forme d'une huile orangée utilisée telle quelle dans les synthèses ultérieures.

Le (tert-butoxycarbonylamino-2 acétyl)-3 [(norbornène-5 yl)-2-(2RS)]-2 thiazolidinecarboxylate-4 de tert-butyle-(4R) (mélange d'isomères) peut être préparé d'une manière analogue à celle décrite à l'exemple 1C mais à partir de 4,75 g de [(norbornène-5 yl)-2-(2RS)]-2 thiazolidinecarboxylate-4 de tert-butyle-(4R) (mélange d'isomères), de 2,96 g d'acide tert-butoxycarbonylamino-2 acétique et de 3,48 g de N,N'-dicyclohexylcarbodiimide. Le produit brut est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi 1,36 g de (tert-butoxycarbonylamino-2 acétyl)-3 [(norbornène-5 yl)-2-(2RS)]-2 thiazolidinecarboxylate-4 de tert-butyle-(4R) (mélange d'isomères) sous forme d'un produit amorphe jaune-orangé utilisé tel quel dans les synthèses ultérieures.

Le [(norbornène-5 yl)-2-(2RS)]-2 thiazolidinecarboxylate-4 de tert-butyle-(4R) (mélange d'isomères) peut être préparé d'une manière analogue à celle décrite à l'exemple 2C mais à partir de 6,3 g de tert-butoxycarbonyl-3 [(norbornène-5 yl)-2-(2RS)]-2 thiazolidinecarboxylate-4 de tert-butyle-(4R) (mélange d'isomères) et de 2,41 cm³ d'iodotriméthylsilane. On obtient ainsi 4,75 g de [(norbornène-5 yl)-2-(2RS)]-2 thiazolidinecarboxylate-4 de tert-butyle-(4R) (mélange d'isomères) sous forme d'une huile jaune utilisée telle quelle dans les synthèses ultérieures.

### EXEMPLE 10

On opère comme à l'exemple 1 mais à partir de 0,57 g de {[(tert-butoxycarbonyl-4 tert-butyl-2 thiazolidinyl-3)-2 oxo-2 éthyl]-3 uréido}-3 phénylacétate de benzyle-(4R) (isomère A), de 0,50 g de formiate d'ammonium et de 0,5 g de palladium sur charbon à 10%. On obtient ainsi 0,07 g d'acide ((tert-butoxycarbonyl-4 tert-butyl-2 thiazolidinyl-3)-2 oxo-2 éthyl]-3 uréido}-3 phénylacétique-(4R) (isomère A) sous forme d'un produit amorphe.

RMN (200 MHz, δ en ppm, DMSO D₆). 0,92 (s, 9H, C(CH₃)₃), 1,48 (s, 9H, COOC(CH₃)₃), de 3,20 à 3,65 (mt, 2H, S-CH₂-), 3,34 (s, 2H, Ar-CH₂COO), 3,90 et 4,10 (2dd, J = 17,0 et 5,5 Hz, 2H, N-CH₂-CO-), 5,14 (t, J = 7,5 Hz, 1H, -N-CH-CO-), 5,40 (s, 1H, -N-CH-S-), 6,50 (t, J = 5,5 Hz, 1H, -N-CH-CO), 6,80 (d, J = 8,0 Hz, 1H, H en position 4 de Ar-NH-CO), 7,15 (t, J = 8,0 Hz, 1H, H en position 5 de Ar-NH-CO-), 7,20 à 7,30 (mt, 2H de Ar-NH-CO-), 8,90 (s, 1 H, -CO-NH-Ar).

Le {[(tert-butoxycarbonyl-4 tert-butyl-2 thiazolidinyl-3)-2 oxo-2 éthyl]-3 uréido}-3 phénylacétate de benzyle-(4R) (isomère A) peut être préparé d'une manière analogue à celle décrite à l'exemple lA mais à partir de 0,9 g d'(amino-2 acétyl)-3 tert-butyl-2 thiazolidinecarboxylate-4 de tert-butyle-(4R) (isomère A) et de 1,18 g d'isocyanato-3 phénylacétate de benzyle. Le produit brut est purifié par chromatographie sur silice [éluant : acétate d'éthyle-chlorure de méthylène (10/90 en volumes)]. Les fractions contenant le produit attendu sont réunies et concentrées à sec sous pression réduite à 40°C. On obtient ainsi 0,81 g de ([(tert-butoxycarbonyl-4 tert-butyl-2 thiazolidinyl-3)-2 oxo-2 éthyl]-3 uréido}-3 phénylacétate de benzyle-(4R) (isomère A) sous forme d'une huile épaisse utilisée telle quelle dans les synthèses ultérieures.

L'(amino-2 acétyl)-3 tert-butyl-2 thiazolidinecarboxylate-4 de tert-butyle-(4R) (isomère A) peut être préparé comme à l'exemple 1B mais à partir de 2,0 g de (tert-butoxycarbonylamino-2 acétyl)-3 tert-butyl-2 thiazolidinecarboxylate-4 de tert-butyle-(4R) (isomère A) et de 0,75 cm³ d'iodotriméthylsilane. On obtient ainsi 0,9 g d'(amino-2 acétyl)-3 tert-butyl-2 thiazolidinecarboxylate.4 de tert-butyle-(4R) (isomère A) sous forme d'une huile jaune utilisée telle quelle dans les synthèses ultérieures.

Le (tert-butoxycarbonylamino-2 acétyl)-3 tert-butyl-2 thiazolidinecarboxylate-4 de tert-butyle-(4R) (isomère A) peut être préparé d'une manière analogue à celle décrite à l'exemple 1C mais à partir de 8,0 g de tert-butyl-2 thiazolidinecarboxylate-4 de tert-butyle-(2RS, 4R), de 5,71 g d'acide tert-butoxycarbonylamino-2 acétique et de 6,7 g de N,N'-dicyclohexylcarbodiimide. Le produit brut est purifié par chromatographie sur silice [éluant : acétate d'éthyle-cyclohexane (20/80 en volumes)]. Les fractions contenant le produit attendu sont réunies et concentrées à sec sous pression réduite à 40°C. On obtient ainsi 2,0 g de (tert-butoxycarbonylamino-2 acétyl)-3 tert-butyl-2 thiazolidinecarboxylate-4 de tert-butyle-(4R) (isomère A) sous forme d'une huile incolore utilisée telle quelle dans les synthèses ultérieures.

Le tert-butyl-2 thiazolidinecarboxylate-4 de tert-butyle-(2RS, 4R) peut être préparé d'une manière analogue à celle décrite à l'exemple 1D mais à partir de 10,0 g d'acide tert-butyl-2 thiazolidinecarboxylique-4 (2RS, 4R) en solution dans 200 cm³ de chloroforme, de 5 cm³ d'acide sulfurique concentré et d'un excès d'isobutène. On obtient ainsi 13,0 g de tert-butyl-2 thiazolidinecarboxylate-4 de tert-butyle-(2RS, 4R) sous forme d'une huile jaune utilisée telle quelle dans les synthèses ultérieures.

L'acide tert-butyl-2 thiazolidinecarboxylique-4 (2RS, 4R) peut être préparé d'une manière analogue à celle décrite à l'exemple à l'exemple 1E mais à partir de 7,6 g de L-cystéine et de 5,17 g de pivalaldéhyde. On obtient ainsi 10,4 g d'acide tert-butyl-2 thiazolidinecarboxylique-4 (2RS, 4R) fondant à 184°C.

### EXEMPLE 11

On opère d'une manière analogue à celle décrite à l'exemple 1 mais à partir de 1,36 g de {{[(benzyloxycarbonyléthyl-1)-3 phényl-(S)]-3 uréido}-2 acétyl}-3 butyl-2 thiazolidinecarboxylate de tert-butyle-(4R) (isomère A), 0,9 g de formiate d'ammonium et 1,3 g de palladium sur charbon à 10%. On obtient ainsi 0,65 g d'acide {[(tert-butoxycarbonyl-4 butyl-2 thiazolidinyl-3-(4R))-2 oxo-2 éthyl]-3 uréido}-3 phényl-2 propionique-(S) (isomère A) fondant à 102°C; [α]_{D}²⁵ = -1,1° ± 0,8° (C% = 0,53; DMF).

RMN (200 MHz, δ en ppm, DMSO D₆ + 2 gouttes de CD₃COOD). 0,90 (t, 3H : -CH₂-CH₃), 1,40 (mt, 7H : -CH₂-CH₂-CH₃ et -CH₃), 1,50 [s, 9H : -COOC(CH₃)₃], de 1,50 à 2,15 (mt, 2H : -CH₂-), de 3,20 à 3,50 (mt, 2H : -S-CH₂-), 3,67 (q, 1H : Ar-CH-COO-), de 3,95 à 4,10 (mt, 2H : N-CH₂-CO-), 4,97 (t, 1H : N-CH-CO-), 5,35 (dd, 1H : N-CH-S-), 6,25 (mf résiduel : -NH-CO-), 6,90 [d large, 1H : Ar (-H 5)], 7,19 [t, 1H : Ar (-H 5)], de 7,25 à 7,40 [mt, 2H : Ar (-H2 et -H6)], 8,50 (mf résiduel: Ar-NH-CO-).
A Le {{[((benzyloxycarbonyléthyl-1)-3 phényl-(S)]-3 uréido}-2 acétyl}-3 butyl-2 thiazolidinecarboxylate de tert-butyle-(4R) (isomère A) peut être préparé d'une manière analogue à celle décrite à l'exemple 1A mais à partir de 2,4 g d'(amino-2 acétyl)-3 butyl-2 thiazolidinecarboxylate-4 de tert-butyle-(4R) (isomere A) et de 2,3 g d'(isocyanato-3 phényl)-2 propionate de benzyle-(S). Le produit brut obtenu est purifié par chromatographie sur silice [éluant : oxyde de diisopropyle-acétate d'éthyle (95/5 en volumes)]. Les fractions contenant le produit attendu sont réunies et concentrées à sec sous pression réduite à 40°C. On obtient ainsi 2,0 g de {{[(benzyloxycarbonyléthyl-1)-3 phényl-(S)]-3 uréido}-2 acétyl}-3 butyl-2 thiazolidinecarboxylate de tert-butyle-(4R) (isomère A) sous forme d'un produit amorphe utilisé tel quel dans les synthèses ultérieures.
B L' (amino-2 acétyl)-3 butyl-2 thiazolidinecarboxylate-4 de tert-butyle-(4R) (isomère A) peut être préparé d'une manière analogue à celle décrite à l'exemple 1B mais à partir de 10,0 g de (tert-butoxycarbonylamino-2 acétyl)-3 butyl-2 thiazolidinecarboxylate-4 de tert-butyle-(4R) (isomère A) et de 4,0 cm³ d'iodotriméthylsilane. On obtient ainsi 5,9 g d'(amino-2 acétyl)-3 butyl-2 thiazolidinecarboxylate-4 de tert-butyle-(4R) (isomère A) sous forme d'une huile jaune utilisée telle quelle dans les synthèses ultérieures.
C Le (tert-butoxycarbonylamino-2 acétyl)-3 butyl-2 thiazolidinecarboxylate-4 de tert-butyle-(4R) (isomère A) peut être préparé d'une manière analogue à celle décrite à l'exemple 1C mais à partir de 25,0 g de butyl-2 thiazolidine-carboxylate-4 de tert-butyle-(2RS,4R), de 12,3 g d'acide tert-butoxycarbonylamino-2 acétique et de 14,4 g de N,N'-dicyclohexylcarbodiimide. Le produit brut est purifié par chromatographie sur silice [éluant : chlorure de méthylène-méthanol (99/1 en volumes)]. Les fractions contenant le produit attendu sont réunies et concentrées à sec sous pression réduite à 40°C. On obtient ainsi 12,5 g de (tert-butoxycarbonylamino-2 acétyl)-3 butyl-2 thiazolidinecarboxylate-4 de tert-butyle-(4R) (isomère A) sous forme d'une meringue crème utilisée telle quelle dans les synthèses ultérieures.
D Le butyl-2 thiazolidinecarboxylate-4 de tert-butyle-(2RS,4R) peut être préparé d'une manière analogue à celle décrite à l'exemple 1D mais à partir de 50,0 g d'acide butyl-2 thiazolidinecarboxylique-4-(2RS,4R) en suspension dans 650 cm³ de chloroforme, de 13 cm³ d'acide sulfurique concentré et d'un excès d'isobutène. On obtient ainsi 25,0 g de butyl-2 thiazolidinecarboxylate-4 de tert-butyle-(2RS,4R) sous forme d'une huile jaune utilisée telle quelle dans les synthèses ultérieures.
E L'acide butyl-2 thiazolidine carboxylique-4-(2RS,4R) peut être préparé d'une manière analogue à celle décrite à l'exemple lE mais à partir de 39,0 g de L-cystéine et de 30,1 g de valéraldéhyde. On obtient ainsi 52,4 g d'acide butyl-2 thiazolidinecarboxylique-4-(2RS,4R) fondant à 195 °C et utilisé tel quel dans les synthèses ultérieures.
F L'(isocyanato-3 phényl)-2 propionate de benzyle-(S) peut être préparé comme à l'exemple 1F mais à partir de 2,85 g d'(amino-3 phényl)-2 propionate de benzyle-(S), de 1,48 cm³ de chloroformiate de trichlorométhyle et de 0,24 g de charbon. On obtient ainsi 3,1 g d'(isocyanato-3 phényl)-2 propionate de benzyle-(S) sous forme d'une huile utilisée telle quelle dans les synthèses ultérieures.
G L'(amino-3 phényl)-2 propionate de benzyle-(S) peut être préparé de la manière suivante : à une solution de 8,0 g de (nitro-3 phényl)-2 propionate de benzyle-(S) dans un mélange de 35 cm³ de méthanol et de 300 cm³ d'eau, on ajoute 75 g de chlorure d'ammonium et 37,0 g de zinc en poudre. Le milieu réactionnel est chauffé à reflux pendant une heure puis refroidi à une température voisine de 0°C et filtré. Le filtrat est extrait par trois fois 200 cm³ d'oxyde de diéthyle. Les phases organiques sont réunies, lavées successivement par 100 cm³ d'eau puis 100 cm³ d'une solution aqueuse saturée de chlorure de sodium, séchées sur sulfate de magnésium et concentrées sous pression réduite. On obtient ainsi 6,7 g d'(amino-3 phényl)-2 propionate de benzyle-(S) sous forme d'une huile jaune utilisée telle quelle dans les synthèses ultérieures.
H Le (nitro-3 phényl)-2 propionate de benzyle-(S) peut être préparé de la manière suivante : à un mélange contenant 9,75 g d'acide (nitro-3 phényl)-2 propionique-(S) et 0,5 cm³ de N,N-diméthyl-formamide dans 100 cm³ de dichloro-1,2 éthane, on ajoute 4,72 cm³ de chlorure d'oxalyle. Le milieu réactionnel est agité pendant trois heures à une température voisine de 25°C, puis on ajoute 5,4 g d'alcool benzylique. L'agitation est poursuivie 12 heures à cette même température puis le mélange réactionnel est lavé successivement par 2 fois 200 cm³ d'une solution aqueuse saturée d'hydrogénocarbonate de sodium, 100 cm³ d'eau et 100 cm³ d'une solution aqueuse saturée de chlorure de sodium. Les phases organiques sont réunies, lavées successivement par 100 cm³ d'eau puis 100 cm³ d'une solution aqueuse saturée de chlorure de sodium, séchées sur sulfate de magnésium et concentrées sous pression réduite. Le résidu est purifié par chromatographie sur silice [éluant : acétate d'éthyle-cyclohexane (30/70 en volumes)]. Les fractions contenant le produit attendu sont réunies et concentrées sous pression réduite. On obtient ainsi 11,5 g de (nitro-3 phényl)-2 propioniate de benzyle-(S) sous forme d'une huile jaune utilisée telle quelle dans les synthèses ultérieures.
I L'acide (nitro-3 phényl)-2 propionique-(S) peut être préparé de la manière suivante : une solution de 21,5 g de (nitro-3 phényl)-2 N-[hydroxy-2 phényl-1 éthyl-(R)]-propionamide-(S) dans un mélange de 450 cm³ de dioxanne-1,4 et de 450 cm³ d'une solution aqueuse 4N d'acide chlorhydrique est chauffée à une température voisine de 80°C pendant cinq heures, puis agitée douze heures à une température voisine de 20°C. Le milieu réactionnel est concentré de moitié par évaporation sous pression réduite, dilué par addition de 500 cm³ d'eau distillée et extrait par deux fois 500 cm³ d'oxyde de diéthyle. Les phases organiques sont réunies, lavées successivement par trois fois 250 cm³ d'eau puis 250 cm³ d'une solution aqueuse saturée de chlorure de sodium, séchées sur sulfate de magnésium et concentrées sous pression réduite. On obtient ainsi 14 g d'acide (nitro-3 phényl)-2 propionique-(S) sous forme d'un solide crème utilisé tel quel dans les synthèses ultérieures.
J Le (nitro-3 phényl)-2 N-[hydroxy-2 phényl-1 éthyl-(R)]-propionamide-(S) peut être préparé de la manière suivante : à un mélange contenant 39,0 g d'acide (nitro-3 phényl)-2 propionique-(RS) et 0,5 cm³ de N,N-diméthyl-formamide dans 400 cm³ de dichloro-1,2 éthane, on ajoute lentement 17,2 cm³ de chlorure d'oxalyle. Le milieu réactionnel est agité pendant trois heures à une température voisine de 20°C puis concentré sous pression réduite. Le résidu est dissous dans 150 cm³ de dichloro-1,2 éthane et ajouté à une solution de 27,4 g de phényl-2 glycinol-(R) en maintenant la température du milieu réactionnel en dessous de 10°C. Le mélange réactionnel est agité pendant douze heures à une température voisine de 20°C puis est lavé successivement par 1000 cm³ d'eau distillée, 500 cm³ d'une solution aqueuse normale d'acide chlorhydrique, deux fois 500 cm³ d'eau distillée et 500 cm³ d'une solution aqueuse saturée de chlorure de sodium. La phase organique est séchée sur sulfate de magnésium et concentrée sous pression réduite. Les deux diastéréoisomères obtenus sont séparés par chromatographie sur silice [éluant : chlorure de méthylène-acétate d'éthyle (70/30 en volumes)]. Les fractions contenant chacun des deux diastéréoisomères sont réunies et concentrées sous pression réduite. On obtient ainsi 21,0 g de (nitro-3 phényl)-2 N-[hydroxy-2 phényl-1 éthyl-(R)]-propionamide-(R) (premier produit d'élution) fondant à 135°C et 19,0 g de (nitro-3 phényl)-2 N-[hydroxy-2 phényl-1 éthyl-(R)]-propionamide-(S) (deuxième produit d'élution) fondant à 150°C.

L'acide (nitro-3 phényl)-2 propionique-(RS) peut être préparé selon la méthode décrite par E. FELDER et coll. J. Med. Chem., 13, 559 (1970).

### EXEMPLE 12

On opère d'une manière analogue à celle décrite à l'exemple lA mais à partir de 1,2 g d'(amino-2 acétyl)-3 butyl-2 thiazolidinecarboxylate-4 de tert-butyle-(4R) (isomère A) et de 0,53 g d'isocyanate de méthyl-3 phényle. Le produit brut est purifié par chromatographie sur silice [éluant : oxyde de diisopropyle-acétate d'éthyle (95/5 en volumes)]. Les fractions contenant le produit attendu sont réunies et concentrées à sec sous pression réduite à 40°C. On obtient ainsi 0,8 g de ([(méthyl-3 phényl)-3 uréido]-2 acétyl}-3 butyl-2 thiazolidinecarboxylate-4 de tert-butyle-(4R) (isomère A) sous forme d'un produit amorphe; [α]_{D}²⁵ = -39° ± 1° (C% = 0,53; DMF).

RMN (200 MHz, δ en ppm, DMSO D₆ + 2 gouttes de CD₃COOD, 373 K). 0,90 (t, 3H : CH₃), 1,38 (mt, 4H : CH₂-CH₂-CH₃),1,50 [s, 9H : -COOC(CH₃)₃], de 1,50 à 2,15 (mt, 2H : -CH₂-), 2,30 (s, 3H : Ar-CH₃), de 3,15 à 3,50 (mt, 2H : -S-CH₂-), de 3,90 à 4,10 (mt, 2H : N-CH₂-CO-), 4,95 (t, 1H : -CH-CO-), 5,30 (dd, 1H : N-CH-S-), 6,25 (mf résiduel: -NH-CO-), 6,75 [d large, 1H : Ar (-H 4)], 7,10 [t, 1H : Ar (-H 5)], de 7,15 à 7,25 [mt, 2H: Ar (-H 2 et -H 6)].

### EXEMPLE 13

On opère d'une manière analogue à celle décrite à l'exemple 1 mais à partir de 1,18 g de {[(tert-butoxycarbonyl-4 butyl-2 thiazolidinyl-3)-2 oxo-2 éthyl]-3 uréido}-3 benzoate de benzyle-(4R) (isomère A), de 0,8 g de formiate d'ammonium et 1,2 g de palladium sur charbon à 10%. On obtient ainsi 0,25 g d'acide {[(tert-butoxycarbonyl-4 butyl-2 thiazolidinyl-3)-2 oxo-2 éthyl]-3 uréido}-3 benzoïque-(4R) (isomère A) sous forme d'un produit amorphe; [α]_{D}²⁵ = -35,4° ± 1,0° (C% = 0,52; DMF).

RMN (200 MHz, δ en ppm, DMSO D₆ + 2 gouttes de CD₃COOD, 373 K). 0,92 (t, 3H : CH₃), 1,40 (mt, 4H : -CH₂-CH₂-CH₃), 1,52 [s, 9H : -COOC(CH₃)₃], de 1,50 à 2,15 (mt, 2H : -CH₂-), de 3,20 à 3,50 (mt, 2H : -S-CH₂-), de 3,90 à 4,10 (mt, 2H : N-CH₂-CO-), 4,95 (t, 1H : N-CH-CO-), 5,32 (dd, 1H : N-CH-S-), 6,30 (mf résiduel: -NH-CO-), 7,35 [t, 1H : Ar (-H 5)], 7,52 et 7,62 [2d larges, 1H chacun : Ar (.H 4 et .H 6)], 8,05 [s large, 1H : Ar (-H 2)], 8,70 (mf résiduel : Ar-NH-CO-).
A Le {[(tert-butoxycarbonyl-4 butyl-2 thiazolidinyl-3)-2 oxo-2 éthyl]-3 uréido}-3 benzoate de benzyle-(4R) (isomère A) peut être préparé d'une manière analogue à celle décrite à l'exemple 1A mais à partir de 2,4 g d'(amino-2 acétyl)-3 butyl-2 thiazolidinecarboxylate-4 de tert-butyle-(4R) (isomère A) et de 2,3 g d'isocyanato-3 benzoate de benzyle. Le produit brut obtenu est purifié par chromatographie sur silice [éluant : oxyde de diisopropyle-acétate d'éthyle (95/5 en volumes)]. Les fractions contenant le produit attendu sont réunies et concentrées à sec sous pression réduite à 40°C. On obtient ainsi 1,6 g de {[(tert-butoxycarbonyl-4 butyl-2 thiazolidinyl-3)-2 oxo-2 éthyl]-3 uréido)-3 benzoate de benzyle-(4R) (isomère A) sous forme d'un produit amorphe utilisé tel quel dans les synthèses ultérieures.

### EXEMPLE 14

On opère d'une manière analogue à celle décrite à l'exemple lA mais à partir de 2,51 g d' (amino-2 acétyl)-3 (tétrahydro-1,2,3,6 phényl-1-(RS))-2 thiazolidinecarboxylate-4 de tert-butyle-(2R, 4R) et de 1,06 g d'isocyanate de méthyl-3 phényle. Le produit brut obtenu est purifié par chromatographie sur silice [éluant : chlorure de méthylène-acétate d'éthyle (90/10 en volumes)]. Les fractions contenant le produit attendu sont réunies et concentrées à sec sous pression réduite à 40°C. On obtient ainsi 1,74 g de {[(méthyl-3 phényl)-3 uréido]-2 acétyl}-3 (tétrahydro-1,2,3,6 phényl-1-(RS))-2 thiazolidine-carboxylate-4 de tert-butyle-(2R, 4R) sous forme d'un produit amorphe.

RMN (250 MHz, δ en ppm, DMSO D₆, 350 K). On observe le mélange des deux diastéréoisomères 50/50, de 1,10 à 1,50 et de 1,80 à 2,30 (mt, 7H : CH- et -CH₂- du tétrahydro-1,2,5,6 phényl), 1,47 et 1,49 [2s, 9H en totalité: -COOC(CH₃)₃], 2,25 (s, 3H : Ar-CH₃), de 3,25 à 3,45 (mt, 2H : -S-CH₂-), 4,08 (mt, 2H : N-CH₂-CO-), 5,00 (t large, 1H : N-CH-CO-), 5,30 (mt, 1H : N-CH-S-), 5,66 (mt, 2H : -CH=CH- du tétrahydro-1,2,5,6 phényl), 6,75 [d large, 1H Ar (-H 4)], 7,10 [t, 1H Ar (-H 5)], de 7,15 à 7,25 [mt, 2H : Ar (-H 2 et -H 6)].

### EXEMPLE 15

On opère d'une manière analogue à celle décrite à l'exemple 1 mais à partir de 1,36 g de {{[tert-butoxycarbonyl-4 (phényl-2 phényl)-2 thiazolidinyl-3]-2 oxo-2 éthyl}-3 uréido}-3 phénylacétate de benzyle-(2R, 4R), 0,44g de formiate d'ammonium et 0,6 g de palladium sur charbon à 10%. On obtient ainsi 0,25 g d'acide {{[tert-butoxycarbonyl-4 (phényl-2 phényl)-2 thiazolidinyl-3]-2 oxo-2 éthyl}-3 uréido}-3 phénylacétique-(2R, 4R) fondant à 128°C; [α]_{D}²⁵ = -26,2° ± 0,9° (C% = 0,56; MeOH).

RMN (200 MHz, δ en ppm, DMSO D₆ + 2 gouttes de CD₃COOD, 393 K) 1,60 [s, 9H : -COOC(CH₃)₃], 3,30 et 3,38 (2dd, 1H chacun: -CH₂-S-), 3,49 (s, 2H : Ar-CH₂-COO-), de 3,60 à 3,75 et 3,86 (respectivement mf et d, 1H chacun: N-CH₂-CO-), 4,95 (dd, 1H : N-CH-COO-), 6,08 (s, 1H : N-CH-S-), 6,14 (mf résiduel : -CO-NH-), 6,85 [d, 1H : Ar (-H 4)], 7,15 [t, 1H : Ar (-H 5)], de 7,20 à 7,35 [mt, 2H : Ar (-H 2 et -H 6)], de 7,20 à 7,60 et 8,09 (respectivement mt et d large, 8H et 1H : -H aromatiques), 8,40 (s large résiduel: -CO-NH-Ar).
A Le {{[tert-butoxycarbonyl-4 (phényl-2 phényl)-2 thiazolidinyl-3]-2 oxo-2 éthyl}-3 uréido}-3 phénylacétate de benzyle-(2R, 4R) peut être préparé d'une manière analogue à celle décrite à l'exemple lA mais à partir de 2,64 g d'(amino-2 acétyl)-3 (phényl-2 phényl)-2 thiazolidinecarboxylate-4 de tert-butyle-(2R,4R) et de 3,54 g d'isocyanato-3 phénylacétate de benzyle. Le produit brut obtenu est purifié par chromatographie sur silice [éluant : chlorure de méthylène-acétate d'éthyle (90/10 en volumes)]. Les fractions contenant le produit attendu sont réunies et concentrées à sec sous pression réduite à 40°C. On obtient ainsi 0,87 g de {{[tert-butoxycarbonyl-4 (phényl-2 phényl)-2 thiazolidinyl-3]-2 oxo-2 éthyl}-3 uréido}-3 phénylacétate de benzyle-(2R, 4R) sous forme d'un produit amorphe utilisé tel quel dans les synthèses ultérieures.
B L' (amino-2 acétyl)-3 (phényl-2 phényl)-2 thiazolidinecarboxylate-4 de tert-butyle-(2R,4R) peut être préparé d'une manière analogue à celle décrite à l'exemple 1B mais à partir de 4,0 g de (tert-butoxycarbonylamino-2 acétyl)-3 (phényl-2 phényl)-2 thiazolidinecarboxylate-4 de tert-butyle-(2R,4R) et de 1,43 cm³ d'iodotriméthylsilane. On obtient ainsi 3,9 g d'(amino-2 acétyl)-3 (phényl-2 phényl)-2 thiazolidinecarboxylate-4 de tert-butyle-(2R,4R) sous forme d'une huile jaune utilisée telle quelle dans les synthèses ultérieures.
C Le (tert-butoxycarbonylamino-2 acétyl)-3 (phényl-2 phényl)-2 thiazolidine-carboxylate-4 de tert-butyle-(2R,4R) peut être préparé d'une manière analogue à celle décrite à l'exemple 1C mais à partir de 5,44 g de (phényl-2 phényl)-2 thiazolidinecarboxylate-4 de tert-butyle-(2RS,4R), de 2,8 g d'acide tert-butoxycarbonylamino-2 acétique et de 3,3 g de N,N'-dicyclohexylcarbodiimide. Le produit brut est purifié par chromatographie sur silice [éluant : cyclohexane-acétate d'éthyle (80/20 en volumes)]. Les fractions contenant le produit attendu sont réunies et concentrées à sec sous pression réduite à 40°C. On obtient ainsi 4,42 g de (tert-butoxycarbonylamino-2 acétyl)-3 (phényl-2 phényl)-2 thiazolidinecarboxylate-4 de tert-butyle-(2R,4R) sous forme d'une meringue blanche utilisée telle quelle dans les synthèses ultérieures.
D Le (phényl-2 phényl)-2 thiazolidinecarboxylate-4 de tert-butyle-(2RS,4R) peut être préparé d'une manière analogue à celle décrite à l'exemple 1D mais à partir de 6,0 g d'acide (phényl-2 phényl)-2 thiazolidinecarboxylique-4-(2RS,4R) en suspension dans 100 cm³ de chloroforme, 3 cm³ d'acide sulfurique concentré et d'un excès d'isobutène. Le produit brut est purifié par chromatographie sur silice [éluant : cyclohexane-acétate d'éthyle (80/20 en volumes)]. Les fractions contenant le produit attendu sont réunies et concentrées à sec sous pression réduite à 40°C. On obtient ainsi 6,1 g de (phényl-2 phényl)-2 thiazolidinecarboxylate-4 de tert-butyle-(2RS,4R) sous forme d'une huile jaune utilisée telle quelle dans les synthèses ultérieures.
E L'acide (phényl-2 phényl)-2 thiazolidinecarboxylique-4-(2RS,4R) peut être préparé d'une manière analogue à celle décrite à l'exemple 1E mais à partir de 5,0 g de L-cystéine et de 7,8 g de phényl-2 benzaldéhyde. On obtient ainsi 8,5 g d'acide (phényl-2 phényl)-2 thiazolidinecarboxylique-4-(2RS,4R) fondant à 190 °C.
F Le phényl-2 benzaldéhyde peut être préparé de la manière suivante : à une solution contenant 8,0 g de bromo-2 benzaldéhyde et 5,2 g d'acide phénylboronique dans 80 cm³ de diméthoxyéthane on ajoute 2,5 g de palladium(0) tétrakis (triphénylphosphine) et 40 cm³ d'une solution aqueuse de carbonate de sodium 2M. Le milieu réactionnnel est chauffé au reflux pendant 12 heures puis après retour à une température au voisinage de 25°C dilué par 200 cm³ d'acétate d'éthyle. La phase organique est séparée par décantation, lavée par 2 fois 50 cm³ d'eau, séchée sur sulfate de magnésium et concentrée à sec sous pression réduite à 40°C. Le produit brut obtenu est purifié par chromatographie sur silice [éluant : cyclohexane-acétate d'éthyle (95/5 en volumes)]. Les fractions contenant le produit attendu sont réunies et concentrées à sec sous pression réduite à 40°C. On obtient ainsi, 8,1 g de phényl-2 benzaldéhyde sous forme d'une huile jaune et utilisée telle quelle dans les synthèses ultérieures.

### EXEMPLE 16

On opère d'une manière analogue à celle décrite à l'exemple 1A mais à partir de 1,32 g d'(amino-2 acétyl)-3 (phényl-2 phényl)-2 thiazolidinecarboxylate-4 de tert-butyle-(2R,4R) et de 0,47 g d'isocyanate de méthyl-3 phényle. Le produit brut est purifié par chromatographie sur silice [éluant : chlorure de méthylène-acétate d'éthyle (90/10 en volumes)]. Les fractions contenant le produit attendu sont réunies et concentrées à sec sous pression réduite à 40°C. On obtient ainsi, après battage dans le pentane, 0,22 g de {[(méthyl-3 phényl)-3 uréido]-2 acétyl}-3 (phényl-2 phényl)-2 thiazolidinecarboxylate-4 de tert-butyle-(2R,4R) sous forme d'un produit amorphe [α]_{D}²⁵ = -9,8 ± 0,9° (C% = 0,49; CHCl₃).

RMN (300 MHz, δ en ppm, DMSO D₆ + 2 gouttes de CD₃COOD, 393 K). On observe le mélange des deux rotamères (50/50) : 1,50 et 1,60 [2s, 9H en totalité : -COOC(CH₃)₃], 2,24 (s, 3H, Ar-CH₃), de 3,10 à 4,10 (mt, 4H : -CH₂-S- et N-CH₂-CO-), 4,67 et 5,19 (respectivement t et dd, 1H en totalité: N-CH-COO-), 5,98 et 6,00 ( 2s, 1H : N-CH-S-), 6,22 et 6,31 (2t, 1H en totalité: -NH-CO-), 6,73 [d, 1H : Ar (-H 4)], de 7,05 à 7,20 [mt, 3H : Ar (-H2, -H5 et -H6)], de 7,20 à 7,60 - 7,91 et 8,25 (respectivement mt et 2d larges, 8H -1H : -H aromatiques), 8,62 et 8,71 (2s larges, 1H en totalité: -CO-NH-Ar).

### EXEMPLE 17

A une solution de 0,22 g d'acide {{[tert-butoxycarbonyl-4 (fluoro-2 phényl)1-2 thiazolidinyl-3-(2R,4R)]-2 oxo-2 éthyl}-3 uréido}-3 phényl-2 propionique-(S) dans 10 cm³ de tétrahydrofuranne on ajoute 12,4 cm³ d'une solution aqueuse d'hydroxyde de sodium 0,1 N. Le milieu réactionnel est agité 48 heures à une température voisine de 25°C puis le solvant organique est éliminé par évaporation sous pression réduite. La solution aqueuse obtenue est filtrée, amenée à un pH voisin de 3 par addition d'une solution aqueuse d'acide sulfurique 1N. Le précipié obtenu est séparé par filtration, lavé par 2 fois 5 cm³ d'eau distillée et séché à l'air. On obtient ainsi 0,15 g d'acide {{{[carboxy-4 (fluoro-2 phényl)l-2 thiazolidinyl-3-(2R,4RS)]-2 oxo-2 éthyl}-3 uréido}-3 phényl-2 propionique-(S) fondant à 148°C.

RMN (300 MHz, δ en ppm, DMSO D₆ + 2 gouttes de CD₃COOD, 393 K). On observe le mélange des deux diastéréoisomères (65/35) : 1,38 (d, 3H; -CH₃), de 3,25 à 3,60 (mt, 2H : -S-CH₂-), 3,58 (q, 1H : Ar- CH-COO-), 3,77 et 4,09 (respectivement mt et 2d, 1H chacun: N-CH₂-CO-), 5,05 et 5,34 (respectivement tet dd, 1H en totalité: N-CH-CO-), 6,38 et 6,54 (2s, 1H en totalité: N-CH-S-), 6,85 [d large, 1H : Ar (-H 4)], de 7,10 à 8,00 [mt, 7H : Ar (-H 5 -H 2 et -H6 et aromatiques)].

L'acide {{[tert-butoxycarbonyl-4 (fluoro-2 phényl)1-2 thiazolidinyl-3-(2R,4R)]-2 oxo-2 éthyl}-3 uréido}-3 phényl-2 propionique-(S) peut être préparé selon la méthode décrite dans le brevet WO9301165.

Les médicaments selon l'invention sont constitués par un composé de formule (I) sous forme libre ou sous forme d'un sel, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles dans le traitement et la prévention des désordres liés à la CCK et à la gastrine au niveau du système nerveux et de l'appareil gastrointestinal. Ces composés peuvent donc être utilisés dans le traitement et la prévention des psychoses, des troubles anxieux, de la dépression, de la neurodégénération, des attaques de panique, de la maladie de Parkinson, de la diskynésie tardive, du syndrôme du colon irritable, de la pancréatite aiguë, des ulcères, des désordres de la motilité intestinale, de certaines tumeurs sensibles à la CCK, des troubles de la mémoire, dans le sevrage aux traitements chroniques et abus d'alcool ou de médicaments, comme constricteurs de la pupille de l'oeil, comme analgésiques, comme potentialisateurs de l'activité analgésique des médicaments analgésiques narcotiques et non narcotiques et comme régulateurs de l'appétit.

Les doses dépendent de l'effet recherché, de la durée du traitement et de la voie d'administration utilisée; elles sont généralement comprises entre 0,05 g et 1 g par jour par voie orale pour un adulte avec des doses unitaires allant de 10 mg à 500 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Acide {[(tert-butoxycarbonyl-4 cyclohexyl-2 thiazolidinyl-3)-2 oxo-2 éthyl]-3 uréido}-3 phényl acétique-(4R) (isomère A) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- {[(méthyl-3 phényl)-3 uréido]-2 acétyl}-3 benzyl-2 thiazolidinecarboxylate-4 de tert-butyle-(4R)( isomère A) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Acide {[(tert-butoxycarbonyl-4 benzyl-2 thiazolidinyl-3)-2 oxo-2 éthyl]-3 uréido}-3 phénylacétique-(4R) (isomère A). 10 mg
- Acide benzoïque 80 mg
- Alcool benzylique 0,06 cm³
- Benzoate de sodium 80 mg
- Ethanol à 95 % 0,4 cm³
- Hydroxyde de sodium 24 mg
- Propylène glycol 1,6 cm³
- Eau q.s.p. 4 cm3

## Revendications

1. Composés de formule : 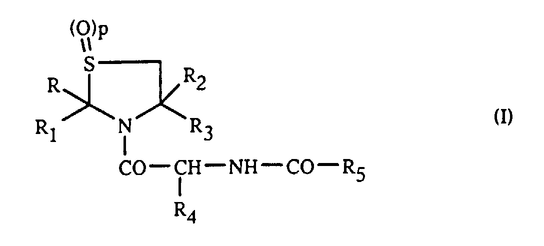 dans laquelle,
R représente un radical alkyle contenant 1 à 12 atomes de carbone, en chaîne droite ou ramifiée et éventuellement mono ou polyinsaturé, cycloalkyle contenant 3 à 12 atomes de carbone et éventuellement mono ou polyinsaturé, polycycloalkyle contenant 6 à 12 atomes de carbone et éventuellement mono ou polyinsaturé, phénylalkyle dont le noyau phényle est éventuellement substitué (par un ou plusieurs substituants choisis parmi les radicaux alkyle, alcoxy ou les atomes d'halogène), diphénylalkyle, cinnamyle, pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle, oxo-2 pipéridyle, quinuclidinyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-NR₇R_{8,} -NH- CO-CH₃, trifluorométhyle, phényle ou trifluorométhoxy,
R₁ représente un atome d'hydrogène ou un radical alkyle, cycloalkyle, phénylalkyle ou phényle éventuellement substitué par un ou plusieurs substituants choisis parmi les atomes d'halogène et les radicaux alkyle et alcoxy,
R₂ représente une chaîne -(CH₂)ₙ-CO-R₆, -(CH₂)ₘ-O-CO-R"₆, -(CH₂)ₘ-NR₉R₁₀ ou un radical oxazolinyle éventuellement substitué par un ou plusieurs radicaux alkyle ou alkyl-3 oxadiazolyle,
R₃ représente un atome d'hydrogène ou un radical alkyle, cycloalkyle, phénylalkyle ou phényle éventuellement substitué par un ou plusieurs substituants choisis parmi les atomes d'halogène et les radicaux alkyle et alcoxy,
R₄ représente un atome d'hydrogène ou un radical alkyle,
R₅ représente un radical phényle (éventuellement substitué par un ou plusieurs substituants choisis parmi les atomes d'halogène et les radicaux alkyle, alcoxy et alkylthio), naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₁, -SO₂-NH-SO₂-R₁₁, -CO-NH-CO-R₁₁, -CO-NH-SO₂-R₁₁, -B(OH)₂, -C(NH₂)=NOH, -SO₂-NH-R₁₂, -CO-NH-R₁₂, 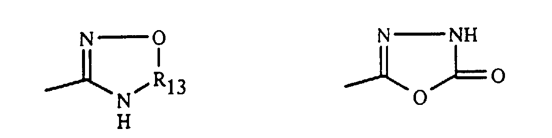 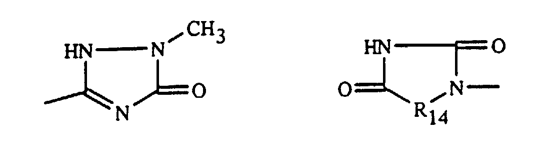 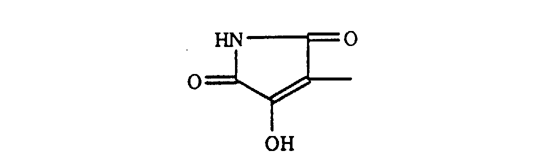 ou diméthyl-2,2 dioxo-4,6 dioxanne-1,3-yl-5,
R₆ représente un radical hydroxy, alcoxy, cycloalkyloxy, cycloalkylalkyloxy, phényle ou -NR₉R₁₀ ,
R"₆ représente un radical alcoxy, cycloalkyloxy, cycloalkylalkyloxy, phényle ou -NR₉R₁₀,
R₇ représente un atome d'hydrogène ou un radical alkyle, phénylalkyle ou phényle éventuellement substitué par un ou plusieurs substituants choisis parmi les atomes d'halogène et les radicaux alkyle, alcoxy et alkylthio,
R₈ représente un radical alkyle, phénylalkyle ou phényle éventuellement substitué par un ou plusieurs substituants choisis parmi les atomes d'halogène et les radicaux alkyle, alcoxy et alkylthio,
ou bien R₇ et R₈ forment avec l'atome d'azote auquel ils sont rattachés un hétérocycle mono ou polycyclique saturé ou insaturé contenant 4 à 9 atomes de carbone et un ou plusieurs hétéroatomes (O, N) et éventuellement substitué par un ou plusieurs radicaux alkyle,
R₉ représente un atome d'hydrogène ou un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
R₁₀ représente un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
ou bien R₉ et R₁₀ forment ensemble avec l'atome d'azote auquel ils sont rattachés un hétérocycle mono ou polycyclique saturé ou insaturé contenant 4 à 9 atomes de carbone et un ou plusieurs hétéroatomes (O, N, S) et éventuellement substitué par un ou plusieurs radicaux alkyle,
R₁₁ représente un radical alkyle, cycloalkyle, trifluorométhyle, phényle éventuellement substitué par un ou plusieurs substituants choisis parmi les radicaux cyano, alcoxy, nitro, amino et les atomes d'halogène,
R₁₂ représente un radical tétrazolyl-5,
R₁₃ représente C=O ou S=O,
R₁₄ représente O ou C=O,
p est égal à 0, 1 ou 2,
n est égal à 0, 1 ou 2,
m est égal à 1 ou 2,
X représente un atome d'hydrogène, un radical alkyle ou phénylalkyle,
alk représente un radical alkyle ou alkylène,
alk' représente un radical hydroxyalkyle, hydroxyalkylène, alkoxyalkyle ou alkoxyalkylène,
étant entendu que n est différent de 0 lorsque R₁ et R₃ représentent chacun un atome d'hydrogène et R représente un radical pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-NR₇R_{8,} -NH-CO-CH₃, trifluorométhyle ou trifluorométhoxy et étant entendu que, sauf mention contraire, les radicaux alkyle, alkylène et alcoxy et les portions alkyle, alkylène et alcoxy contiennent 1 à 4 atomes de carbone en chaîne droite ou ramifiée, les radicaux et portions acyle contiennent 2 à 4 atomes de carbone et les radicaux et portions cycloalkyle contiennent 3 à 6 atomes de carbone,
ainsi que leurs sels et leurs isomères lorsqu'ils comportent au moins un centre asymétrique.

2. Composés de formule (I) selon la revendication 1 pour lesquels R représente un radical isopropényle, cyclohexyle, tétrahydrophényle, cyclopentadiène, dihydrophényle, norbornyle, adamantyle ou norbornényle ainsi que leurs sels et leurs isomères lorsqu'ils comportent au moins un centre asymétrique.

3. Composés de formule (I) selon la revendication 1 pour lesquels R₇ et R₈ forment avec l'atome d'azote auquel ils sont rattachés un hétérocycle choisi parmi les cycles pipéridino éventuellement substitué par un ou plusieurs radicaux alkyle ou tétrahydro-1,2,3,4 quinoléine ainsi que leurs sels et leurs isomères lorsqu'ils comportent au moins un centre asymétrique.

4. Composés de formule (I) selon la revendication 1 pour lesquels R₉ et R₁₀ forment avec l'atome d'azote auquel ils sont rattachés un hétérocycle choisi parmi les cycles pipéridino, perhydroazépinyl-1, tétrahydro-1,2,3,6 pyridyl-1, tétrahydro-1,2,3,4 quinolyl-1, pyrrolidinyl-1, tétrahydro-1,2,3,4 isoquinolyl-2, thiomorpholino ou indolinyl-1, ces cycles pouvant être éventuellement substitués par au moins un radical alkyle ainsi que leurs sels et leurs isomères lorsqu'ils comportent au moins un centre asymétrique.

5. Composés de formule (I) selon la revendication 1 pour lesquels R représente un radical alkyle contenant 1 à 12 atomes de carbone en chaîne droite ou ramifiée, cycloalkyle contenant 3 à 12 atomes de carbone et éventuellement monoinsaturé, polycycloalkyle contenant 6 à 12 atomes de carbone et éventuellement mono ou polyinsaturé, phénylalkyle, phényle éventuellement substitué par un ou plusieurs substituants choisis parmi les atomes d'halogène et les radicaux alkyle, -alk-COOX et carboxy, R₁ représente un atome d'hydrogène, R₂ représente une chaîne -(CH₂)ₙ-CO-R₆, R₃.représente un atome d'hydrogène, R₄.représente un atome d'hydrogène et R₅ représente un radical phénylamino dont le noyau phényle est substitué par un radical alkyle, -alk-COOX ou carboxy ainsi que leurs sels et leurs isomères lorsqu'ils comportent au moins un centre asymétrique.

6. Les composés suivants :
- acide {[(tert-butoxycarbonyl-4 cyclohexyl-2 thiazolidinyl-3)-2 oxo-2 éthyl]-3 uréido}-3 phénylacétique-(4R),
- {[(méthyl-3 phényl)-3 uréido]-2 acétyl}-3 benzyl-2 thiazolidinecarboxylate-4 de tert-butyle-(4R),
- acide {[(tert-butoxycarbonyl-4 benzyl-2 thiazolidinyl-3)-2 oxo-2 éthyl]-3 uréido}-3 phénylacétique-(4R),
- acide {{[tert-butoxycarbonyl-4 (tétrahydro-1,2,3,6 phényl-1-(RS))-2 thiazolidinyl-3]-2 oxo-2 éthyl}-3 uréido}-3 phénylacétique-(2R,4R),
- acide {{[tert-butoxycarbonyl-4 [(norbornène-5 yl)-2-(2RS)]-2 thiazolidinyl-3]-2 oxo-2 éthyl}-3 uréido}-3 phénylacétique-(4R),
- {{[tert-butoxycarbonyl-4 [(norbornène-5 yl)-2-(2RS)]-2 thiazolidinyl-3]-2 oxo-2 éthyl}-3 uréido}-3 phénylacétate de méthyle-(4R),
- acide {{[tert-butoxycarbonyl-4 [(norbornène-5 yl)-2-(2RS)]-2 thiazolidinyl-3]-2 oxo-2 éthyl}-3 uréido}-3 phénylacétique-(4R),
- acide {{[tert-butoxycarbonyl-4 (norbomyl-2-(2RS))-2 thiazolidinyl-3]-2 oxo-2 éthyl}-3 uréido}-3 phénylacétique-(4R),
- {[(méthyl-3 phényl)-3 uréido]-2 acétyl}-3 [(norbornène-5 yl-2-(RS)]-2 thiazolidinecarboxylate de tert-butyle-(4R),
- acide {(tert-butoxycarbonyl-4 tert-butyl-2 thiazolidinyl-3)-2 oxo-2 éthyl]-3 uréido}-3 phénylacétique-(4R),
- acide {[(tert-butoxycarbonyl-4 butyl-2 thiazolidinyl-3-(4R))-2 oxo-2 éthyl]-3 uréido}-3 phényl-2 propionique-(S),
- {[(méthyl-3 phényl)-3 uréido]-2 acétyl}-3 butyl-2 thiazolidinecarboxylate-4 de tert-butyle-(4R),
- acide {[(tert-butoxycarbonyl-4 butyl-2 thiazolidinyl-3)-2 oxo-2 éthyl]-3 uréido}-3 benzoïque-(4R),
- {[(méthyl-3 phényl)-3 uréido]-2 acétyl}-3 (tétrahydro-1,2,3,6 phényl-1-(RS))-2 thiazolidinecarboxylate-4 de tert-butyle-(2R, 4R),
- acide {{[tert-butoxycarbonyl-4 (phényl-2 phényl)-2 thiazolidinyl-3]-2 oxo-2 éthyl}-3 uréido}-3 phénylacétique-(2R, 4R),
- {[(méthyl-3 phényl)-3 uréido]-2 acétyl}-3 (phényl-2 phényl)-2 thiazolidinecarboxylate-4 de tert-butyle-(2R,4R),
- acide {{{[carboxy-4 (fluoro-2 phényl)1-2 thiazolidinyl-3-(2R,4RS)]-2 oxo-2 éthyl}-3 uréido}-3 phényl-2 propionique-(S),
leurs isomères, les mélanges de leurs isomères et leurs sels.

7. Procédé de préparation des composés de formule (I) selon la revendication 1 pour lesquels p est égal à 0 et R₅ représente un radical phénylamino dont le noyau phényle est éventuellement substitué caractérisé en ce que l'on fait réagir un dérivé réactif de l'acide carbamique, obtenu éventuellement in situ par action d'un dérivé réactif de l'acide carbonique choisi parmi le N,N'-diimidazole carbonyle, le phosgène, le diphosgène, le triphosgène et le chloroformiate de p-nitrophényle sur un dérivé de formule : 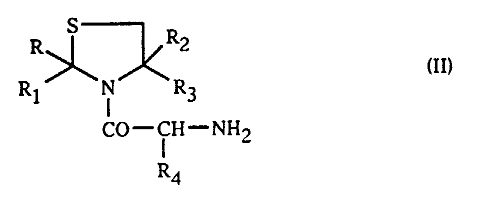 dans laquelle R, R₁, R_{2,} R₃ et R₄ ont les mêmes significations que dans la revendication 1, sur une aniline dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H, -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, diméthyl-2,2 dioxo-4,6 dioxanne-1,3-yl-5, -alk-SO₂H, -SO₂-N H-CO-R₁₁, -SO₂-NH-SO₂-R₁₁, -CO-NH-CO-R₁₁, -CO-NH-SO₂-R₁₁, -B(OH)₂, -C(NH₂)=NOH, -SO₂-NH-R₁₂, -CO-NH-R₁₂, diméthyl-2,2 dioxo-4,6 dioxanne-1,3-yl-5 ou 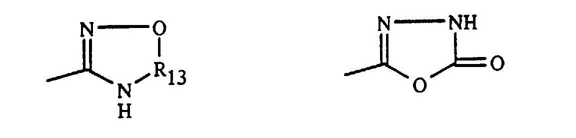 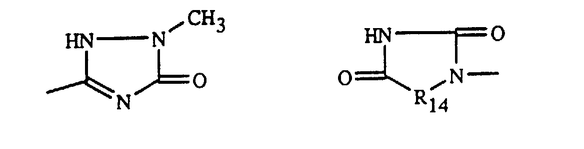 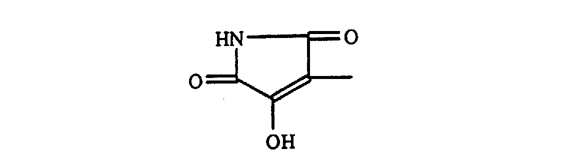 isole le produit et le transforme éventuellement en sel.

8. Procédé de préparation des composés de formule (I) selon la revendication 1 pour lesquels p est égal à 0 et R₅ représente un radical phénylamino dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est différent d'un atome d'hydrogène caractérisé en ce que l'on fait réagir un dérivé de formule : 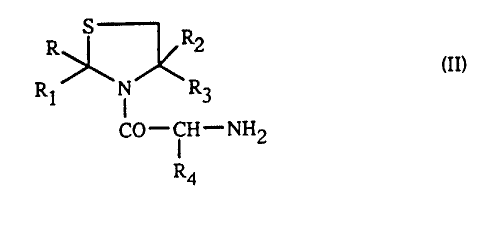 dans laquelle R, R₁, R_{2,} R₃ et R₄ ont les mêmes significations que dans la revendication 1 sur un phénylisocyanate dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est différent d'un atome d'hydrogène, isole le produit et le transforme éventuellement en sel.

9. Procédé de préparation des composés de formule (I) selon la revendication 1 pour lesquels p est égal à 0 et R₅ représente un radical phényle éventuellement substitué, naphtyle, indolyle, quinolyle caractérisé en ce que l'on fait réagir un dérivé de formule : 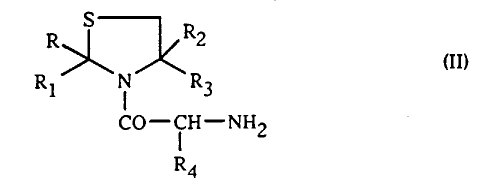 dans laquelle R, R₁, R_{2,} R₃ et R₄ ont les mêmes significations que dans la revendication 1 sur un acide de formule HOOC-R₅ dans laquelle R₅ a les mêmes significations que précédemment ou un dérivé réactif de cet acide, isole le produit et le transforme éventuellement en sel.

10. Procédé de préparation des composés de formule (I) selon la revendication 1 pour lesquels R₅ représente un radical phénylamino dont le noyau phényle est substitué par un radical carboxy, -alk-COOH, -O-alk-COOH, -alk'-COOH, -CH=CH-COOH, -CO-COOH, -S-alk-COOH, -SO-alk-COOH, -SO₂-alk-COOH, -C(=NOH)-COOH, -O-CH₂-alk'-COOH ou -CX=N-O-alk-COOH et/ou R₂ représente une chaîne -(CH₂)ₙ-COOH caractérisé en ce que l'on hydrolyse ou, selon le cas, hydrogénolyse un ester correspondant de formule (I), isole le produit et le transforme éventuellement en sel.

11. Procédé de préparation des composés de formule (I) selon la revendication 1 pour lesquels p est égal à 0 et R₅ représente un radical phénylamino dont le noyau phényle est substitué par un radical hydroxyiminoalkyle ou alcoxyiminoalkyle caractérisé en ce que l'on fait réagir un dérivé acylé correspondant de formule (I) sur un dérivé de formule :
H₂N-OR₁₈ (XI)
dans laquelle R₁₈ représente un atome d'hydrogène ou un radical alkyle, isole le produit et le transforme éventuellement en sel.

12. Procédé de préparation composés de formule (I) selon la revendication 1 pour lesquels p est égal à 0, R₂ représente une chaîne -(CH₂)ₙ-CO-R₆, n est égal à 0, R₆ représente un radical alcoxy, cycloalkyloxy ou cycloalkylalkyloxy et R₅ représente un radical phényle éventuellement substitué, naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est différent d'un atome d'hydrogène caractérisé en ce que l'on fait réagir un dérivé de formule : 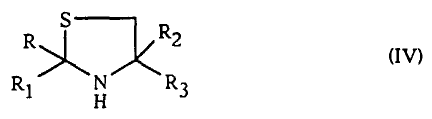 dans laquelle R, R₁ et R₃ ont les mêmes significations que dans la formule (I) et R₂ a les mêmes significations que précédemment, sur un acide de formule : 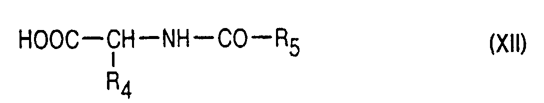 dans laquelle R₅ a les mêmes significations que ci-dessus ou un dérivé réactif de cet acide et R₄ a les mêmes significations que dans la formule (I).

13. Procédé de préparation des composés de formule (I) selon la revendication 1 pour lesquels p est égal à 0 et R représente un radical cycloalkyle saturé ou un radical polycycloalkyle saturé caractérisé en ce que l'on hydrogène un dérivé insaturé correspondant de formule (I), isole le produit et le transforme éventuellement en sel.

14. Procédé de préparation des composés de formule (I) selon la revendication 1 pour lesquels p est égal à 1 ou 2 caractérisé en ce que l'on oxyde un composé de formule (I) correspondant pour lequel p est égal à 0, isole le produit et le transforme éventuellement en sel.

15. Médicaments caractérisés en ce qu'ils contiennent comme principe actif au moins un des composés selon l'une des revendications 1 à 6.

16. Médicaments selon la revendication 15 utilisables pour le traitement des désordres liés à la CCK et à la gastrine.

## Patentansprüche

1. Verbindungen der Formel (I) 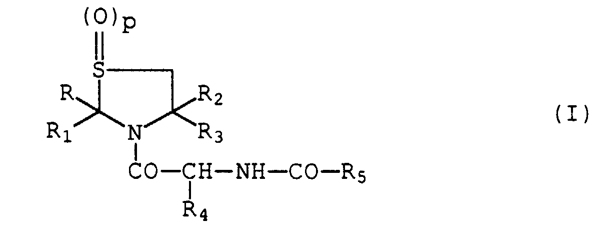 in der
R einen Rest Alkyl mit 1 bis 12 Kohlenstoffatomen in gerader oder verzweigter Kette und gegebenenfalls mono- oder poly-ungesättigt; Cycloalkyl mit 3 bis 12 Kohlenstoffatomen und gegebenenfalls mono- oder poly-ungesättigt; Polycycloalkyl mit 6 bis 12 Kohlenstoffatomen und gegebenenfalls mono- oder poly-ungesättigt; Phenylalkyl, dessen Phenylkern gegebenenfalls substituiert ist (durch einen oder mehrere Substituenten, ausgewählt unter den Resten Alkyl, Alkoxy oder den Halogenatomen); Diphenylalkyl, Cinnamyl, Pyridyl, gegebenenfalls substituiert durch einen oder mehrere Reste Alkyl; Furyl, gegebenenfalls substituiert durch einen oder mehrere Reste Alkyl; Thienyl, gegebenenfalls substituiert durch einen oder mehrere Reste Alkyl; Chinolyl, gegebenenfalls substituiert durch einen oder mehrere Reste Alkyl; Naphthyl, gegebenenfalls substituiert durch einen oder mehrere Reste Alkyl; Indolyl, gegebenenfalls substituiert durch einen oder mehrere Reste Alkyl; Oxo-2-piperidyl; Chinuclidinyl oder Phenyl darstellt, gegebenenfalls substituiert durch einen oder mehrere Substituenten, ausgewählt unter den Halogenatomen und den Resten Alkyl, Alkoxy, Hydroxy, Nitro, Amino, Monoalkylamino, Dialkylamino, Alkoxycarbonyl, -CO-NR₇R₈, -NH-CO-CH₃, Trifluormethyl, Phenyl oder Trifluormethoxy;
R₁ ein Wasserstoffatom oder einen Rest Alkyl, Cycloalkyl, Phenylalkyl oder Phenyl bedeutet, gegebenenfalls substituiert durch einen oder mehrere Substituenten, ausgewählt unter den Halogenatomen und den Resten Alkyl und Alkoxy;
R₂ eine Kette -(CH₂)ₙ-CO-R₆, -(CH₂)ₘ-O-CO-R"₆, -(CH₂)ₘ-NR₉R₁₀ oder einen Rest Oxazolinyl darstellt, gegebenenfalls substituiert durch einen oder mehrere Reste Alkyl oder 3-Alkyl-oxadiazolyl;
R₃ ein Wasserstoffatom oder einen Rest Alkyl, Cycloalkyl, Phenylalkyl oder Phenyl bedeutet, gegebenenfalls substituiert durch einen oder mehrere Substituenten, ausgewählt unter den Halogenatomen und den Resten Alkyl und Alkoxy;
R₄ ein Wasserstoffatom oder ein Rest Alkyl ist;
R₅ einen Rest Phenyl (gegebenenfalls substituiert durch einen oder mehrere Substituenten, ausgewählt unter den Halogenatomen und den Resten Alkyl, Alkoxy und Alkylthio); Naphthyl; Indolyl; Chinolyl oder Phenylamino darstellt, dessen Phenylkern gegebenenfalls substituiert ist durch einen oder mehrere Substituenten, ausgewählt unter den Halogenatomen und den Resten Alkyl, Alkoxy, Alkylthio, Trifluormethyl, Carboxy, Alkoxycarbonyl, Hydroxy, Nitro, Amino, Acyl, Cyano, Sulfamoyl, Carbamoyl, Hydroxyiminoalkyl, Alkoxyiminoalkyl, Hydroxyaminocarbonyl, Alkoxyaminocarbonyl, 5-Tetrazolyl, 5-Tetrazolyl-alkyl, Trifluormethylsulfonamido, Alkylsulfinyl, mono- oder poly-Hydroxyalkyl, Sulfo, -alk-O-CO-alk, -alk-COOX, -alk-O-alk, -alk'-COOX, -O-alk-COOX, -CH=CH-COOX, -CO-COOX, -alk-SO₃H (in Form des Salzes), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₁, -SO₂-NH-SO₂-R₁₁, -CO-NH-CO-R₁₁, -CO-NH-SO₂-R₁₁, - B(OH)₂, -C(NH₂)=NOH, -SO₂-NH-R₁₂, -CO-NH-R₁₂, 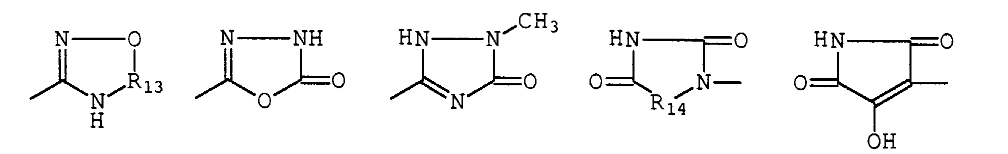 oder 2,2-Dimethyl-4,6-dioxo-1,3-dioxan-5-yl darstellt;
R₆ einen Rest Hydroxy, Alkoxy, Cycloalkyloxy, Cycloalkylalkyloxy, Phenyl oder -NR₉R₁₀ bedeutet;
R"₆ einen Rest Alkoxy, Cycloalkyloxy, Cycloalkylalkyloxy, Phenyl oder -NR₉R₁₀ bedeutet;
R₇ ein Wasserstoffatom oder einen Rest Alkyl, Phenylalkyl oder Phenyl darstellt, gegebenenfalls substituiert durch einen oder mehrere Substituenten, ausgewählt unter den Halogenatomen und den Resten Alkyl, Alkoxy und Alkylthio;
R₈ einen Rest Alkyl, Phenylalkyl oder Phenyl darstellt, gegebenenfalls substituiert durch einen oder mehrere Substituenten, ausgewählt unter den Halogenatomen und den Resten Alkyl, Alkoxy und Alkylthio;
oder auch R₇ und R₈ zusammen mit dem Stickstoffatom, an das sie gebunden sind, einen gesättigten oder ungesättigten, mono- oder polycyclischen Heterocyclus mit 4 bis 9 Kohlenstoffatomen und einem oder mehreren Heteroatomen (O, N) und gegebenenfalls substituiert durch einen oder mehrere Reste Alkyl bilden;
R₉ ein Wasserstoffatom oder einen Rest Alkyl, Cycloalkylalkyl, Cycloalkyl, Phenylalkyl oder Phenyl bedeutet, gegebenenfalls substituiert durch einen oder mehrere Substituenten, ausgewählt unter den Halogenatomen und den Resten Alkyl, Alkoxy und Alkylthio;
R₁₀ einen Rest Alkyl, Cycloalkylalkyl, Cycloalkyl, Phenylalkyl oder Phenyl bedeutet, gegebenenfalls substituiert durch einen oder mehrere Substituenten, ausgewählt unter den Halogenatomen und den Resten Alkyl, Alkoxy und Alkylthio;
oder auch R₉ und R₁₀ zusammen mit dem Stickstoffatom, an das sie gebunden sind, einen gesättigten oder ungesättigten, mono- oder polycyclischen Heterocyclus mit 4 bis 9 Kohlenstoffatomen und einem oder mehreren Heteroatomen (O, N, S) und gegebenenfalls substituiert durch einen oder mehrere Reste Alkyl bilden;
R₁₁ einen Rest Alkyl, Cycloalkyl, Trifluormethyl oder Phenyl darstellt, gegebenenfalls substituiert durch einen oder mehrere Substituenten, ausgewählt unter den Resten Cyano, Alkoxy, Nitro, Amino und den Halogenatomen;
R₁₂ einen Rest 5-Tetrazolyl bedeutet;
R₁₃ C=O oder S=O bedeutet;
R₁₄ O oder C=O bedeutet;
p gleich 0, 1 oder 2 ist;
n gleich 0, 1 oder 2 ist;
m gleich 1 oder 2 ist;
X ein Wasserstoffatom, einen Rest Alkyl oder Phenylalkyl darstellt;
alk einen Rest Alkyl oder Alkylen bedeutet;
alk' einen Rest Hydroxyalkyl, Hydroxyalkylen, Alkoxyalkyl oder Alkoxyalkylen bedeutet;
mit der Maßgabe, daß n von 0 verschieden ist, wenn R₁ und R₃ jeweils ein Wasserstoffatom darstellen und R einen Rest Pyridyl bedeutet, gegebenenfalls substituiert durch einen oder mehrere Reste Alkyl; Furyl, gegebenenfalls substituiert durch einen oder mehrere Reste Alkyl; Thienyl, gegebenenfalls substituiert durch einen oder mehrere Reste Alkyl; Chinolyl, gegebenenfalls substituiert durch einen oder mehrere Reste Alkyl; Naphthyl, gegebenenfalls substituiert durch einen oder mehrere Reste Alkyl; Indolyl, gegebenenfalls substituiert durch einen oder mehrere Reste Alkyl oder Phenyl, gegebenenfalls substituiert durch einen oder mehrere Substituenten, ausgewählt unter den Halogenatomen und den Resten Alkyl, Alkoxy, Hydroxy, Nitro, Amino, Monoalkylamino, Dialkylamino, Alkoxycarbonyl, -CO-NR₇R₈, -NH-CO-CH₃, Trifluormethyl oder Trifluormethoxy;
und mit der Maßgabe, daß außer bei gegenteiliger Erwähnung die Reste Alkyl, Alkylen und Alkoxy und die Teile Alkyl, Alkylen und Alkoxy 1 bis 4 Kohlenstoffatome in gerader oder verzweigter Kette enthalten, die Reste und Teile Acyl 2 bis 4 Kohlenstoffatome enthalten und die Reste und Teile Cycloalkyl 3 bis 6 Kohlenstoffatome enthalten;
sowie ihre Salze und ihre Isomere, wenn sie mindestens ein asymmetrisches Zentrum umfassen.

2. Verbindungen der Formel (I) nach Anspruch 1, worin R einen Rest Isoprenyl, Cyclohexyl, Tetrahydrophenyl, Cyclopentadien, Dihydrophenyl, Norbornyl, Adamantyl oder Norbornenyl darstellt, sowie ihre Salze und ihre Isomere, wenn sie mindestens ein asymmetrisches Zentrum umfassen.

3. Verbindungen der Formel (I) nach Anspruch 1, worin R₇ und R₈ zusammen mit dem Stickstoffatom, an das sie gebunden sind, einen Heterocyclus bilden, ausgewählt unter den Ringen Piperidino, gegebenenfalls substituiert durch einen oder mehrere Reste Alkyl, oder 1,2,3,4-Tetrahydrochinolin, sowie ihre Salze und ihre Isomere, wenn sie mindestens ein asymmetrisches Zentrum umfassen.

4. Verbindungen der Formel (I) nach Anspruch 1, worin R₉ und R₁₀ zusammen mit dem Stickstoffatom, an das sie gebunden sind, einen Heterocyclus bilden, ausgewählt unter den Ringen Piperidino, 1-Perhydroazepinyl, 1,2,3,6-Tetrahydro-1-pyridyl, 1,2,3,4-Tetrahydro-1-chinolyl, 1-Pyrrolidinyl, 1,2,3,4-Tetrahydro-2-isochinolyl, Thiomorpholino oder 1-Indolyl, wobei diese Ringe gegebenenfalls durch mindestens einen Rest Alkyl substituiert sein können, sowie ihre Salze und ihre Isomere, wenn sie mindestens ein asymmetrisches Zentrum umfassen.

5. Verbindungen der Formel (I) nach Anspruch 1, worin R einen Rest Alkyl mit 1 bis 12 Kohlenstoffatomen in gerader oder verzweigter Kette, Cycloalkyl mit 3 bis 12 Kohlenstoffatomen und gegebenenfalls mono-ungesättigt; Polycycloalkyl mit 6 bis 12 Kohlenstoffatomen und gegebenenfalls mono- oder poly-ungesättigt; Phenylalkyl, Phenyl, gegebenenfalls substituiert durch einen oder mehrere Substituenten, ausgewählt unter den Halogenatomen und den Resten Alkyl, -alk-COOX und Carboxy, darstellt, R₁ ein Wasserstoffatom ist, R₂ eine Kette -(CH₂)ₙ-CO-R₆ bedeutet, R₃ ein Wasserstoffatom ist, R₄ ein Wasserstoffatom ist und R₅ einen Rest Phenylamino bedeutet, dessen Phenylkern substituiert ist durch einen Rest Alkyl, -alk-COOX oder Carboxy, sowie ihre Salze und ihre Isomere, wenn sie mindestens ein asymmetrisches Zentrum umfassen.

6. Die folgenden Verbindungen:
- 3-{3-[2-(4-tert.-Butoxycarbonyl-2-cyclohexyl-3-thiazolidinyl)-2-oxo-ethyl]-ureido}-phenylessigsäure-(4R),
- 3-{2-[3-(3-Methylphenyl)-ureido]-acetyl}-2-benzyl-thiazolidin-4-carbonsäure-tert.-butylester-(4R),
- 3-{3-[2-(4-tert.-Butoxycarbonyl-2-benzyl-3-thiazolidinyl)-2-oxo-ethyl]-ureido}-phenylessigsäure-(4R),
- 3-{3-{2-[4-tert.-Butoxycarbonyl-2-(1,2,3,6-tetrahydro-1-phenyl-(RS))-3-thiazolidinyl]-2-oxo-ethyl}-ureido}-phenylessigsäure-(2R,4R),
- 3-{3-{2-[4-tert.-Butoxycarbonyl-2-[2-(5-norbornenyl)-(2RS)]-3-thiazolidinyl]-2-oxo-ethyl}-ureido}-phenylessigsäure-(4R),
- 3-{3-{2-[4-tert.-Butoxycarbonyl-2-[2-(5-norbornenyl)-(2RS)]-3-thiazolidinyl]-2-oxo-ethyl}-ureido}-phenylessigsäure-methylester-(4R),
- 3-{3-{2-[4-tert.-Butoxycarbonyl-2-[2-(5-norbornenyl)-(2RS)]-3-thiazolidinyl]-2-oxo-ethyl}-ureido}-phenylessigsäure-(4R),
- 3-{3-{2-[4-tert.-Butoxycarbonyl-2-(2-norbornyl)-(2RS))-3-thiazolidinyl]-2-oxo-ethyl}-ureido}-phenylessigsäure-(4R),
- 3-{2-[3-(3-Methylphenyl)-ureido]-acetyl}-2-[2-(5-norbornenyl)-(RS)]-thiazolidincarbonsäure-tert.-butylester-(4R),
- 3-{3-[2-(4-tert.-Butoxycarbonyl-2-tert.-butyl-3-thiazolidinyl)-2-oxo-ethyl]-ureido}-phenylessigsäure-(4R),
- 3-{3-[2-(4-tert.-Butoxycarbonyl-2-butyl-3-thiazolidinyl-(4R))-2-oxo-ethyl]-ureido}-phenyl-2-propionsäure-(S),
- 3-{2-[3-(3-Methylphenyl)-ureido}-acetyl}-2-butyl-thiazolidin-4-carbonsäure-tert.-butylester-(4R),
- 3-{3-[2-(4-tert.-Butoxycarbonyl-2-butyl-3-thiazolidinyl)-2-oxo-ethyl]-ureido}-benzoesäure-(4R),
- 3-{2-[3-(3-Methylphenyl)-ureido]-acetyl)-2-(1,2,3,6-tetrahydro-1-phenyl-(RS))-thiazolidin-4-carbonsäure-tert.-butylester-(2R, 4R),
- 3-{3-{2- [4-tert.-Butoxycarbonyl-2-(2-phenyl-phenyl)-3-thiazolidinyl]-2-oxo-ethyl}-ureido}-phenylessigsäure-(2R,4R),
- 3-{2-[3-(3-Methylphenyl)-ureido]-acetyl}-2-(2-phenyl-phenyl)-thiazolidin-4-carbonsäure-tert.-butylester-(2R,4R),
- 3-{3-{2-[4-Carboxy-2-(2-fluor-phenyl)-3-thiazolidinyl-(2R, 4SR)]-2-oxo-ethyl}-ureido}-2-phenylpropionsäure-(S),
ihre Isomere, die Mischungen ihrer Isomere und ihre Salze.

7. Verfahren zur Herstellung der Verbindungen der Formel (I) nach Anspruch 1, worin p gleich 0 ist und R₅ einen Rest Phenylamino darstellt, dessen Phenylkern gegebenenfalls substituiert ist, dadurch gekennzeichnet, daß man ein reaktives Derivat der Carbaminsäure, gegebenenfalls erhalten in situ durch Einwirkung eines reaktiven Derivates der Kohlensäure, ausgewählt unter N,N'-Carbonyldiimidazol, Phosgen, Diphosgen, Triphosgen und p-Nitrophenylchloroformiat, auf ein Derivat der Formel (II) 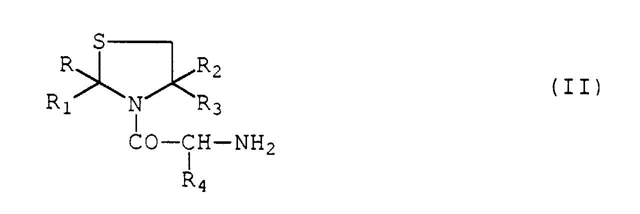 in der R, R₁, R₂, R₃ und R₄ die gleichen Bedeutungen wie in Anspruch 1 besitzen, mit einem Anilin zur Reaktion bringt, dessen Phenylkern gegebenenfalls substituiert ist durch einen oder mehrere Substituenten, ausgewählt unter den Halogenatomen und den Resten Alkyl, Alkoxy und Alkylthio, Trifluormethyl, Carboxy, Alkoxycarbonyl, Hydroxy, Nitro, Amino, Acyl, Cyano, Sulfamoyl, Carbamoyl, Hydroxyiminoalkyl, Alkoxyiminoalkyl, Hydroxyaminocarbonyl, Alkoxyaminocarbonyl, 5-Tetrazolyl, 5-Tetrazolyl-alkyl, Trifluormethylsulfonamido, Alkylsulfinyl, mono- oder poly-Hydroxyalkyl, Sulfo, -alk-O-CO-alk, -alk-COOX, -alk-O-alk, -alk'-COOX, -O-alk-COOX, -CH=CH-COOX, -CO-COOX, -alk-SO₃H, -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, 2,2-Dimethyl-4,6-dioxo-1,3-dioxan-5-yl, -alk-SO₂H, -SO₂-NH-CO-R₁₁, -SO₂-NH-SO₂-R₁₁, -CO-NH-CO-R₁₁, -CO-NH-SO₂-R₁₁, -B(OH)₂, -C(NH₂)=NOH, -SO₂-NH-R₁₂, -CO-NH-R₁₂, 2,2-Dimethyl-4,6-dioxo-1,3-dioxan-5-yl oder 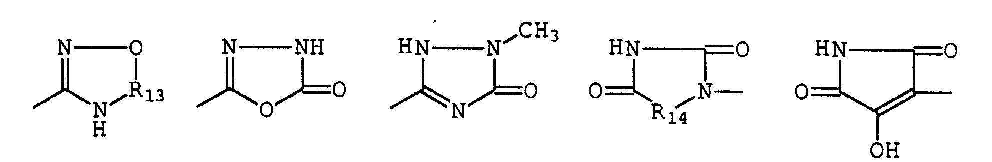 darstellt, das Produkt isoliert und gegebenenfalls in Salz überführt.

8. Verfahren zur Herstellung der Verbindungen der Formel (I) nach Anspruch 1, worin p gleich 0 ist und R₅ einen Rest Phenylamino darstellt, dessen Phenylkern gegebenenfalls substituiert ist durch einen oder mehrere Substituenten, ausgewählt unter den Halogenatomen und den Resten Alkyl, Alkoxy und Alkylthio, Trifluormethyl, Nitro, Acyl, Cyano, Sulfamoyl, Alkoxycarbonyl, Carbamoyl, Alkoxyiminoalkyl, Alkoxyaminocarbonyl, -alk-O-CO-alk, -CH=CH-alk', alk-O-alk, Trifluormethylsulfonamido, -alk-SO₃H (in Form von Salz), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX oder alk'-COOX, worin X von einem Wasserstoffatom verschieden ist, dadurch gekennzeichnet, daß man ein Derivat der Formel (II) 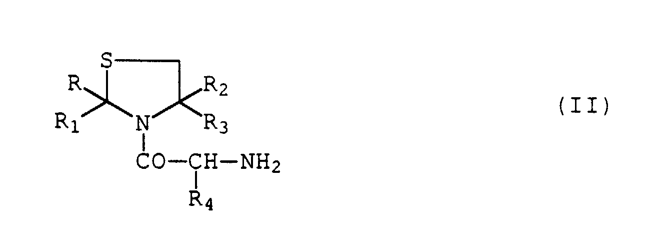 in der R, R₁, R₂, R₃ und R₄ die gleichen Bedeutungen wie in Anspruch 1 besitzen, mit einem Phenylisocyanat zur Reaktion bringt, dessen Phenylkern gegebenenfalls substituiert ist durch einen oder mehrere Substituenten, ausgewählt unter den Halogenatomen und den Resten Alkyl, Alkoxy und Alkylthio, Trifluormethyl, Nitro, Acyl, Cyano, Sulfamoyl, Alkoxycarbonyl, Carbamoyl, Alkoxyiminoalkyl, Alkoxyaminocarbonyl, -alk-O-CO-alk, -CH=CH-alk', alk-O-alk, Trifluormethylsulfonamido, -alk-SO₃H (in Form von Salz), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX oder alk'-COOX, worin X von einem Wasserstoffatom verschieden ist, das Produkt isoliert und gegebenenfalls in Salz überführt.

9. Verfahren zur Herstellung der Verbindungen der Formel (I) nach Anspruch 1, worin p gleich 0 ist und R₅ einen Rest Phenyl, gegebenenfalls substituiert, Naphthyl, Indolyl oder Chinolyl darstellt, dadurch gekennzeichnet, daß man ein Derivat der Formel (II) 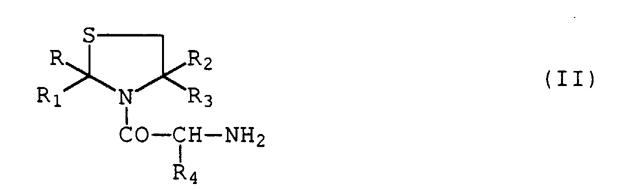 in der R, R₁, R₂, R₃ und R₄ die gleichen Bedeutungen wie in Anspruch 1 besitzen, mit einer Säure der Formel HOOC-R₅ oder mit einem reaktiven Derivat dieser Säure zur Reaktion bringt, worin R₅ die gleichen Bedeutungen wie vorstehend besitzt, das Produkt isoliert und gegebenenfalls in Salz überführt.

10. Verfahren zur Herstellung der Verbindungen der Formel (I) nach Anspruch 1, worin R₅ einen Rest Phenylamino darstellt, dessen Phenylkern substituiert ist durch einen Rest Carboxy, -alk-COOH, -O-alk-COOH, -alk'-COOH, -CH=CH-COOH, -CO-COOH, -S-alk-COOH, -SO-alk-COOH, -SO₂-alk-COOH, -C(=NOH)-COOH, -O-CH₂-alk'-COOH oder -CX=N-O-alk-COOH und/oder R₂ eine Kette -(CH₂)ₙ-COOH bedeutet, dadurch gekennzeichnet, daß man einen entsprechenden Ester der Formel (I) hydrolysiert oder gegebenenfalls hydrogenolysiert, das Produkt isoliert und gegebenenfalls in Salz überführt.

11. Verfahren zur Herstellung der Verbindungen der Formel (I) nach Anspruch 1, worin p gleich 0 ist und R₅ einen Rest Phenylamino darstellt, dessen Phenylkern durch einen Rest Hydroxyiminoalkyl oder Alkoxyiminoalkyl substituiert ist, dadurch gekennzeichnet, daß man ein entsprechendes, acyliertes Derivat der Formel (I) mit einem Derivat der Formel (XI)
H₂N-OR₁₈ (XI)
zur Reaktion bringt, worin R₁₈ ein Wasserstoffatom oder einen Rest Alkyl bedeutet, das Produkt isoliert und gegebenenfalls in Salz überführt.

12. Verfahren zur Herstellung der Verbindungen der Formel (I) nach Anspruch 1, worin p gleich 0 ist, R₂ eine Kette -(CH₂)ₙ-CO-R₆ mit n gleich 0 darstellt, worin R₆ einen Rest Alkoxy, Cycloalkyloxy oder Cycloalkylalkyloxy bedeutet und R₅ einen Rest Phenyl, gegebenenfalls substituiert, Naphthyl, Indolyl, Chinolyl oder Phenylamino darstellt, dessen Phenylkern gegebenenfalls substituiert ist durch einen oder mehrere Substituenten, ausgewählt unter den Halogenatomen und den Resten Alkyl, Alkoxy und Alkylthio, Trifluormethyl, Nitro, Acyl, Cyano, Sulfamoyl, Alkoxycarbonyl, Carbamoyl, Alkoxyiminoalkyl, Alkoxyaminocarbonyl, -alk-O-CO-alk, -CH=CH-alk', alk-O-alk, Trifluormethylsulfonamido, -alk-SO₃H (in Form von Salz), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX oder alk'-COOX, worin X von einem Wasserstoffatom verschieden ist, dadurch gekennzeichnet, daß man ein Derivat der Formel (IV) 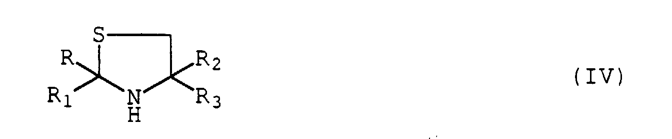 in der R, R₁ und R₃ die gleichen Bedeutungen wie in der Formel (I) und R₂ die gleichen Bedeutungen wie vorstehend besitzen, mit einer Säure der Formel (XII) 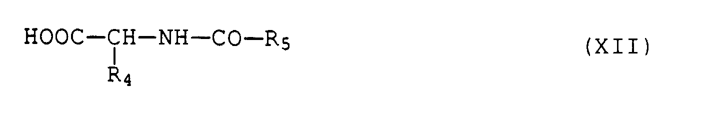 oder mit einem reaktiven Derivat dieser Säure zur Reaktion bringt, worin R₅ die gleichen Bedeutungen wie oben und R₄ die gleichen Bedeutungen wie in Formel (I) besitzen.

13. Verfahren zur Herstellung der Verbindungen der Formel (I) nach Anspruch 1, worin p gleich 0 ist und R einen gesättigten Rest Cycloalkyl oder einen gesättigten Rest Polycycloalkyl darstellt, dadurch gekennzeichnet, daß man ein entsprechendes, ungesättigtes Derivat der Formel (I) hydriert, das Produkt isoliert und gegebenenfalls in Salz überführt.

14. Verfahren zur Herstellung der Verbindungen der Formel (I) nach Anspruch 1, worin p gleich 1 oder 2 ist, dadurch gekennzeichnet, daß man eine entsprechende Verbindung der Formel (I), in der p gleich 0 ist, oxidiert, das Produkt isoliert und gegebenenfalls in Salz überführt.

15. Arzneimittel, dadurch gekennzeichnet, daß sie als Wirkstoff mindestens eine der Verbindungen nach einem der Ansprüche 1 bis 6 enthalten.

16. Arzneimittel nach Anspruch 15, anwendbar zur Behandlung von Störungen, die mit CCK und Gastrin in Verbindung stehen.

## Claims

1. Compounds of formula: 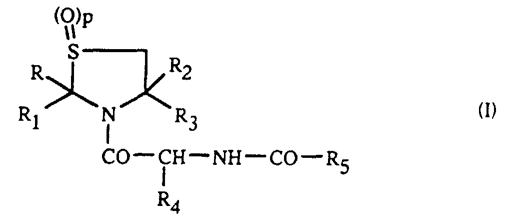 in which
R represents an unbranched- or branched-chain alkyl radical containing 1 to 12 carbon atoms and optionally mono- or polyunsaturated, a cycloalkyl radical containing 3 to 12 carbon atoms and optionally mono- or polyunsaturated, a polycycloalkyl radical containing 6 to 12 carbon atoms and optionally mono- or polyunsaturated, a phenylalkyl radical in which the phenyl ring is optionally substituted (with one or more substituents chosen from alkyl and alkoxy radicals or halogen atoms), a diphenylalkyl or cinnamyl radical, a pyridyl radical optionally substituted with one or more alkyl radicals, a furyl radical optionally substituted with one or more alkyl radicals, a thienyl radical optionally substituted with one or more alkyl radicals, a quinolyl radical optionally substituted with one or more alkyl radicals, a naphthyl radical optionally substituted with one or more alkyl radicals, an indolyl radical optionally substituted with one or more alkyl radicals, a 2-oxopiperidyl or quinuclidinyl radical or a phenyl radical optionally substituted with one or more substituents chosen from halogen atoms and alkyl, alkoxy, hydroxyl, nitro, amino, monoalkylamino, dialkylamino, alkoxycarbonyl, -CO-NR₇R₈, -NH-CO-CH₃, trifluoromethyl, phenyl or trifluoromethoxy radicals,
R₁ represents a hydrogen atom, an alkyl, cycloalkyl or phenylalkyl radical or a phenyl radical optionally substituted with one or more substituents chosen from halogen atoms and alkyl and alkoxy radicals,
R₂ represents a chain -(CB₂)ₙ-CO-R₆, -(CH₂)ₘ-O-CO-R"₆ or -(CH₂)ₘ-NR₉R₁₀, an oxazolinyl radical optionally substituted with one or more alkyl radicals or a 3-alkyloxadiazolyl radical,
R₃ represents a hydrogen atom, an alkyl, cycloalkyl or phenylalkyl radical or a phenyl radical optionally substituted with one or more substituents chosen from halogen atoms and alkyl and alkoxy radicals,
R₄ represents a hydrogen atom or an alkyl radical,
R₅ represents a phenyl radical (optionally substituted with one or more substituents chosen from halogen atoms and alkyl, alkoxy and alkylthio radicals), a naphthyl, indolyl or quinolyl radical or a phenylamino radical in which the phenyl ring is optionally substituted with one or more substituents chosen from halogen atoms and alkyl, alkoxy, alkylthio, trifluoromethyl, carboxyl, alkoxycarbonyl, hydroxyl, nitro, amino, acyl, cyano, sulphamoyl, carbamoyl, hydroxyiminoalkyl, alkoxyiminoalkyl, hydroxyaminocarbonyl, alkoxyaminocarbonyl, 5-tetrazolyl, 5-tetrazolylalkyl, trifluoromethylsulphonamido, alkylsulphinyl, mono- or polyhydroxyalkyl, sulpho, -alk-O-CO-alk, -alk-COOX, -alk-O-alk, -alk'-COOX, -O-alk-COOX, -CH=CH-COOX, -CO-COOX, -alk-SO₃H (in salt form), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₁, -SO₂-NH-SO₂-R₁₁, -CO-NH-CO-R₁₁, -CO-NH-SO₂-R₁₁, -B(OH)₂, -C(NH₂)=NOH, -SO₂-NH-R₁₂, -CO-NH-R₁₂, 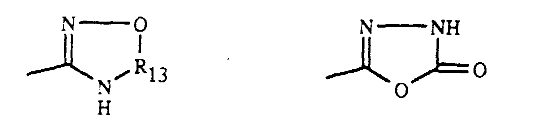 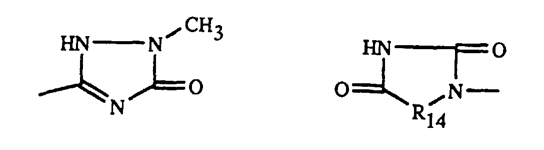 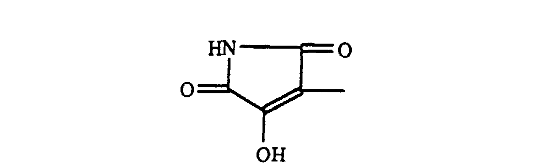 or 2,2-dimethyl-4,6-dioxo-1,3-dioxan-5-yl radicals,
R₆ represents a hydroxyl, alkoxy, cycloalkyloxy, cycloalkylalkyloxy or phenyl radical or a radical -NR₉R₁₀,
R"₆ represents an alkoxy, cycloalkyloxy, cycloalkylalkyloxy or phenyl radical or a radical -NR₉R₁₀,
R₇ represents a hydrogen atom, an alkyl or phenylalkyl radical or a phenyl radical optionally substituted with one or more substituents chosen from halogen atoms and alkyl, alkoxy and alkylthio radicals,
R₈ represents an alkyl or phenylalkyl radical or a phenyl radical optionally substituted with one or more substituents chosen from halogen atoms and alkyl, alkoxy and alkylthio radicals,
or alternatively R₇ and R₈, with the nitrogen atom to which they are attached, form a saturated or unsaturated mono- or polycyclic heterocycle containing 4 to 9 carbon atoms and one or more hetero atoms (O, N) and optionally substituted with one or more alkyl radicals,
R₉ represents a hydrogen atom, an alkyl, cycloalkylalkyl, cycloalkyl or phenylalkyl radical or a phenyl radical optionally substituted with one or more substituents chosen from halogen atoms and alkyl, alkoxy and alkylthio radicals,
R₁₀ represents an alkyl, cycloalkylalkyl, cycloalkyl or phenylalkyl radical or a phenyl radical optionally substituted with one or more substituents chosen from halogen atoms and alkyl, alkoxy and alkylthio radicals, or alternatively R₉ and R₁₀, together with the nitrogen atom to which they are attached, form a saturated or unsaturated mono- or polycyclic heterocycle containing 4 to 9 carbon atoms and one or more hetero atoms (O, N, S) and optionally substituted with one or more alkyl radicals,
R₁₁ represents an alkyl, cycloalkyl or trifluoromethyl radical or a phenyl radical optionally substituted with one or more substituents chosen from cyano, alkoxy, nitro and amino radicals and halogen atoms,
R₁₂ represents a 5-tetrazolyl radical,
R₁₃ represents C=P or S=O,
R₁₄ represents O or C=O,
p is equal to 0, 1 or 2,
n is equal to 0, 1 or 2,
m is equal to 1 or 2,
X represents a hydrogen atom or an alkyl or phenylalkyl radical,
alk represents an alkyl or alkylene radical,
alk' represents a hydroxyalkyl, hydroxyalkylene, alkoxyalkyl or alkoxyalkylene radical,
on the understanding that n is other than 0 when R₁ and R₃ each represent a hydrogen atom and R represents a pyridyl radical optionally substituted with one or more alkyl radicals, a furyl radical optionally substituted with one or more alkyl radicals, a thienyl radical optionally substituted with one or more alkyl radicals, a quinolyl radical optionally substituted with one or more alkyl radicals, a naphthyl radical optionally substituted with one or more alkyl radicals, an indolyl radical optionally substituted with one or more alkyl radicals or a phenyl radical optionally substituted with one or more substituents chosen from halogen atoms and alkyl, alkoxy, hydroxyl, nitro, amino, monoalkylamino, dialkylamino, alkoxycarbonyl, -CO-NR₇R₈, -NH-CO-CH₃, trifluoromethyl or trifluoromethoxy radicals, and on the understanding that, except where otherwise stated, the alkyl, alkylene and alkoxy radicals and the alkyl, alkylene and alkoxy portions contain 1 to 4 carbon atoms in an unbranched or branched chain, the acyl radicals and portions contain 2 to 4 carbon atoms and the cycloalkyl radicals and portions contain 3 to 6 carbon atoms,
as well as their salts and their isomers when they contain at least one asymmetric centre.

2. Compounds of formula (I) according to claim 1 for which R represents an isopropenyl, cyclohexyl, tetrahydrophenyl, cyclopentadienyl, dihydrophenyl, norbornyl, adamantyl or norbornenyl radical, as well as their salts and their isomers when they contain at least one asymmetric centre.

3. Compounds of formula (I) according to claim 1 for which R₇ and R₈, with the nitrogen atom to which they are attached, form a heterocycle chosen from a piperidino ring optionally substituted with one or more alkyl radicals and a 1,2,3,4-tetrahydroquinoline ringsystem, as well as their salts and their isomers when they contain at least one asymmetric centre.

4. Compounds of formula (I) according to claim 1 for which R₉ and R₁₀, with the nitrogen atom to which they are attached, form a heterocycle chosen from piperidino, perhydro-1-azepinyl, 1,2,3,6-tetrahydro-1-pyridyl, 1,2,3,4-tetrahydro-1-quinolyl, 1-pyrrolidinyl, 1,2,3,4-tetrahydro-2-isoquinolyl, thiomorpholino and 1-indolinyl ring-systems, it being possible for these ring-systems to be optionally substituted with at least one alkyl radical, as well as their salts and their isomers when they contain at least one asymmetric centre.

5. Compounds of formula (I) according to claim 1 for which R represents an unbranched- or branched-chain alkyl radical containing 1 to 12 carbon atoms, a cycloalkyl radical containing 3 to 12 carbon atoms and optionally monounsaturated, a polycycloalkyl radical containing 6 to 12 carbon atoms and optionally mono- or polyunsaturated, a phenylalkyl radical or a phenyl radical optionally substituted with one or more substituents chosen from halogen atoms and alkyl, -alk-COOX and carboxyl radicals, R₁ represents a hydrogen atom, R₂ represents a chain -(CH₂)ₙ-CO-R₆, R₃ represents a hydrogen atom, R₄ represents a hydrogen atom and R₅ represents a phenylamino radical in which the phenyl ring is substituted with an alkyl, -alk-COOX or carboxyl radical, as well as their salts and their isomers when they contain at least one asymmetric centre.

6. The following compounds:
- (4R)-3-{3-[2-(4-tert-butoxycarbonyl-2-cyclohexyl-3-thiazolidinyl)-2-oxoethyl]ureido}phenylacetic acid,
- tert-butyl (4R)-3-{2-[3-(3-methylphenyl)ureido]-acetyl}-2-benzyl-4-thiazolidinecarboxylate,
- (4R)-3-{3-[2-(4-tert-butoxycarbonyl-2-benzyl-3-thiazolidinyl)-2-oxoethyl]ureido}phenylacetic acid,
- (2R,4R)-3-[3-{2-[4-tert-butoxycarbonyl-2-((RS)-1,2,3,6-tetrahydro-1-phenyl)-3-thiazolidinyl]-2-oxoethyl}-ureido]phenylacetic acid,
- (4R)-3-[3-{2-[4-tert-butoxycarbonyl-2-[(2RS)-5-norbornen-2-yl]-3-thiazolidinyl]-2-oxoethyl}ureido] -phenylacetic acid,
- methyl (4R)-3-[3-{2-[4-tert-butoxycarbonyl-2-[(2RS)-5-norbornen-2-yl]-3-thiazolidinyl]-2-oxoethyl}ureido]-phenylacetate,
- (4R)-3-[3-{2-[4-tert-butoxycarbonyl-2-[(2RS)-5-norbornen-2-yl]-3-thiazolidinyl]-2-oxoethyl}ureido]-phenylacetic acid,
- (4R)-3-[3-(2-[4-tert-butoxycarbonyl-2-((2RS)-2-norbornyl)-3-thiazolidinyl}-2-oxoethyl}ureido]-phenyl-acetic acid,
- tert-butyl (4R)-3-{2-[3-(3-methylphenyl)ureido]-acetyl}-2-[(RS)-5-norbornen-2-yl]thiazolidine-carboxylate,
- (4R)-3-{3-[2-(4-tert-butoxycarbonyl-2-tert-butyl-3-thiazolidinyl)-2-oxoethyl]ureido}phenylaeetic acid,
- (S)-3-{3-t2-((4R)-4-tert-butoxycarbonyl-2-butyl-3-thiazolidinyl)-2-oxoethyl]ureido}-2-phenylpropionic acid,
- tert-butyl (4R)-3-{2-[3-(3-methylphenyl)ureido]-acetyl}-2-butyl-4-thiazolidinecarboxylate,
- (4R)-3-{3-[2-(4-tert-butoxycarbonyl-2-butyl-3-thiazolidinyl)-2-oxoethyl]ureido}benzoic acid,
- tert-butyl (2R,4R)-3-{2-[3-(3-methylphenyl)ureido]-acetyl}-2-((RS)-1,2,3,6-tetrahydro-1-phenyl)-4-thiazolidinecarboxylate,
- (2R,4R)-3-[3-{2-[4-tert-butoxycarbonyl-2-(2-phenylphenyl)-3-thiazolidinyl]-2-oxoethyl}ureido]-phenylacetic acid,
- tert-butyl (2R,4R)-3-{2-[3-(3-methylphenyl)ureido]-acetyl}-2-(2-phenylphenyl)-4-thiazolidinecarboxylate,
- (S)-3- [3-{2-[(2R,4RS)-4-carboxy-2-(2-fluorophenyl)-3-thiazolidinyl]-2-oxoethyl}ureido]-2-phenylpropionic acid, their isomers, the mixtures of their isomers and their salts.

7. Process for preparing the compounds of formula (I) according to claim 1 for which p is equal to 0 and R₅ represents a phenylamino radical in which the phenyl ring is optionally substituted, characterized in that a reactive derivative of carbamic acid, optionally obtained in situ by the action of a reactive derivative of carbonic acid chosen from N,N'-carbonyldiimidazole, phosgene, diphosgene, triphosgene and p-nitrophenyl chloroformate on a derivative of formula: 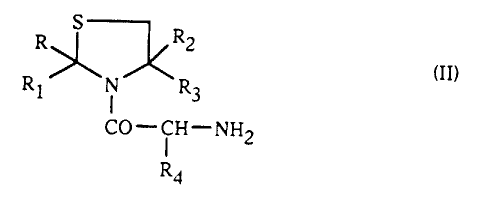 in which R, R₁, R₂, R₃ and R₄ have the same meanings as in claim 1, is reacted with an aniline in which the phenyl ring is optionally substituted with one or more substituents chosen from halogen atoms and alkyl, alkoxy, alkylthio, trifluoromethyl, carboxyl, alkoxycarbonyl, hydroxyl, nitro, amino, acyl, cyano, sulphamoyl, carbamoyl, hydroxyiminoalkyl, alkoxyiminoalkyl, hydroxyaminocarbonyl, alkoxyaminocarbonyl, 5-tetrazolyl, 5-tetrazolylalkyl, trifluoromethylsulphonamido, alkylsulphinyl, mono- or polyhydroxyalkyl, sulpho, -alk-O-CO-alk, -alk-COOX, -alk-O-alk, -alk'-COOX, -O-alk-COOX, -CH=CH-COOX, -CO-COOX, -alk-SO₃H, -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, 2,2-dimethyl-4,6-dioxo-1,3-dioxan-5-yl, -alk-SO₂H, -SO₂-NH-CO-R₁₁, -SO₂-NH-SO₂-R₁₁, -CO-NH-CO-R₁₁, -CO-NH-SO₂-R₁₁, -B(OH)₂, -C(NH₂)=NOH, -SO₂-NH-R₁₂, -CO-NH-R₁₂ or 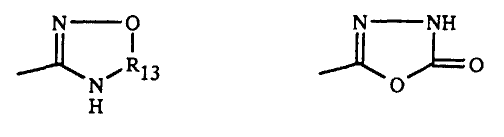 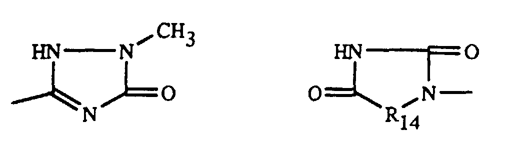 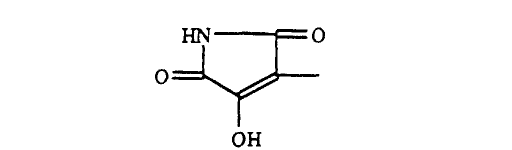 radicals, and the product is isolated and optionally converted to a salt.

8. Process for preparing the compounds of formula (I) according to claim 1 for which p is equal to 0 and R₅ represents a phenylamino radical in which the phenyl ring is optionally substituted with one or more substituents chosen from halogen atoms and alkyl, alkoxy, alkylthio, trifluoromethyl, nitro, acyl, cyano, sulphamoyl, alkoxycarbonyl, carbamoyl, alkoxyiminoalkyl, alkoxyaminocarbonyl, -alk-O-CO-alk, -CH=CH=alk', -alk-O-alk, trifluoromethylsulphonamido, -alk-SO₃H (in salt form), -O-alk-COOX, -CH=CH-COOX, -CO-COOX, -S-alk-COOX, -SO-alk-COOX, SO₂-alk-COOX, -O-CH₂-alk'-COOX, -CX=N-O-alk-COOX, -alk-COOX or -alk'-COOX radicals in which X is other than a hydrogen atom, characterized in that a derivative of formula: 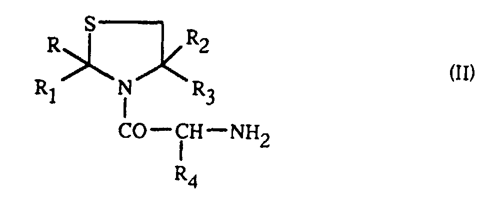 in which R, R₁, R₂, R₃ and R₄ have the same meanings as in claim 1, is reacted with a phenyl isocyanate in which the phenyl ring is optionally substituted with one or more substituents chosen from halogen atoms and alkyl, alkoxy, alkylthio, trifluoromethyl, nitro, acyl, cyano, sulphamoyl, alkoxycarbonyl, carbamoyl, alkoxyiminoalkyl, alkoxyaminocarbonyl, -alk-O-CO-alk, -CH=CH-alk', -alk-O-alk, trifluoromethylsulphonamido, -alk-SO₃H (in salt form), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX or -alk'-COOX radicals in which X is other than a hydrogen atom, and the product is isolated and optionally converted to a salt.

9. Process for preparing the compounds of formula (I) according to claim 1 for which p is equal to 0 and R₅ represents an optionally substituted phenyl radical or a naphthyl, indolyl or quinolyl radical, characterized in that a derivative of formula: 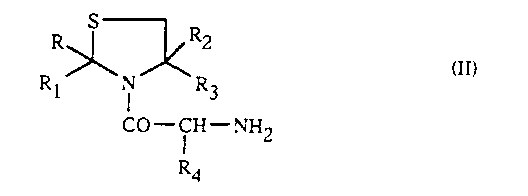 in which R, R₁, R₂, R₃ and R₄ have the same meanings as in claim 1, is reacted with an acid of formula HOOC-R₅ in which R₅ has the same meanings as above, or a reactive derivative of this acid, and the product is isolated and optionally converted to a salt.

10. Process for preparing the compounds of formula (I) according to claim 1 for which R₅ represents a phenylamino radical in which the phenyl ring is substituted with a carboxyl, -alk-COOH, -O-alk-COOH, -alk'-COOH, -CH=CH-COOH, -CO-COOH, -S-alk-COOH, -SO-alk-COOH, -SO₂-alk-COOH, -C(=NOH)-COOH, -O-CH₂- alk'-COOH or -CX=N-O-alk-COOH radical and/or R₂ represents a chain -(CH₂)ₙ-COOH, characterized in that a corresponding ester of formula (I) is hydrolysed or, depending on the case, subjected to hydrogenolysis, and the product is isolated and optionally converted to a salt.

11. Process for preparing the compounds of formula (I) according to claim 1 for which p is equal to 0 and R₅ represents a phenylamino radical in which the phenyl ring is substituted with a hydroxyiminoalkyl or alkoxyiminoalkyl radical, characterized in that a corresponding acylated derivative of formula (I) is reacted with a derivative of formula:
H₂N-OR₁₈ (XI)
in which R₁₈ represents a hydrogen atom or an alkyl radical, and the product is isolated and optionally converted to a salt.

12. Process for preparing compounds of formula (I) according to claim 1 for which p is equal to 0, R₂ represents a chain -(CH₂)ₙ-CO-R₆, n is equal to 0, R₆ represents an alkoxy, cycloalkyloxy or cycloalkylalkyloxy radical and R₅ represents an optionally substituted phenyl radical, a naphthyl, indolyl or quinolyl radical or a phenylamino radical in which the phenyl ring is optionally substituted with one or more substituents chosen from halogen atoms and alkyl, alkoxy, alkylthio, trifluoromethyl, nitro, acyl, cyano, sulphamoyl, alkoxycarbonyl, carbamoyl, alkoxyiminoalkyl, alkoxyaminocarbonyl, -alk-O-CO-alk, -CH=CH-alk', -alk-O-alk, trifluoromethylsulphonamido, -alk-SO₃H (in salt form), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX or -alk'-COOX radicals in which X is other than a hydrogen atom, characterized in that a derivative of formula: 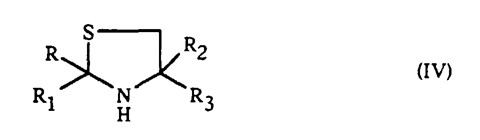 in which R, R₁ and R₃ have the same meanings as in the formula (I) and R₂ has the same meanings as above, is reacted with an acid of formula: 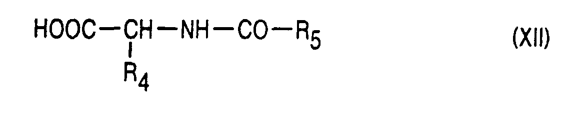 in which R₅ has the same meanings as above, or a reactive derivative of this acid, and R₄ has the same meanings as in the formula (I).

13. Process for preparing the compounds of formula (I) according to claim 1 for which p is equal to 0 and R represents a saturated cycloalkyl radical or a saturated polycycloalkyl radical, characterized in that a corresponding unsaturated derivative of formula (I) is hydrogenated, and the product is isolated and optionally converted to a salt.

14. Process for preparing the compounds of formula (I) according to claim 1 for which p is equal to 1 or 2, characterized in that a corresponding compound of formula (I) for which p is equal to 0 is oxidized, and the product is isolated and optionally converted to a salt.

15. Medicinal products, characterized in that they contain as active principle at least one of the compounds according to one of claims 1 to 6.

16. Medicinal products according to claim 15, which can be used for the treatment of disorders linked to CCK and to gastrin.
